(19) **Europäisches Patentamt** / **European Patent Office** / **Office européen des brevets**

(11) **EP 4 467 649 A1**

(12) **EUROPEAN PATENT APPLICATION**
published in accordance with Art. 153(4) EPC

(43) Date of publication:
27.11.2024 Bulletin 2024/48

(21) Application number: 23743280.2

(22) Date of filing: 18.01.2023

(51) International Patent Classification (IPC):
*C12N 15/53* (2006.01)      *C12N 1/15* (2006.01)
*C12N 1/19* (2006.01)      *C12N 1/21* (2006.01)
*C12N 5/10* (2006.01)      *C12N 9/04* (2006.01)
*C12N 15/63* (2006.01)      *C12P 21/02* (2006.01)

(52) Cooperative Patent Classification (CPC):
C12N 5/10; C12N 9/0004; C12N 15/63;
C12N 15/74; C12N 15/80; C12N 15/81; C12P 21/02

(86) International application number:
PCT/JP2023/001336

(87) International publication number:
WO 2023/140286 (27.07.2023 Gazette 2023/30)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 18.01.2022 JP 2022005869

(71) Applicant: Kikkoman Corporation
Noda-shi, Chiba 278-8601 (JP)

(72) Inventors:
• KUZE Keitaro
  Noda-shi, Chiba 278-8601 (JP)
• ICHIYANAGI Atsushi
  Noda-shi, Chiba 278-8601 (JP)
• HIZUME Tatsuya
  Noda-shi, Chiba 278-8601 (JP)

(74) Representative: Mewburn Ellis LLP
Aurora Building
Counterslip
Bristol BS1 6BX (GB)

Remarks:
The complete document including Reference
Table(s) and the Sequence Listing(s) can be
downloaded from the EPO website

(54) **RECOMBINANT EXPRESSION GLUTAMATE OXIDASE**

(57) A glutamate oxidase that requires no protease treatment is produced. Provided is a glutamate oxidase mutant comprising a deletion of a predetermined amino acid sequence.

EP 4 467 649 A1

## Description

Technical Field

The present invention relates to a recombinantly expressed glutamate oxidase.

Background Art

In previous times, L-glutamate measurement was performed using a L-glutamate decarboxylase and L-glutamate dehydrogenase. However, these measurements were both complicated because carbon dioxide measurement in the measurement system of decarboxylase was time-consuming and the measurement system of dehydrogenase involved measuring the change in absorbance of a coenzyme.

Subsequently, a L-glutamate oxidase that acts only on L-glutamate from solid culture of streptomycetes was reported (Non Patent Literature 1). The L-glutamate oxidase is encoded as one polypeptide having $\alpha$, $\gamma$, and $\beta$ chains and two such polypeptides form a homodimer. This homodimer is cleaved by a protease and becomes the mature form. The mature form is a heterohexamer constituted by $\alpha_2\beta_2\gamma_2$.

A recombinantly expressed homodimer had weak activity as a L-glutamate oxidase, was inferior in substrate affinity to the original L-glutamate oxidase of streptomycetes, and was also unstable against heat. This recombinantly expressed homodimer enzyme, when subjected to protease treatment, was converted into the same structure as that of the original L-glutamate oxidase of streptomycetes and also acquired enzyme chemical properties equivalent thereto (Non Patent Literature 2). A method of producing this precursor at a large scale using recombinant *Escherichia coli* and subjecting the precursor to protease treatment is used in the industry.

Patent Literature 1 describes a L-glutamate oxidase mutant. Patent Literature 1 has no description on the thermal stability of the prepared L-glutamate oxidase mutant.

Citation List

Patent Literature

Patent Literature 1: WO 2021/193598

Non Patent Literature

Non Patent Literature 1: Kusakabe H et al., Agric. Biol. Chem., 47, 1323-1328 (1983)
Non Patent Literature 2: Arima J et al., J. Biochem., 134, 805-812 (2003)

Summary of Invention

Technical Problem

In a particular embodiment, an object of the present disclosure is to provide a method for conveniently producing a L-glutamate oxidase. Further, in a particular embodiment, an object of the present disclosure is to provide a L-glutamate oxidase. Further, in a particular embodiment, an object of the present disclosure is to provide a L-glutamate oxidase having a high sequence identity with SEQ ID NO: 58.

In the case of recombinant expression of a L-glutamate oxidase, in particular, in the case of large-scale recombinant expression of a L-glutamate oxidase for use in the industry, conventional production methods in the industry required protease treatment to obtain a mature form of the L-glutamate oxidase. However, the protease treatment process was complicated and caused high costs.

Solution to Problem

The present inventors carried out concentrated studies in order to attain the above objects. As a result, the present inventors found that, as one example, by using a genetically engineered glutamate oxidase from streptomycetes, the objects could be attained at least in part, and thereby completed the present invention including this as one embodiment. The present inventors also found that by using a glutamate oxidase having a particular mutation, the objects above could be attained at least in part, thereby completing the present invention including this as one embodiment.

The present disclosure provides the following embodiments.

[1] A glutamate oxidase mutant having a deleted region, wherein

all or part of an inter-γ-β region located between the γ chain region and the β chain region of a wild type glutamate oxidase is deleted,
1 to 10 carboxy terminal amino acids of the γ chain region of the wild type glutamate oxidase are deleted or not deleted,
1 to 4 amino terminal amino acids of the β chain region of the wild type glutamate oxidase are deleted or not deleted,
the deleted region is one continuous deleted region, and the glutamate oxidase mutant comprises glutamate oxidase activity.

[2] The glutamate oxidase mutant according to Embodiment 1, wherein 31 to 54 continuous amino acids are deleted.
[3] The glutamate oxidase mutant according to Embodiment 1, wherein the C terminus of the deleted region is located at the position corresponding to the 510th position, the position corresponding to the 508th position, or the position corresponding to the 507th position of SEQ ID NO: 1.
[4] The mutant according to Embodiment 1, wherein the amino acid sequence of the region corresponding to the 459th to 507th positions of SEQ ID NO: 1 is deleted and thermal stability is improved compared to a glutamate oxidase in which all or part of the region corresponding to the 459th to 507th positions of SEQ ID NO: 1 is not deleted.
[5] The mutant according to Embodiment 4, wherein the amino acid sequence of the region corresponding to the 459th to 507th positions of SEQ ID NO: 1 is deleted and thermal stability is improved compared to a glutamate oxidase in which the amino acid sequence of the region corresponding to the 459th to 466th positions of SEQ ID NO: 1 is not deleted.
[6] The mutant according to Embodiment 4 or 5, wherein

the improved thermal stability is evaluated from residual activity after heat treatment at 45°C for 30 minutes or 35 minutes or heat treatment at 65°C for 30 minutes, and
the residual activity of the mutant according to Embodiment 4 after being subjected to heat treatment at 45°C for 30 minutes or 35 minutes or heat treatment at 65°C for 30 minutes is 110% or more when the residual activity of a glutamate oxidase comprising no deletion of the amino acid sequence after being subjected to the same treatment as above is defined as 100%, or
the residual activity of the mutant according to Embodiment 5 after being subjected to heat treatment at 45°C for 30 minutes or 35 minutes or heat treatment at 65°C for 30 minutes is 110% or more when the residual activity of a glutamate oxidase comprising no deletion of the amino acid sequence after being subjected to the same treatment as above is defined as 100%.

[7] The mutant according to any of Embodiments 1 to 6, wherein the mutant comprises an amino acid sequence identity of 90% or more with the amino acid sequence of SEQ ID NO: 58, SEQ ID NO: 59, SEQ ID NO: 12 or SEQ ID NO: 13, comprises a deletion of the amino acid sequence, and comprises glutamate oxidase activity.
[8] The mutant according to any of Embodiments 1 to 7, wherein

the mutant further comprises an amino acid substitution compared to the amino acid sequence of SEQ ID NO: 1 and/or SEQ ID NO: 58, at one or more positions selected from the group consisting of
the position corresponding to the 87th position of SEQ ID NO: 1,
the position corresponding to the 103rd position of SEQ ID NO: 1,
the position corresponding to the 133rd position of SEQ ID NO: 1,
the position corresponding to the 186th position of SEQ ID NO: 1,
the position corresponding to the 297th position of SEQ ID NO: 1,
the position corresponding to the 376th position of SEQ ID NO: 1,
the position corresponding to the 393rd position of SEQ ID NO: 1,
the position corresponding to the 428th position of SEQ ID NO: 1,
the position corresponding to the 516th position of SEQ ID NO: 1,
the position corresponding to the 566th position of SEQ ID NO: 1,
the position corresponding to the 568th position of SEQ ID NO: 1,
the position corresponding to the 585th position of SEQ ID NO: 1, and
the position corresponding to the 615th position of SEQ ID NO: 1, and

the thermal stability of the mutant after the amino acid substitution is improved compared to a glutamate oxidase before the substitution.

[9] The mutant according to Embodiment 8, wherein

the amino acid substitution at the position corresponding to the 87th position of SEQ ID NO: 1 is substitution with tyrosine,
the amino acid substitution at the position corresponding to the 103rd position of SEQ ID NO: 1 is substitution with an amino acid selected from the group consisting of phenylalanine, leucine, valine, isoleucine and methionine,
the amino acid substitution at the position corresponding to the 133rd position of SEQ ID NO: 1 is substitution with an amino acid selected from the group consisting of leucine and tyrosine,
the amino acid substitution at the position corresponding to the 186th position of SEQ ID NO: 1 is substitution with an amino acid selected from the group consisting of glutamic acid, aspartic acid, tyrosine, glutamine, asparagine, alanine, leucine, cysteine, methionine, phenylalanine, serine, histidine and threonine,
the amino acid substitution at the position corresponding to the 297th position of SEQ ID NO: 1 is substitution with an amino acid selected from the group consisting of leucine, valine, isoleucine and methionine,
the amino acid substitution at the position corresponding to the 376th position of SEQ ID NO: 1 is substitution with an amino acid selected from the group consisting of phenylalanine, leucine, isoleucine and methionine,
the amino acid substitution at the position corresponding to the 393rd position of SEQ ID NO: 1 is substitution with an amino acid selected from the group consisting of leucine, valine, isoleucine and methionine,
the amino acid substitution at the position corresponding to the 428th position of SEQ ID NO: 1 is substitution with an amino acid selected from the group consisting of tyrosine and methionine,
the amino acid substitution at the position corresponding to the 516th position of SEQ ID NO: 1 is substitution with phenylalanine,
the amino acid substitution at the position corresponding to the 566th position of SEQ ID NO: 1 is substitution with an amino acid selected from the group consisting of phenylalanine, leucine, valine and methionine,
the amino acid substitution at the position corresponding to the 568th position of SEQ ID NO: 1 is substitution with an amino acid selected from the group consisting of isoleucine and methionine,
the amino acid substitution at the position corresponding to the 585th position of SEQ ID NO: 1 is substitution with an amino acid selected from the group consisting of leucine and methionine, or
the amino acid substitution at the position corresponding to the 615th position of SEQ ID NO: 1 is substitution with an amino acid selected from the group consisting of leucine, valine, and phenylalanine.

[10] The mutant according to Embodiment 9, wherein

the mutant comprises an amino acid sequence identity of 90% or more with the amino acid sequence of SEQ ID NO: 69, comprises a deletion of the amino acid sequence,
comprises an amino acid substitution compared to the amino acid sequence of SEQ ID NO: 58, at one or more positions selected from the group consisting of
the position corresponding to the 87th position of SEQ ID NO: 1,
the position corresponding to the 103rd position of SEQ ID NO: 1,
the position corresponding to the 133rd position of SEQ ID NO: 1,
the position corresponding to the 186th position of SEQ ID NO: 1,
the position corresponding to the 297th position of SEQ ID NO: 1,
the position corresponding to the 376th position of SEQ ID NO: 1,
the position corresponding to the 393rd position of SEQ ID NO: 1,
the position corresponding to the 428th position of SEQ ID NO: 1,
the position corresponding to the 516th position of SEQ ID NO: 1,
the position corresponding to the 566th position of SEQ ID NO: 1,
the position corresponding to the 568th position of SEQ ID NO: 1,
the position corresponding to the 585th position of SEQ ID NO: 1, and
the position corresponding to the 615th position of SEQ ID NO: 1,
the amino acid substitution being any of the amino acid substitutions described in Embodiment 9, and
the mutant comprises glutamate oxidase activity.

[11] The glutamate oxidase mutant according to any of Embodiments 1 to 10, wherein the amino acid sequence of the region corresponding to the 670th to 687th positions of SEQ ID NO: 1 is further deleted.
[12] A composition, reagent, electrode, sensor or kit comprising the glutamate oxidase mutant according to any of Embodiments 1 to 10.

[13] A polynucleotide encoding the glutamate oxidase mutant according to any of Embodiments 1 to 11.

[14] A vector comprising the polynucleotide according to Embodiment 13.

[15] A host cell comprising the vector according to Embodiment 14.

[16] A method for producing a glutamate oxidase mutant comprising the steps of:

culturing the host cell according to Embodiment 15 to produce the glutamate oxidase mutant according to any of Embodiments 1 to 11, and
obtaining the produced glutamate oxidase mutant.

[17] A method for bringing the glutamate oxidase mutant according to any of Embodiments 1 to 11 or the composition, reagent, electrode, sensor or kit according to Embodiment 12 into contact with a sample containing glutamic acid to oxidize the glutamic acid contained in the sample.

[18] The method according to Embodiment 17, comprising detecting glutamic acid.

[19] A glutamate oxidase mutant comprising an amino acid substitution, wherein

the thermal stability of the glutamate oxidase mutant after the amino acid substitution is improved compared to a glutamate oxidase before the substitution, and
the glutamate oxidase mutant satisfies a condition selected from the group consisting of:

(i) when the amino acid sequence of the glutamate oxidase mutant is aligned with the amino acid sequence of SEQ ID NO: 1, the glutamate oxidase mutant comprises a substitution of the amino acid at the position corresponding to a position selected from the group consisting of the 87th, 103rd, 133rd, 186th, 297th, 376th, 393rd, 428th, 516th, 566th, 568th, 585th, and 615th positions of SEQ ID NO: 1,
(ii) the glutamate oxidase mutant of said (i) consists of an amino acid sequence comprising a substitution, deletion or addition of one or several amino acids at a position other than the positions corresponding to the 87th, 103rd, 133rd, 186th, 297th, 376th, 393rd, 428th, 516th, 566th, 568th, 585th, and 615th positions of SEQ ID NO: 1,
(iii) with regard to the glutamate oxidase mutant of said (i) or (ii), the full length amino acid sequence thereof comprises an amino acid sequence identity of 70% or more, 80% or more, or 90% or more with the amino acid sequence of SEQ ID NO: 1 or SEQ ID NO: 58, and
(iv) with regard to the glutamate oxidase mutant of said (i) or (ii), the full length amino acid sequence thereof comprises an amino acid sequence identity of 70% or more, 80% or more, or 90% or more with the amino acid sequence of SEQ ID NO: 1 or SEQ ID NO: 58, the amino acid substitution at the position corresponding to the 291st position of SEQ ID NO: 1 in the glutamate oxidase mutant is arginine, and the amino acid sequence of the positions corresponding to the 51st to 56th positions of SEQ ID NO: 1 is Gly-Xaa-Gly-Xaa-Xaa-Gly (wherein Xaa represents an amino acid).

[20] The glutamate oxidase mutant according to Embodiment 19, wherein

the amino acid substitution at the position corresponding to the 87th position of SEQ ID NO: 1 is substitution with tyrosine,
the amino acid substitution at the position corresponding to the 103rd position of SEQ ID NO: 1 is substitution with an amino acid selected from the group consisting of phenylalanine, leucine, valine, isoleucine and methionine,
the amino acid substitution at the position corresponding to the 133rd position of SEQ ID NO: 1 is substitution with an amino acid selected from the group consisting of leucine and tyrosine,
the amino acid substitution at the position corresponding to the 186th position of SEQ ID NO: 1 is substitution with an amino acid selected from the group consisting of glutamic acid, aspartic acid, tyrosine, asparagine, glutamine, alanine, leucine, cysteine, methionine, phenylalanine, serine, histidine and threonine,
the amino acid substitution at the position corresponding to the 297th position of SEQ ID NO: 1 is substitution with an amino acid selected from the group consisting of leucine, valine, isoleucine and methionine,
the amino acid substitution at the position corresponding to the 376th position of SEQ ID NO: 1 is substitution with an amino acid selected from the group consisting of phenylalanine, leucine, isoleucine and methionine,
the amino acid substitution at the position corresponding to the 393rd position of SEQ ID NO: 1 is substitution with an amino acid selected from the group consisting of leucine, valine, isoleucine and methionine,
the amino acid substitution at the position corresponding to the 428th position of SEQ ID NO: 1 is substitution with an amino acid selected from the group consisting of tyrosine and methionine,
the amino acid substitution at the position corresponding to the 516th position of SEQ ID NO: 1 is substitution with phenylalanine,
the amino acid substitution at the position corresponding to the 566th position of SEQ ID NO: 1 is substitution with an amino acid selected from the group consisting of phenylalanine, leucine, valine and methionine,
the amino acid substitution at the position corresponding to the 568th position of SEQ ID NO: 1 is substitution with an amino acid selected from the group consisting of isoleucine and methionine,
the amino acid substitution at the position corresponding to the 585th position of SEQ ID NO: 1 is substitution with an amino acid selected from the group consisting of leucine and methionine, or
the amino acid substitution at the position corresponding to the 615th position of SEQ ID NO: 1 is substitution with an amino acid selected from the group consisting of leucine, valine, and phenylalanine.

The present specification encompasses the contents disclosed in JP Patent Application No. 2022-005869, which is a priority document of the present application.

Advantageous Effects of Invention

In a particular embodiment, one effect of the present invention is that a recombinant expression glutamate oxidase that requires no protease treatment can be obtained. In a particular embodiment, a glutamate oxidase with improved thermal stability can be obtained.

Description of Embodiments

A L-glutamate oxidase is encoded as one polypeptide having $\alpha$, $\gamma$, and $\beta$ chains by a GLOD gene and two such polypeptides form a homodimer. In nature, this homodimer is cleaved by a protease into the mature form. The mature form is a heterohexamer composed of $\alpha_2\beta_2\gamma_2$.

Unless specified otherwise, the term GLOD refers to a L-glutamate oxidase in the present specification. L-glutamate oxidase is an oxidoreductase classified as EC 1.4.3.11 and catalyzes the following chemical reaction.

$$[\text{Formula 1}] \qquad \text{L-glutamate} + O_2 + H_2O \rightarrow \text{2-oxoglutaric acid} + NH_3 + H_2O_2$$

GLOD uses flavin adenine dinucleotide (FAD) as a coenzyme. Oxidoreductases using FAD as a coenzyme are known to have a FAD binding motif sequence, for example, a Gly-Xaa-Gly-Xaa-Xaa-Gly motif (wherein Xaa is any amino acid). For example, when taking SEQ ID NO: 1 as the standard sequence (with reference to the sequence of SEQ ID NO: 1), "Gly-Ala-Gly-Ile-Ala-Gly", the amino acid sequence of the 51st to 56th positions of SEQ ID NO: 1, amounts to the FAD binding motif sequence represented by Gly-Xaa-Gly-Xaa-Xaa-Gly. In one embodiment, with regard to the mutant of the present disclosure, Gly at the positions corresponding to the 51st, 53rd, and 56th positions, respectively, when taking SEQ ID NO: 1 as the standard sequence, need not be subjected to amino acid substitution. In one embodiment, Gly at the positions corresponding to the 51st, 53rd, and 56th positions of SEQ ID NO: 1 in the L-glutamate oxidase are not subjected to amino acid substitution.

A GLOD is known to recognize the substrate glutamate through an arginine residue. For example, Arg (R) at the 291st position with reference to SEQ ID NO: 1 (i.e., by taking SEQ ID NO: 1 as the standard sequence) is an important position for recognizing glutamate. In one embodiment, with regard to the mutant of the present disclosure, the position corresponding to the 291st position of SEQ ID NO: 1 does not have to undergo an amino acid substitution. In one embodiment, with regard to the mutant of the present disclosure, when the amino acid at the position corresponding to the 291st position of SEQ ID NO: 1 is Arg (R), this amino acid is not substituted.

(Standard sequence)

In the present specification, for convenience, each position of a GLOD is defined with reference to SEQ ID NO: 1 (i.e., by taking SEQ ID NO: 1 as the standard sequence). SEQ ID NO: 1 is the amino acid sequence of the glutamate oxidase from *Streptomyces* sp. X-119-6 (StGLOD). However, the N terminal secretory signal sequence (MTTDTARRHTGAER) of the naturally occurring sequence is excised. Further, in SEQ ID NO: 1, the 1st amino acid of the $\alpha$ chain region immediately after cleavage of the secretory signal sequence in wild type is altered from the wild type Ala to Met. The position of this Met is defined as the position corresponding to the 1st position (position 1) of SEQ ID NO: 1 in the present specification.

(Each subunit of GLOD)

In the present specification, for convenience, the $\alpha$ chain of the GLOD is defined as the polypeptide constituted of 375 amino acid residues from the 1st to 375th positions (ANEM ... EGEP) with reference to SEQ ID NO: 1. In the present specification, for convenience, the γ chain of the GLOD is defined as the polypeptide constituted of 91 amino acids from the 376th to 466th positions (YAAT... AEAA) with reference to SEQ ID NO: 1. In the present specification, for convenience, the β chain of the GLOD is defined as the polypeptide constituted of 163 amino acids from the 507th to 669th positions with reference to SEQ ID NO: 1. The region from the 467th to 506th positions with reference to SEQ ID NO: 1 is excised by a protease in nature (LALP ... SELR). The C terminal sequence from the 670th to 687th positions (RRGAAAATEPMREEALTS), with reference to SEQ ID NO: 1, is a region that is excised by a protease.

(Amino acid sequence deletion type GLOD mutant)

The present inventors believed that in the amino acid sequence of the GLOD, a region to be excised by a protease would interfere with the exhibition of GLOD functions. Therefore, a GLOD mutant sequence, in which the amino acid sequence between the γ and β regions is deleted, was prepared and this was expressed. When removing the region to be excised by a protease, in one embodiment, the last 8 amino acids (GEDDAEAA) of the γ region were also removed from the mutant. Further, the first amino acid residue G of the β chain was also removed from the mutant. As such, the boundary between the γ chain region and β chain region of the prepared mutant was a sequence represented by γ chain region ... QQW-GVRP... β chain region in which the C terminal QQW of the γ chain was connected to the N terminal GVRP of the β chain. As a result, a mutant exhibiting GLOD activity was obtained. This amino acid sequence deletion type GLOD mutant requires no protease treatment for exhibiting activity. In one embodiment, the amino acid sequence deletion type GLOD mutant is not subjected to protease treatment when recombinantly expressed.

The present inventors further continued development and prepared connected region substitution type mutants by replacing said boundary sequence (γ chain region ... QQW-GVRP... β chain region) with various amino acid sequences. More specifically, a mutant M7GLODΔ49C having the amino acid sequence of SEQ ID NO: 59, a mutant StGLODΔ49Ai having the amino acid sequence of SEQ ID NO: 12, or a mutant StGLODΔ49C having the amino acid sequence of SEQ ID NO: 13 was prepared as a mutant comprising a modification of said boundary sequence (γ chain region ... QQW-GVRP... β chain region), and recombinant expression was carried out and the activity of the mutants were confirmed. As a result, surprisingly, all the mutants exhibited GLOD activity. These amino acid sequence deletion type GLOD mutants also do not require any protease treatment to exhibit activity.

The mutant M7GLODΔ49C having the amino acid sequence of SEQ ID NO: 59 may be viewed as 56 amino acids from the 456th to 511th positions of M7GLOD having the amino acid sequence of SEQ ID NO: 58 being deleted and 7 amino acids (QDAAEPP) different from the sequence of the M7GLOD being inserted into the positions. In other words, in M7GLODΔ49C, the 456th to 504th positions of SEQ ID NO: 58 are deleted and the 505th to 511th positions are substituted with QDAAEPP. Alternatively, in M7GLODΔ49C, the 457th to 505th positions of SEQ ID NO: 58 are deleted, the 456th position is substituted with Q, and the 506th to 511th positions are substituted with DAAEPP. Alternatively, in M7GLODΔ49C, the 458th to 506th positions of SEQ ID NO: 58 are deleted, the 456th to 457th positions are substituted with QD, and the 507th to 511th positions are substituted with AAEPP. Alternatively, in M7GLODΔ49C, the 459th to 507th positions of SEQ ID NO: 58 are deleted, the 456th to 458th positions are substituted with QDA, and the 508th to 511th positions are substituted with AEPP. Alternatively, in M7GLODΔ49C, the 460th to 508th positions of SEQ ID NO: 58 are deleted, the 456th to 459th positions are substituted with QDAA, and the 509th to 511th positions are substituted with EPP. Alternatively, in M7GLODΔ49C, the 461st to 509th positions of SEQ ID NO: 58 are deleted, the 456th to 460th positions are substituted with QDAAE, and the 510th to 511th positions are substituted with PP. Alternatively, in M7GLODΔ49C, the 462nd to 510th positions of SEQ ID NO: 58 are deleted, the 456th to 461st positions are substituted with QDAAEP, and the 511th position is substituted with P. Alternatively, in M7GLODΔ49C, the 462nd to 510th positions of SEQ ID NO: 58 are deleted and the 456th to 462nd positions are substituted with QDAAEPP.

The mutant StGLODΔ49Ai having the amino acid sequence of SEQ ID NO: 12 may be viewed as 56 amino acids from the 456th to 511th positions of StGLOD having the amino acid sequence of SEQ ID NO: 1 being deleted and 7 amino acids (RKLDKTE) different from the sequence of StGLOD being inserted into the positions. In other words, in StGLODΔ49Ai, the 456th to 504th positions of SEQ ID NO: 1 are deleted and the 505th to 511th positions are substituted with RKLDKTE. Alternatively, in StGLODΔ49Ai, the 457th to 505th positions of SEQ ID NO: 1 are deleted, the 456th position is substituted with R, and the 506th to 511th positions are substituted with KLDKTE. Alternatively, in StGLODΔ49Ai, the 458th to 506th positions of SEQ ID NO: 1 are deleted, the 456th to 457th positions are substituted with RK, and the 507th to 511th positions are substituted with LDKTE. Alternatively, in StGLODΔ49Ai, the 459th to 507th positions of SEQ ID NO: 1 are deleted, the 456th to 458th positions are substituted with RKL, and the 508th to 511th positions are substituted with DKTE. Alternatively, in StGLODΔ49Ai, the 460th to 508th positions of SEQ ID NO: 1 are deleted, the 456th to 459th positions are substituted with RKLD, and the 509th to 511th positions are substituted with KTE. Alternatively, in StGLODΔ49Ai, the 461st to 509th positions of SEQ ID NO: 1 are deleted, the 456th to 460th positions are substituted with RKLDK, and the 510th to 511th positions are substituted with TE. Alternatively, in StGLODΔ49Ai, the 462nd to 510th positions of SEQ ID NO: 1 are deleted, the 456th to 461st positions are substituted with RKLDKT, and the 511th position is substituted with E. Alternatively, in StGLODΔ49Ai, the 462nd to 510th positions of SEQ ID NO: 1 are deleted and the 456th to 462nd positions are substituted with RKLDKTE.

The mutant StGLODΔ49C having the amino acid sequence of SEQ ID NO: 13 may be viewed as 56 amino acids from the 456th to 511th positions of StGLOD having the amino acid sequence of SEQ ID NO: 1 being deleted and 7 amino acids (QDAAEPP) different from the sequence of StGLOD being inserted into the positions. In other words, in StGLODΔ49C, the 456th to 504th positions of SEQ ID NO: 1 are deleted and the 505th to 511th positions are substituted with QDAAEPP. Alternatively, in StGLODΔ49C, the 457th to 505th positions of SEQ ID NO: 1 are deleted, the 456th position is substituted with Q, and the 506th to 511th positions are substituted with DAAEPP. Alternatively, in StGLODΔ49C, the 458th to 506th positions of SEQ ID NO: 1 are deleted, the 456th to 457th positions are substituted with QD, and the 507th to 511th positions are substituted with AAEPP. Alternatively, in StGLODΔ49C, the 459th to 507th positions of SEQ ID NO: 1 are deleted, the 456th to 458th positions are substituted with QDA, and the 508th to 511th positions are substituted with AEPP. Alternatively, in StGLODΔ49C, the 460th to 508th positions of SEQ ID NO: 1 are deleted, the 456th to 459th positions are substituted with QDAA, and the 509th to 511th positions are substituted with EPP. Alternatively, in StGLODΔ49C, the 461st to 509th positions of SEQ ID NO: 1 are deleted, the 456th to 460th positions are substituted with QDAAE, and the 510th to 511th positions are substituted with PP. Alternatively, in StGLODΔ49C, the 462nd to 510th positions of SEQ ID NO: 1 are deleted, the 456th to 461st positions are substituted with QDAAEP, and the 511th position is substituted with P. Alternatively, in StGLODΔ49C, the 462nd to 510th positions of SEQ ID NO: 1 are deleted and the 456th to 462nd positions are substituted with QDAAEPP.

The present inventors further continued development and prepared a plurality of mutants by altering the length of the region to be deleted. In one embodiment, the present disclosure provides

a glutamate oxidase mutant having a deleted region, wherein
all or part of an inter-γ-β region located between the γ chain region and the β chain region of a wild type glutamate oxidase is deleted,
1 to 10 carboxy terminal amino acids of the γ chain region of the wild type glutamate oxidase are deleted or not deleted,
1 to 4 amino terminal amino acids of the β chain region of the wild type glutamate oxidase are deleted or not deleted,
the deleted region is one continuous deleted region, and the glutamate oxidase mutant comprises glutamate oxidase activity.

The present inventors further continued development and prepared a large number of mutants differing in the length of the region to be deleted. In one embodiment, in a deletion mutant,
the positions corresponding to the 457th to 510th positions of SEQ ID NO: 1 are deleted (dΔ54),

the positions corresponding to the 458th to 5 10th positions of SEQ ID NO: 1 are deleted (dΔ53),
the positions corresponding to the 459th to 5 10th positions of SEQ ID NO: 1 are deleted (dΔ52),
the positions corresponding to the 460th to 5 10th positions of SEQ ID NO: 1 are deleted (dΔ51),
the positions corresponding to the 461th to 510th positions of SEQ ID NO: 1 are deleted (dΔ50),
the positions corresponding to the 462th to 5 10th positions of SEQ ID NO: 1 are deleted (dΔ49),
the positions corresponding to the 463th to 5 10th positions of SEQ ID NO: 1 are deleted (dΔ48),
the positions corresponding to the 464th to 5 10th positions of SEQ ID NO: 1 are deleted (dΔ47),
the positions corresponding to the 465th to 5 10th positions of SEQ ID NO: 1 are deleted (dΔ46),
the positions corresponding to the 466th to 5 10th positions of SEQ ID NO: 1 are deleted (dΔ45),
the positions corresponding to the 467th to 5 10th positions of SEQ ID NO: 1 are deleted (dΔ44),
the positions corresponding to the 468th to 5 10th positions of SEQ ID NO: 1 are deleted (dΔ43),
the positions corresponding to the 469th to 5 10th positions of SEQ ID NO: 1 are deleted (dΔ42),
the positions corresponding to the 470th to 510th positions of SEQ ID NO: 1 are deleted (dΔ41),
the positions corresponding to the 471th to 510th positions of SEQ ID NO: 1 are deleted (dΔ40),
the positions corresponding to the 472th to 510th positions of SEQ ID NO: 1 are deleted (dΔ39),
the positions corresponding to the 473th to 510th positions of SEQ ID NO: 1 are deleted (dΔ38),
the positions corresponding to the 474th to 510th positions of SEQ ID NO: 1 are deleted (dΔ37),
the positions corresponding to the 475th to 510th positions of SEQ ID NO: 1 are deleted (dΔ36),
the positions corresponding to the 476th to 510th positions of SEQ ID NO: 1 are deleted (dΔ35),
the positions corresponding to the 477th to 510th positions of SEQ ID NO: 1 are deleted (dΔ34),

the positions corresponding to the 478th to 510th positions of SEQ ID NO: 1 are deleted (dΔ33),
the positions corresponding to the 479th to 510th positions of SEQ ID NO: 1 are deleted (dΔ32), or
the positions corresponding to the 480th to 510th positions of SEQ ID NO: 1 are deleted (dΔ31).

In another embodiment, in a deletion mutant,

the positions corresponding to the 455th to 508th positions of SEQ ID NO: 1 are deleted (DΔ54),
the positions corresponding to the 456th to 508th positions of SEQ ID NO: 1 are deleted (DΔ53),
the positions corresponding to the 457th to 508th positions of SEQ ID NO: 1 are deleted (DΔ52),
the positions corresponding to the 458th to 508th positions of SEQ ID NO: 1 are deleted (DΔ51),
the positions corresponding to the 459th to 508th positions of SEQ ID NO: 1 are deleted (DΔ50),
the positions corresponding to the 460th to 508th positions of SEQ ID NO: 1 are deleted (DΔ49),
the positions corresponding to the 461th to 508th positions of SEQ ID NO: 1 are deleted (DΔ48),
the positions corresponding to the 462th to 508th positions of SEQ ID NO: 1 are deleted (DΔ47),
the positions corresponding to the 463th to 508th positions of SEQ ID NO: 1 are deleted (DΔ46),
the positions corresponding to the 464th to 508th positions of SEQ ID NO: 1 are deleted (DΔ45),
the positions corresponding to the 465th to 508th positions of SEQ ID NO: 1 are deleted (DΔ44),
the positions corresponding to the 466th to 508th positions of SEQ ID NO: 1 are deleted (DΔ43),
the positions corresponding to the 467th to 508th positions of SEQ ID NO: 1 are deleted (DΔ42),
the positions corresponding to the 468th to 508th positions of SEQ ID NO: 1 are deleted (DΔ41),
the positions corresponding to the 469th to 508th positions of SEQ ID NO: 1 are deleted (DΔ40),
the positions corresponding to the 470th to 508th positions of SEQ ID NO: 1 are deleted (DΔ39),
the positions corresponding to the 471th to 508th positions of SEQ ID NO: 1 are deleted (DΔ38),
the positions corresponding to the 472th to 508th positions of SEQ ID NO: 1 are deleted (DΔ37),
the positions corresponding to the 473th to 508th positions of SEQ ID NO: 1 are deleted (DΔ36),
the positions corresponding to the 474th to 508th positions of SEQ ID NO: 1 are deleted (DΔ35),
the positions corresponding to the 475th to 508th positions of SEQ ID NO: 1 are deleted (DΔ34),
the positions corresponding to the 476th to 508th positions of SEQ ID NO: 1 are deleted (DΔ33),
the positions corresponding to the 477th to 508th positions of SEQ ID NO: 1 are deleted (DΔ32), or
the positions corresponding to the 478th to 508th positions of SEQ ID NO: 1 are deleted (DΔ31).

In one embodiment, the present disclosure provides a mutant comprising a deletion of RWGEDDAEAALTVP ESVRNLPTGLLGAHPSVDEQLIDDEQVEYLRNSTLRGGVR with reference to SEQ ID NO: 58. For convenience, this deletion mutant may also be referred to as M7GLOD-dΔ54 in the present specification. In one embodiment, the present disclosure provides a mutant in which any of the following deleted regions differing in chain length with reference to SEQ ID NO: 58 is deleted.

[Table 1]

| |
|---|
| RWGEDDAEAALTVPESVRNLPTGLLGAHPSVDEQLIDDEQVEYLRNSTLRGGVR (M7GLOD-dΔ54) |
| WGEDDAEAALTVPESVRNLPTGLLGAHPSVDEQLIDDEQVEYLRNSTLRGGVR (M7GLOD-dΔ53) |
| GEDDAEAALTVPESVRNLPTGLLGAHPSVDEQLIDDEQVEYLRNSTLRGGVR (M7GLOD-dΔ52) |
| EDDAEAALTVPESVRNLPTGLLGAHPSVDEQLIDDEQVEYLRNSTLRGGVR (M7GLOD-dΔ51) |
| DDAEAALTVPESVRNLPTGLLGAHPSVDEQLIDDEQVEYLRNSTLRGGVR (M7GLOD-dΔ50) |
| DAEAALTVPESVRNLPTGLLGAHPSVDEQLIDDEQVEYLRNSTLRGGVR (M7GLOD-dΔ49) |
| AEAALTVPESVRNLPTGLLGAHPSVDEQLIDDEQVEYLRNSTLRGGVR(M7GLOD-dΔ48) |
| EAALTVPESVRNLPTGLLGAHPSVDEQLIDDEQVEYLRNSTLRGGVR(M7GLOD-dΔ47) |
| AALTVPESVRNLPTGLLGAHPSVDEQLIDDEQVEYLRNSTLRGGVR(M7GLOD-dΔ46) |
| ALTVPESVRNLPTGLLGAHPSVDEQLIDDEQVEYLRNSTLRGGVR(M7GLOD-dΔ45) |
| LTVPESVRNLPTGLLGAHPSVDEQLIDDEQVEYLRNSTLRGGVR(M7GLOD-dΔ44) |
| TVPESVRNLPTGLLGAHPSVDEQLIDDEQVEYLRNSTLRGGVR(M7GLOD-dΔ43) |
| VPESVRNLPTGLLGAHPSVDEQLIDDEQVEYLRNSTLRGGVR(M7GLOD-dΔ42) |
| PESVRNLPTGLLGAHPSVDEQLIDDEQVEYLRNSTLRGGVR(M7GLOD-dΔ41) |
| ESVRNLPTGLLGAHPSVDEQLIDDEQVEYLRNSTLRGGVR(M7GLOD-dΔ40) |
| SVRNLPTGLLGAHPSVDEQLIDDEQVEYLRNSTLRGGVR(M7GLOD-dΔ39) |
| VRNLPTGLLGAHPSVDEQLIDDEQVEYLRNSTLRGGVR(M7GLOD-dΔ38) |

(continued)

| |
|---|
| RNLPTGLLGAHPSVDEQLIDDEQVEYLRNSTLRGGVR(M7GLOD-dΔ37) |
| NLPTGLLGAHPSVDEQLIDDEQVEYLRNSTLRGGVR(M7GLOD-dΔ36) |
| LPTGLLGAHPSVDEQLIDDEQVEYLRNSTLRGGVR(M7GLOD-dΔ35) |
| PTGLLGAHPSVDEQLIDDEQVEYLRNSTLRGGVR(M7GLOD-dΔ34) |
| TGLLGAHPSVDEQLIDDEQVEYLRNSTLRGGVR(M7GLOD-dΔ33) |
| GLLGAHPSVDEQLIDDEQVEYLRNSTLRGGVR(M7GLOD-dΔ32) |
| LLGAHPSVDEQLIDDEQVEYLRNSTLRGGVR(M7GLOD-dΔ31) |

In another embodiment, the present disclosure provides a mutant in which any of the following deleted regions differing in chain length with reference to SEQ ID NO: 58 is deleted.

[Table 2]

| |
|---|
| YQRWGEDDAEAALTVPESVRNLPTGLLGAHPSVDEQLIDDEQVEYLRNSTLRGG (M7GLOD-DΔ54) |
| QRWGEDDAEAALTVPESVRNLPTGLLGAHPSVDEQLIDDEQVEYLRNSTLRGG (M7GLOD-DΔ53) |
| RWGEDDAEAALTVPESVRNLPTGLLGAHPSVDEQLIDDEQVEYLRNSTLRGG (M7GLOD-DΔ52) |
| WGEDDAEAALTVPESVRNLPTGLLGAHPSVDEQLIDDEQVEYLRNSTLRGG (M7GLOD-DΔ51) |
| GEDDAEAALTVPESVRNLPTGLLGAHPSVDEQLIDDEQVEYLRNSTLRGG(M7GLOD-DΔ50) |
| EDDAEAALTVPESVRNLPTGLLGAHPSVDEQLIDDEQVEYLRNSTLRGG(M7GLOD-DΔ49) |
| DDAEAALTVPESVRNLPTGLLGAHPSVDEQLIDDEQVEYLRNSTLRGG(M7GLOD-DΔ48) |
| DAEAALTVPESVRNLPTGLLGAHPSVDEQLIDDEQVEYLRNSTLRGG(M7GLOD-DΔ47) |
| AEAALTVPESVRNLPTGLLGAHPSVDEQLIDDEQVEYLRNSTLRGG(M7GLOD-DΔ46) |
| EAALTVPESVRNLPTGLLGAHPSVDEQLIDDEQVEYLRNSTLRGG(M7GLOD-DΔ45) |
| AALTVPESVRNLPTGLLGAHPSVDEQLIDDEQVEYLRNSTLRGG(M7GLOD-DΔ44) |
| ALTVPESVRNLPTGLLGAHPSVDEQLIDDEQVEYLRNSTLRGG(M7GLOD-DΔ43) |
| LTVPESVRNLPTGLLGAHPSVDEQLIDDEQVEYLRNSTLRGG(M7GLOD-DΔ42) |
| TVPESVRNLPTGLLGAHPSVDEQLIDDEQVEYLRNSTLRGG(M7GLOD-DΔ41) |
| VPESVRNLPTGLLGAHPSVDEQLIDDEQVEYLRNSTLRGG(M7GLOD-DΔ40) |
| PESVRNLPTGLLGAHPSVDEQLIDDEQVEYLRNSTLRGG(M7GLOD-DΔ39) |
| ESVRNLPTGLLGAHPSVDEQLIDDEQVEYLRNSTLRGG(M7GLOD-DΔ38) |
| SVRNLPTGLLGAHPSVDEQLIDDEQVEYLRNSTLRGG(M7GLOD-DΔ37) |
| VRNLPTGLLGAHPSVDEQLIDDEQVEYLRNSTLRGG(M7GLOD-DΔ36) |
| RNLPTGLLGAHPSVDEQLIDDEQVEYLRNSTLRGG(M7GLOD-DΔ35) |
| NLPTGLLGAHPSVDEQLIDDEQVEYLRNSTLRGG(M7GLOD-DΔ34) |
| LPTGLLGAHPSVDEQLIDDEQVEYLRNSTLRGG(M7GLOD-DΔ33) |
| PTGLLGAHPSVDEQLIDDEQVEYLRNSTLRGG(M7GLOD-DΔ32) |
| TGLLGAHPSVDEQLIDDEQVEYLRNSTLRGG(M7GLOD-DΔ31) |

In one embodiment, the present disclosure provides a mutant in which any of the following deleted regions differing in chain length with reference to SEQ ID NO: 1 is deleted.

[Table 3]

| |
|---|
| QWGEDDAEAALALPQSVRNLPTGLLGAHPSVDESRIGEEQVEYYRNSELRGGVR (StGLOD-dΔ54) |

(continued)

| |
|---|
| WGEDDAEAALALPQSVRNLPTGLLGAHPSVDESRIGEEQVEYYRNSELRGGVR (StGLOD-dΔ53) |
| GEDDAEAALALPQSVRNLPTGLLGAHPSVDESRIGEEQVEYYRNSELRGGVR (StGLOD-dΔ52) |
| EDDAEAALALPQSVRNLPTGLLGAHPSVDESRIGEEQVEYYRNSELRGGVR (StGLOD-dΔ51) |
| DDAEAALALPQSVRNLPTGLLGAHPSVDESRIGEEQVEYYRNSELRGGVR (StGLOD-dΔ50) |
| DAEAALALPQSVRNLPTGLLGAHPSVDESRIGEEQVEYYRNSELRGGVR (StGLOD-dΔ49) |
| AEAALALPQSVRNLPTGLLGAHPSVDESRIGEEQVEYYRNSELRGGVR (StGLOD-dΔ48) |
| EAALALPQSVRNLPTGLLGAHPSVDESRIGEEQVEYYRNSELRGGVR (StGLOD-dΔ47) |
| AALALPQSVRNLPTGLLGAHPSVDESRIGEEQVEYYRNSELRGGVR (StGLOD-dΔ46) |
| ALALPQSVRNLPTGLLGAHPSVDESRIGEEQVEYYRNSELRGGVR (StGLOD-dΔ45) |
| LALPQSVRNLPTGLLGAHPSVDESRIGEEQVEYYRNSELRGGVR (StGLOD-dΔ44) |
| ALPQSVRNLPTGLLGAHPSVDESRIGEEQVEYYRNSELRGGVR (StGLOD-dΔ43) |
| LPQSVRNLPTGLLGAHPSVDESRIGEEQVEYYRNSELRGGVR (StGLOD-dΔ42) |
| PQSVRNLPTGLLGAHPSVDESRIGEEQVEYYRNSELRGGVR (StGLOD-dΔ41) |
| QSVRNLPTGLLGAHPSVDESRIGEEQVEYYRNSELRGGVR (StGLOD-dΔ40) |
| SVRNLPTGLLGAHPSVDESRIGEEQVEYYRNSELRGGVR (StGLOD-dΔ39) |
| VRNLPTGLLGAHPSVDESRIGEEQVEYYRNSELRGGVR (StGLOD-dΔ38) |
| RNLPTGLLGAHPSVDESRIGEEQVEYYRNSELRGGVR (StGLOD-dΔ37) |
| NLPTGLLGAHPSVDESRIGEEQVEYYRNSELRGGVR (StGLOD-dΔ36) |
| LPTGLLGAHPSVDESRIGEEQVEYYRNSELRGGVR (StGLOD-dΔ35) |
| PTGLLGAHPSVDESRIGEEQVEYYRNSELRGGVR (StGLOD-dΔ34) |
| TGLLGAHPSVDESRIGEEQVEYYRNSELRGGVR (StGLOD-dΔ33) |
| GLLGAHPSVDESRIGEEQVEYYRNSELRGGVR (StGLOD-dΔ32) |
| LLGAHPSVDESRIGEEQVEYYRNSELRGGVR (StGLOD-dΔ31) |

In another embodiment, the present disclosure provides a mutant in which any of the following deleted regions differing in chain length with reference to SEQ ID NO: 1 is deleted.

[Table 4]

| |
|---|
| YQQWGEDDAEAALALPQSVRNLPTGLLGAHPSVDESRIGEEQVEYYRNSELRGG (StGLOD-DΔ54) |
| QQWGEDDAEAALALPQSVRNLPTGLLGAHPSVDESRIGEEQVEYYRNSELRGG (StGLOD-DΔ53) |
| QWGEDDAEAALALPQSVRNLPTGLLGAHPSVDESRIGEEQVEYYRNSELRGG (StGLOD-DΔ52) |
| WGEDDAEAALALPQSVRNLPTGLLGAHPSVDESRIGEEQVEYYRNSELRGG (StGLOD-DΔ51) |
| GEDDAEAALALPQSVRNLPTGLLGAHPSVDESRIGEEQVEYYRNSELRGG (StGLOD-DΔ50) |
| EDDAEAALALPQSVRNLPTGLLGAHPSVDESRIGEEQVEYYRNSELRGG (StGLOD-DΔ49) |
| DDAEAALALPQSVRNLPTGLLGAHPSVDESRIGEEQVEYYRNSELRGG (StGLOD-DΔ48) |
| DAEAALALPQSVRNLPTGLLGAHPSVDESRIGEEQVEYYRNSELRGG (StGLOD-DΔ47) |
| AEAALALPQSVRNLPTGLLGAHPSVDESRIGEEQVEYYRNSELRGG (StGLOD-DΔ46) |
| EAALALPQSVRNLPTGLLGAHPSVDESRIGEEQVEYYRNSELRGG (StGLOD-DΔ45) |
| AALALPQSVRNLPTGLLGAHPSVDESRIGEEQVEYYRNSELRGG (StGLOD-DΔ44) |
| ALALPQSVRNLPTGLLGAHPSVDESRIGEEQVEYYRNSELRGG (StGLOD-DΔ43) |
| LALPQSVRNLPTGLLGAHPSVDESRIGEEQVEYYRNSELRGG (StGLOD-DΔ42) |

(continued)

| |
|---|
| ALPQSVRNLPTGLLGAHPSVDESRIGEEQVEYYRNSELRGG (StGLOD-DΔ41) |
| LPQSVRNLPTGLLGAHPSVDESRIGEEQVEYYRNSELRGG (StGLOD-DΔ40) |
| PQSVRNLPTGLLGAHPSVDESRIGEEQVEYYRNSELRGG (StGLOD-DΔ39) |
| QSVRNLPTGLLGAHPSVDESRIGEEQVEYYRNSELRGG (StGLOD-DΔ38) |
| SVRNLPTGLLGAHPSVDESRIGEEQVEYYRNSELRGG (StGLOD-DΔ37) |
| VRNLPTGLLGAHPSVDESRIGEEQVEYYRNSELRGG (StGLOD-DΔ36) |
| RNLPTGLLGAHPSVDESRIGEEQVEYYRNSELRGG (StGLOD-DΔ35) |
| NLPTGLLGAHPSVDESRIGEEQVEYYRNSELRGG (StGLOD-DΔ34) |
| LPTGLLGAHPSVDESRIGEEQVEYYRNSELRGG (StGLOD-DΔ33) |
| PTGLLGAHPSVDESRIGEEQVEYYRNSELRGG (StGLOD-DΔ32) |
| TGLLGAHPSVDESRIGEEQVEYYRNSELRGG (StGLOD-DΔ31) |

The above mutants are encompassed by the glutamate oxidase mutant wherein 31 to 54 continuous amino acids are deleted, as referred to in the present specification.

In one embodiment, the C terminus of the deleted region can be located at the position corresponding to the 512th, 511th, 510th, 509th, 508th, 507th, 506th, or 505th position of SEQ ID NO: 1. In one embodiment, the C terminus of the deleted region can be located at the position corresponding to the 510th, 508th, or 507th position of SEQ ID NO: 1. For convenience, the position of the C terminus of the deleted region may also be referred to as a point of origin in the present specification. In one embodiment, in the deletion mutant, 31, 32, 33, 34, 35, 36, 37, 38, 39, 40, 41, 42, 43, 44, 45, 46, 47, 48, 49, 50, 51, 52, 53 or 54 continuous amino acids from the point of origin may be deleted. Since the point of origin is deleted, the glutamate oxidase deletion mutant lacks the point of origin. Further, the glutamate oxidase deletion mutant does not have 31 to 54 continuous amino acids from the point of origin (that is, does not have 31 to 54 amino acids including the amino acid serving as the point of origin).

Without wishing to be bound by any specific theory, the inter-γ-β region of the glutamate oxidase is excised by a protease in nature. As such, it is believed that, even if the excised region is deleted in advance from a recombinantly expressed glutamate oxidase, a glutamate oxidase having activity may likewise be obtained. In this respect, it is believed that the excised region is unlikely to play a role in the active protein since the excised region is absent in the mature protein. As such, with regard to the amino acid sequence of the excised region, when a deletion mutant comprising a deletion of a long sequence and a deletion mutant comprising a deletion of a short sequence are both confirmed to have activity (for example, when a Δ54 mutant and a Δ31 mutant are both confirmed to have activity), it is rationally believed that it is highly plausible that a mutant comprising a deletion of a region having an intermediate length (for example, Δ53 to Δ32 mutants) will also exhibit activity.

When a mutant was prepared, GLOD activity was confirmed. Based on the disclosure of the present specification, those skilled in the art can prepare a similar deletion mutant and confirm the presence or absence of activity thereof by routine tests. Deletion mutants having no activity are excluded from the glutamate oxidase mutant having glutamate oxidase activity.

In one embodiment, the present disclosure provides an amino acid sequence deletion type GLOD mutant wherein the amino acid sequence of the region corresponding to the 459th to 507th positions of SEQ ID NO: 1 is deleted and the mutant comprises glutamate oxidase activity. In a particular embodiment, in the mutant, the region corresponding to the 670th to 687th positions may be further deleted.

In one embodiment, the present disclosure provides an amino acid sequence deletion type GLOD mutant wherein the amino acid sequence of the region corresponding to the 459th to 507th positions of SEQ ID NO: 1 is deleted, the mutant comprises glutamate oxidase activity, and thermal stability is improved compared to a glutamate oxidase in which all or part of the region corresponding to the 459th to 507th positions of SEQ ID NO: 1 is not deleted. In a particular embodiment, in the mutant, the region corresponding to the 670th to 687th positions may be further deleted.

In one embodiment, the present disclosure provides an amino acid sequence deletion type GLOD mutant wherein the amino acid sequence of the region corresponding to the 459th to 507th positions of SEQ ID NO: 1 is deleted, the mutant comprises glutamate oxidase activity, and thermal stability is improved compared to a glutamate oxidase in which the region corresponding to the 459th to 466th positions of SEQ ID NO: 1 is not deleted. In a particular embodiment, in the mutant, the region corresponding to the 670th to 687th positions may be further deleted.

In one embodiment, the deletion mutant may be prepared based on SEQ ID NO: 58. In one embodiment, the deletion mutant may be prepared based on a glutamate oxidase having a sequence identity of 90% or more, for example, 91% or more, 92% or more, 93% or more, 94% or more, 95% or more, 96% or more, 97% or more, 98% or more, or 99% or more with SEQ ID NO: 58.

In another embodiment, the deletion mutant may be prepared based on SEQ ID NO: 1. In one embodiment, the deletion mutant may be prepared based on a glutamate oxidase having a sequence identity of 90% or more, for example, 91% or more, 92% or more, 93% or more, 94% or more, 95% or more, 96% or more, 97% or more, 98% or more, or 99% or more with SEQ ID NO: 1.

In an alternative embodiment, the deletion mutant may be prepared based on a known glutamate oxidase or a glutamate oxidase having a sequence identity of 90% or more, for example, 91% or more, 92% or more, 93% or more, 94% or more, 95% or more, 96% or more, 97% or more, 98% or more, or 99% or more therewith.

(Thermal stability improving effect)

As a result of subjecting these the amino acid sequence deletion type GLOD mutants to heat treatment, surprisingly, the residual activities of all mutants were improved compared to their corresponding GLOD sequences before the amino acid sequence deletion. Residual activity refers to the value of activity of a GLOD sample after heat treatment when the activity of a refrigerated (for example, 4°C) GLOD sample is defined as 1. The residual activity may be a value of 0 or more and 1 or less but also may be a value that exceeds 1 when the GLOD is activated by heat treatment.

In the present specification, mutants comprising a deletion of the amino acid sequence between the γ and β chains, such as StGLODΔ49C, M7GLODΔ49C, and StGLODΔ49Ai, and the mutants described in Tables 1 to 4 may also be referred to as inter-γ-β chain amino acid sequence deletion type GLOD mutants or simply as amino acid sequence deletion type GLOD mutants. The amino acid sequence deletion type GLOD mutant may also be referred to as "GLODΔ". The number of deleted amino acid residues, XX, may be described following the GLODΔ (GLODΔXX). For example, a GLOD mutant in which 49 amino acid residues are deleted may also be referred to as GLODΔ49. A deletion mutant in which the position corresponding to the 5 10th position of SEQ ID NO: 1 is the point of origin may also be referred to as "GLOD-dΔ". For example, a mutant based on SEQ ID NO: 58, in which the position corresponding to the 5 10th position of SEQ ID NO: 1 is the point of origin and 54 amino acids are deleted may also be referred to as M7GLOD-dΔ54. Further, a deletion mutant in which the position corresponding to the 508th position of SEQ ID NO: 1 is the point of origin may also be referred to as "GLOD-DΔ". For example, a mutant based on SEQ ID NO: 58, in which the position corresponding to the 508th position of SEQ ID NO: 1 is the point of origin and 54 amino acids are deleted may also be referred to as M7GLOD-DΔ54. In the present specification, the term amino acid sequence deletion type GLOD mutant encompasses not only StGLOD and M7GLOD but their variants, GLODs having an amino acid sequence identity of 90% or more therewith, and GLOD mutants of other origins in which the region corresponding to the deleted region of SEQ ID NO: 1 or SEQ ID NO: 12 or 13 is deleted.

In one embodiment, with regard to the GLOD mutant of the present disclosure, the residual activity after heat treatment of the modified GLOD mutant may be improved by, for example, 10% or more, 20% or more, 30% or more, 40% or more, 50% or more, 60% or more, 70% or more, 80% or more, 90% or more, 100% or more, 150% or more, 200% or more, 250% or more, 300% or more, 350% or more, for example, 400% or more when the residual activity after heat treatment of a GLOD before modification is defined as 100%. Here, the phrase "the residual activity is improved by 10%" means that the residual activity after heat treatment of the modified GLOD mutant is 110% when the residual activity after heat treatment of a GLOD before modification is defined as 100%.

In one embodiment, the thermal stability of the GLOD mutant of the present disclosure may be evaluated from residual activity after heat treatment at 45°C for 30 minutes or 35 minutes or heat treatment at 65°C for 30 minutes. In one embodiment, the residual activity of a GLODΔ mutant comprising a deletion of the region corresponding to a predetermined region of SEQ ID NO: 1 after being subjected to heat treatment at 45°C for 30 minutes or 35 minutes may be 105% or more, 110% or more, 120% or more, 130% or more, 140% or more, 150% or more, 200% or more, 300% or more, for example, 400% or more when the residual activity of a GLOD comprising no deletion of the predetermined region after being subjected to the same treatment as above is defined as 100%. The predetermined region to be deleted may have 31 to 54 continuous amino acids (including the point of origin) whose C terminal point of origin can be the position corresponding to the 510th, 508th, or 507th position of SEQ ID NO: 1. In one embodiment, the residual activity of a GLODΔ mutant in which the region corresponding to the 459th to 507th positions of SEQ ID NO: 1 is deleted after being subjected to heat treatment at 45°C for 30 minutes or 35 minutes may be 105% or more, 110% or more, 120% or more, 130% or more, 140% or more, 150% or more, 200% or more, 300% or more, for example, 400% or more when the residual activity of a GLOD in which all or part of the 459th to 507th positions of SEQ ID NO: 1 is not deleted after being subjected to the same treatment as above is defined as 100%. In another embodiment, the residual activity of a GLODΔ mutant in which the region corresponding to the 459th to 507th positions of SEQ ID NO: 1 is deleted after being subjected to heat treatment at 45°C for 30 minutes or 35 minutes may be 105% or more, 110% or more, 120% or more, 130% or more, 140% or more, 150% or more, 200% or more, 300% or more, for example, 400% or more when the residual activity of a GLOD in which the amino acid sequence from the 459th to 466th positions of SEQ ID NO: 1 is not deleted after being subjected to the same treatment as above is defined as 100%. In a particular embodiment, in the mutant, the region corresponding to the 670th to 687th positions may be further deleted. In an alternative embodiment, the residual activity of a GLODΔ mutant in which all or part of the region corresponding to the 457th to 510 positions of SEQ ID NO: 1 (for example, a region consisting of 31 to 54 continuous amino acids whose C terminal point of origin is the position corresponding to the 510th, 508th, or 507th position of SEQ ID NO: 1) is deleted after being subjected to heat treatment at 65°C for 30 minutes may be 105% or more, 110% or more, 120% or more, 130% or more, 140% or more, 150% or more, 200% or more, 300% or more, for example, 400% or more when the residual activity of a GLOD having no such deletion after being subjected to the same treatment as above is defined as 100%. In a particular embodiment, in the mutant, the region corresponding to the 670th to 687th positions may be further deleted.

The present inventors found that the thermal stability of a GLODΔ mutant in which the region corresponding to the 459th to 507th positions of SEQ ID NO: 1, or a predetermined region consisting of 31 to 54 continuous amino acids whose C terminal point of origin is the position corresponding to the 510th, 508th, or 507th position of SEQ ID NO: 1, is deleted, is improved compared to a GLOD before modification. Based on these findings, those skilled in the art shall appreciate that the thermal stability of other GLODs having a sequence identity of 90% or more or GLODs of other origins will also be improved by deleting the amino acid sequence of the corresponding region and these Δ mutants can be used in various reactions. The same applies to the region corresponding to the 670th to 687th positions of SEQ ID NO: 1.

In one embodiment, the present disclosure provides a GLODΔ having an amino acid sequence identity of 90% or more with the amino acid sequence of SEQ ID NO: 59, 12, or 13, comprising a deletion of a predetermined region, and having glutamate oxidase activity. The predetermined region may be a region consisting of 31 to 54 continuous amino acids whose C terminal point of origin is the position corresponding to the 510th, 508th, or 507th position of SEQ ID NO: 1. In this GLODΔ, the region corresponding to the 670th to 687th positions of SEQ ID NO: 1 may be further deleted.

GLODs are widely distributed in nature and can be obtained by searching for enzymes from sources of microorganisms, animals, or plants. With regard to microorganisms, GLODs can be obtained from, for example, streptomycetes, filamentous fungi, yeast, or bacteria. In the present specification, the GLOD is not particularly limited by its origin, and GLOD means a GLOD derived from, for example, a microorganism belonging to the genus *Streptomyces* such as Streptomyces sp. X-119-6 and *Streptomyces* sp. MOE7, the genus *Azotobacter,* the genus *Embleya,* the genus *Kitasatospora,* the genus *Saccharothrix,* the genus *Alloactinosynnema,* the genus *Streptoalloteichus,* the genus *Actinoalloteichus,* the genus *Catenulispora,* the genus *Nannocystis,* the genus *Actinobacteria,* the genus *Actinophytocola,* the genus *Sphaerisporangium,* the genus Microbispora, the genus *Streptosporangium,* the genus *Phytohabitans,* the genus *Haliangium,* the genus *Archangium,* the genus *Streptacidiphilus,* the genus *Saccharothrix* or the genus *Trichoderma,* and includes all wild type and variants thereof, unless specified otherwise.

(Obtaining a gene encoding GLOD)

In order to obtain a gene encoding a GLOD (hereinafter, also simply referred to as a "GLOD gene"), gene cloning methods carried out in the art in general can be used. For example, chromosomal DNA or mRNA can be extracted from microbial cells or various cells having GLOD productivity by routine methods. Further, cDNA can be synthesized using mRNA as the template. Using chromosomal DNA or cDNA thus obtained, a library of chromosomal DNA or cDNA can be prepared.

Next, an appropriate probe DNA can be synthesized based on the amino acid sequence of the GLOD above and a GLOD gene can be screened from the chromosomal DNA or cDNA library by using the probe DNA or, alternatively, appropriate primer DNA(s) can be prepared based on the amino acid sequence above and the DNA containing the gene fragment of interest encoding the GLOD can be amplified with an appropriate polymerase chain reaction (PCR method) such as the 5'RACE method and the 3'RACE method, and then, these DNA fragments can be linked to obtain a DNA containing the full-length GLOD gene of interest.

Examples of the GLOD gene include, but are not limited to, a GLOD gene from *Streptomyces* sp. X-119-6 and a GLOD gene from *Streptomyces* sp. MOE7.

The GLOD gene may be linked to a vector. Vectors include any vector, such as a plasmid, bacteriophage, or cosmid vector, and an example thereof is pBluescriptII SK+ (manufactured by Stratagene Corporation). A plasmid may be obtained using conventional techniques. For example, a plasmid comprising a GLOD gene may be extracted and purified with the use of the GenElute Plasmid Miniprep Kit (manufactured by Sigma-Aldrich). The obtained GLOD gene may be engineered to prepare a GLOD mutant gene or a purified enzyme can be obtained.

(Treatment for inducing mutation of GOLD gene)

Mutation of the GLOD gene can be performed by any known method depending on the intended form of mutation. That is, methods of bringing a chemical agent serving as a mutagen into contact with and allowing to act on a GLOD gene or recombinant DNA comprising said gene integrated therein; ultraviolet irradiation methods; genetic engineering techniques; methods of employing protein engineering procedures can be used extensively.

Examples of the chemical agent serving as the mutagen used in the above mutation treatment include hydroxylamine, N-methyl-N'-nitro-N-nitrosoguanidine, nitrous acid, sulfurous acid, hydrazine, formic acid or 5-bromouracil and the like.

The conditions for allowing a chemical agent to contact and act can be determined depending on the type of chemical agent being used and the like and the conditions are not particularly limited as long as the mutation of interest can actually be induced in the GLOD gene. A mutation of interest can be induced usually by allowing a chemical agent preferably having a concentration of 0.5 to 12 M to contact and act on (the gene) at a reaction temperature of 20 to 80°C for 10 minutes or more and preferably 10 to 180 minutes. In the case of ultraviolet irradiation, a mutation can be induced with a routine method as mentioned above.

As a method of employing protein engineering procedures, in general, the method known as Site-Specific Mutagenesis can be used. Examples thereof include the Kramer method (Nucleic Acids Res., 12, 9441-9456 (1984)), the Eckstein method (Nucleic Acids Res., 13, 8749 -8764 (1985):Nucleic Acids Res., 13, 8765 (1985): Nucleic Acids Res, 14, 9679 (1986)) and the Kunkel method (Proc. Natl. Acid. Sci. U.S.A., 82, 488-492 (1985)).

Incidentally, apart from the above gene modifying methods, a modified GDH gene of interest can be directly synthesized using organic synthesis methods or enzyme synthesis methods.

The nucleotide sequence of the GLOD gene may be confirmed using, for example, multi-capillary DNA analysis system Applied Biosystems 3730xlDNA analyzer (manufactured by Thermo Fisher Scientific Inc.).

(Transformation or transduction)

The GLOD gene can be integrated into a vector such as a bacteriophage, cosmid, or a plasmid for transformation of prokaryote cells or eukaryote cells according to routine methods. Using the resultant, the host cell corresponding to the vector may be transformed or transduced with routine methods.

In one embodiment, GLOD may be expressed using prokaryote cells, for example, microorganisms belonging to the genus *Escherichia* such as *Escherichia coli,* microorganisms belonging to the genus *Brevibacillus* such as *Brevibacillus choshinensis,* microorganisms belonging to the genus *Corynebacterium* such as *Corynebacterium glutamicum,* or microorganisms belonging to the genus *Streptomyces* such as *Streptomyces violaceoruber.* Examples of the *Escherichia coli* host include, but are not limited to, various *Escherichia coli* strains such as K-12 strain, JM109 strain, DH5a strain, BL21 strain, JM109(DE3) strain, DH5a(DE3) strain, BL21(DE3) strain, TG1 strain, 1100 strain, W3110 strain and C600 strain. The host can be transformed or transduced to obtain host cells having the GLOD gene introduced therein (transformants). As a method for introducing a recombinant vector into a host cell, if the host cell is a microorganism belonging to *Escherichia coli,* a method of transferring recombinant DNA in the presence of a calcium ion can be employed. Furthermore, an electroporation method may be used. Moreover, commercially available competent cells (for example, ECOS Competent *Escherichia coli* BL21 (DE3); manufactured by Nippon Gene Co., Ltd.) may be used. The GLOD gene may be a codon-optimized gene depending on the expression host.

In one embodiment, GLOD may be expressed using eukaryote cells. Examples of the eukaryotic host cell include yeast. Examples of microorganisms classified as yeast include yeasts belonging to the genus *Zygosaccharomyces,* the genus *Schizosaccharomyces,* the genus *Saccharomyces,* the genus *Pichia* and *the genus Candida.* The gene insert may contain a marker gene which enables selecting transformed cells. Examples of the marker gene include genes which compensate auxotrophy of a host cell, such as URA3 and TRP1. The gene insert may desirably contain a promoter enabling expression of the gene of interest in a host cell or other regulatory sequences (for example, enhancer sequence, terminator sequence, polyadenylation sequence and the like). Specific examples of the promoter include GAL1 promoter and ADH1 promoter. As methods for transforming yeast, known methods such as the method of using lithium acetate (Methods Mol. Cell. Biol., 5, 255-269 (1995)) as well as electroporation (J Microbiol Methods 55 (2003) 481-484) can suitably be used although the transformation method is not limited thereto and various methods including the spheroplast method and glass bead method can be used for transformation.

Other examples of the eukaryotic host cell include mold cells (including filamentous fungi) such as those of the genus *Aspergillus* and the genus *Tricoderma.* The method for preparing a transformant of a mold cell is not particularly limited and includes, for example, a method of inserting a gene encoding a GLOD into a host filamentous fungus with routine methods such that the gene encoding the GLOD is expressed. More specifically, a DNA construct is prepared by inserting a gene encoding a GLOD between an expression inducing promoter and a terminator; and then, a host filamentous fungus is transformed with the DNA construct containing the gene encoding the GLOD to obtain transformants overexpressing the gene encoding the GLOD.

Methods for inserting a gene encoding a GLOD into a host filamentous fungus such that the gene is expressed are not particularly limited and include, for example, a method of directly inserting the gene into the chromosome of a host organism by using homologous recombination; and a method of ligating the gene to a plasmid vector and introducing the vector to a host filamentous fungus.

In the method using homologous recombination, a DNA construct is inserted into the genome of the host filamentous fungus by ligating the DNA construct between sequences homologous to the upstream region and downstream region of a recombination site on the chromosome. By overexpressing the gene under control of its own high expression promotor in the host filamentous fungus a transformant by self-cloning can be obtained. Examples of the high expression promoter include, but are not particularly limited to, the promoter region of TEF1 gene (tef1) serving as a translation elongation factor, the promoter region of $\alpha$-amylase gene (amy) and the promoter region of an alkaline protease gene (alp).

In the method of using a vector, a DNA construct is integrated into a plasmid vector used in transformation of filamentous fungi by a routine method and then the corresponding host filamentous fungus can be transformed (with the plasmid vector) with a routine method.

Such suitable vector-host system is not particularly limited as long as the GLOD can be produced in the host filamentous fungus and includes, for example, pUC19 and filamentous fungus system, pSTA14 (Mol. Gen. Genet. 218, 99-104, 1989) and filamentous fungus system.

It is preferably to use the DNA construct by introducing the same into the chromosome of a host filamentous fungus. As an alternative method, the DNA construct can be integrated into an autonomous replicating vector (Ozeki et al. Biosci. Biotechnol. Biochem. 59, 1133 (1995)) and in this manner, the DNA construct can be used without being introduced into the chromosome.

The DNA construct may comprise a marker gene which enables a transformed cell to be selected. Examples of the marker gene include, but are not particularly limited to, genes compensating auxotrophy of a host such as pyrG, niaD, adeA; and drug resistance genes against chemical agents such as pyrithiamine, hygromycin B and oligomycin. Further, the DNA construct preferably comprises a promoter enabling overexpression of the gene encoding the GLOD in the host cell, a terminator and other regulatory sequences (for example, enhancer, polyadenylation sequence and the like). Examples of the promoter include, but are not particularly limited to, suitable expression induction promoters and constitutive promoters, such as the tef1 promoter, alp promoter, amy promoter and the like. Examples of the terminator include, but are not particularly limited to, the alp terminator, amy terminator and tef1 terminator and the like.

In the DNA construct, if the DNA fragment containing the gene encoding the GLOD to be inserted has a sequence having expression regulating function, then an expression regulatory sequence for the gene encoding the GLOD need not be required. When transformation is carried out by a co-transformation method, the DNA construct need not have a marker gene in some cases.

One embodiment of the DNA construct is e.g., a DNA construct prepared by ligating tef1 gene promoter, a gene encoding GLOD, alp gene terminator and pyrG marker gene to the InFusion Cloning Site present in the multiple cloning site of pUC19.

As a method for transforming filamentous fungi, methods known to those skilled in the art can appropriately be selected and, for example, a protoplast PEG method can be used, in which a protoplast of a host filamentous fungus is prepared, and then, polyethylene glycol and calcium chloride are used (see, for example, Mol. Gen. Genet. 218, 99-104, 1989, JP Patent Publication (Kokai) No. 2007-222055A). As the culture medium for regenerating a transformed filamentous fungus, an appropriate culture medium is used depending on the host filamentous fungus to be used and the transformation marker gene. For example, *if Aspergillus soya* is used as the host filamentous fungus and pyrG gene is used as the transformation marker gene, the transformed filamentous fungus can be regenerated in Czapek-Dox minimal medium (Difco) containing for example, 0.5 % agar and 1.2 M sorbitol.

For example, to obtain the transformed filamentous fungus of the present invention, the promoter of the gene encoding the GLOD that the host filamentous fungus originally has in the chromosome may be substituted with a high expression promoter such as tef1, by using homologous recombination. In this case, a transformation marker gene such as pyrG is preferably inserted in addition to the high expression promoter. For example, for this purpose, a transformation cassette consisting of an upstream region of a gene encoding GLOD-transformation marker gene-high expression promoter-all or part of gene encoding GLOD, can be used (see, Example 1 and Figure 1 in JP Patent Publication (Kokai) No. 2011-239681A). In this case, the upstream region of a gene encoding GLOD and all or part of the gene encoding GLOD are used for homologous recombination. As all or part of the gene encoding GLOD, a region containing the initiation codon up to a midstream region can be used. The length of the region suitable for homologous recombination is preferably 0.5 kb or more.

Whether the transformed filamentous fungus of the present invention was produced or not can be confirmed by culturing the transformed filamentous fungus of the present invention under conditions where GLOD enzyme activity can be confirmed and then confirming the GLOD activity in a culture obtained after culturing.

Further, whether the transformed filamentous fungus of the present invention was produced or not can be confirmed by extracting chromosomal DNA from a transformed filamentous fungus, subjecting the chromosomal DNA to PCR using the chromosomal DNA as the template and confirming production of a PCR product that can be amplified if transformation took place.

For example, PCR is carried out by using a forward primer to the nucleotide sequence of the applied promoter in combination with a reverse primer to the nucleotide sequence of the transformation marker gene, and then, whether or not a product having the estimated length is obtained, is confirmed.

The host may be a known microorganism, a known strain and identicals or equivalents to a known microorganism or strain described in the present specification. Identicals refers to a host that exerts equal functions with regard to recombinant protein expression. Equivalents are modified hosts prepared based on a host known at the time of filing of the present application and includes those developed after filing of the present application and hosts that have properties similar to those of a host known at the time of filing of the present application and have been discovered after filing of the present application. With regard to the academic name or classification of a microorganism, the description of the present specification should prevail if the academic name, genus name, classification or the like is changed after filing of the present application. Such a name or the like is based on that at the time of filing of the present application.

(High throughput screening)

A GLOD can further be subjected to high throughput screening in order to obtain a functional GLOD mutant. For example, a library of transformed strains or transduced strains comprising mutated GLOD genes can be prepared and then the library may be subjected to high throughput screening based on a microtiter plate or to ultrahigh-throughput screening based on droplet microfluids. As an example, a combinatorial library of mutant genes encoding variants is constructed and then a large population of modified GLODs is screened by using, e.g., phage display (for example, Chem. Rev. 105 (11): 4056-72, 2005); yeast display (for example, Comb Chem High Throughput Screen. 2008; 11 (2): 127-34); bacterial display (for example, Curr Opin Struct Biol 17: 474-80, 2007) and the like. Also see, Agresti, et al., "Ultrahigh-throughput screening in drop-based microfluidics for directed evolution" Proceedings of the National Academy of Sciences 107 (9): 4004-4009 (Mar, 2010). The contents of this document on the ultrahigh-throughput screening method that may be used for screening GLOD variants is incorporated herein by reference. For example, a library can be constructed by an error-prone PCR method. Further, saturation mutagenesis may be used to introduce mutations into the region(s) or position(s) described herein or the corresponding region(s) or position(s) thereto as the target to construct a library. Using such library, appropriate cells such as electrocompetent EBY-100 cells, can be transformed and about 10 to the power of seven (10,000,000) mutants can be obtained. Yeast cells transformed with said library can subsequently be subjected to cell sorting. A polydimethoxysiloxane (PDMS) microfluidic device prepared by a standard soft lithography method may be used. Monodispersed droplets can be formed using a flow focus device. Formed droplets containing individual mutants can be subjected to an appropriate sorting device. When screening cells, the presence or absence of GLOD activity can be utilized. For this purpose, the reaction solution having a composition capable of developing color if GLOD functions, may, for example, be used. For example, in the case of using DCIP, absorbance at 600 nm may be measured using a 96 well plate, a 192 well plate, a 384 well plate or a 9600 well plate and a plate reader. Mutation and screening can be repeated a plurality of times. The term mutation used here includes an amino acid substitution, insertion, deletion, and/or addition.

For example, 1 to 10 mutations can be introduced into a GLOD and GLOD activity may be confirmed. Next, a GLOD mutant confirmed to have activity can be used as the starting material, further 1 to 10 mutations can be introduced thereinto and activity may be confirmed. A series of high throughput screening procedures (for example, the above method in which about 10 to the power of seven mutants are obtained and screened) may be repeated by 2 or more rounds, 5 or more rounds, 10 or more rounds, 15 or more rounds, for example, 20 or more rounds. High throughput screening to introduce 1 or more mutations, 5 or more mutations, for example, 10 or more mutations at each round may be repeated, for example, by 10 rounds to rapidly obtain mutants having 10 or more mutations, 50 or more mutations, for example, 100 or more mutations introduced in the starting GLOD and also having activity. Such high throughput screening may be repeated by 20 rounds to rapidly obtain mutants having 20 or more mutations, 100 or more mutations, for example, 200 or more mutations introduced into the starting GLOD and also having activity. These procedures may be performed by repeating a routine process.

Mutation(s) may be introduced into any one or more positions from the first amino acid to the last amino acid of the full length amino acid sequence of the GLOD. However, regions important for enzyme functions, such as the active center, substrate recognition sites, coenzyme recognition motifs and their neighborhoods are excluded. GLOD is widely used in the industry and those skilled in the art have a thorough knowledge on the regions important for enzyme functions, including an active center, substrate recognition site and coenzyme recognition motif. In a particular embodiment, for example, one or more mutations may first be introduced into the part from the 1st to 10th positions of the full length sequence of the GLOD. Next, a GLOD mutant confirmed to have activity can be used as the starting material, and then one or more mutations can be introduced into the 11th to 20th positions and activity may be confirmed. This may be repeated n times ($n \leq 68$). For example, at the 68th round, one or more mutations may be introduced into the 680th to 687th positions. Regions important for enzyme functions or regions not intended to undergo modification may appropriately be skipped during the process. This can introduce a mutation into any position in the full length sequence except for the regions important for enzyme functions and GLOD mutants having, for example, 5 or more, 10 or more, 20 or more, 30 or more, 40 or more, 50 or more, 60 or more, 70 or more, 80 or more, 90 or more, 100 or more, 110 or more, 120 or more, 130 or more, 140 or more, 150 or more, 160 or more, 170 or more, 180 or more, 190 or more, for example, 200 or more mutations and having activity may be obtained rapidly.

Mutation(s) may be introduced at random or may be introduced by rational design. In one embodiment, the mutation to be introduced by rational design or the mutation to be introduced at random may be a conservative amino acid substitution. Conservative amino acid substitutions include an amino acid substitution by which the amino acid before the substitution and the amino acid after the substitution have similar chemical characteristics (for example, Stryer et al., Biochemistry, vol. 5, 2002, p. 44-49). The conservative amino acid substitution may be selected from the group consisting of, for example, (i) a substitution of a basic amino acid with a different type of basic amino acid; (ii) a substitution of an acidic amino acid with a different type of acidic amino acid; (iii) a substitution of an aromatic amino acid with a different type of aromatic amino acid; (iv) a substitution of a nonpolar aliphatic amino acid with a different type of nonpolar aliphatic amino acid; and (v) a substitution of a polar uncharged amino acid with a different type of polar uncharged amino acid. The basic amino acid may be selected from, for example, arginine, histidine, and lysine. The acidic amino acid may be, for example, aspartic acid or glutamic acid. The aromatic amino acid may be selected from, for example, phenylalanine, tyrosine and tryptophan. The nonpolar aliphatic amino acid may be selected from, for example, glycine, alanine, valine, leucine, methionine and isoleucine. The polar uncharged amino acid may be selected from, for example, serine, threonine, cysteine, proline, asparagine and glutamine.

In one embodiment, the mutation to be introduced by rational design or the mutation to be introduced at random includes a substitution between functionally similar amino acids. The list of functionally similar amino acids is well known in the art. In one embodiment, with regard to the substitution between functionally similar amino acids, the amino acid before the substitution and the amino acid after the substitution may fall into any category of amino acid classification defined as follows:

1) glycine (G), alanine (A);
2) aspartic acid (D), glutamic acid (E);
3) asparagine (N), glutamine (Q);
4) arginine (N), lysine (K);
5) isoleucine (I), leucine (L), methionine (M), valine (V), proline (P);
6) phenylalanine (F), tyrosine (Y), tryptophan (W);
7) serine (S), threonine (T); and
8) cysteine (C), methionine (M).

In a typical embodiment, the conservative amino acid substitution or the substitution between functionally similar amino acids does not reside in regions important for enzyme functions of the GLOD, such as the active center, substrate recognition sites, coenzyme recognition motifs and their neighborhoods and thus does not significantly influence the activity of the enzyme.

The GLOD mutant may also comprise an insertion of an additional amino acid compared to a sequence before the mutation. In a typical embodiment, the amino acid insertion does not reside in regions important for enzyme functions, such as the active center, substrate recognition sites, coenzyme recognition motifs and their neighborhoods and thus does not significantly influence the activity of the enzyme. The GLOD mutant may also comprise an addition of an additional amino acid compared to a sequence before the mutation. In one embodiment, the amino acid addition is performed at the N terminus or C terminus of the GLOD and thus does not significantly influence the activity of the enzyme. Examples of addition include, but are not limited to, a short stretch of histidine residues (for example, 2 to 6 histidine residues) for assisting in the purification of the GLOD. Other examples of addition include, but are not limited to, an addition of a signal peptide for assisting in the expression of the GLOD. Examples of the signal peptide include known signal sequences or their functional equivalents.

The GLOD mutant may comprise a deletion of an amino acid compared to a sequence before the mutation. In a typical embodiment, the amino acid deletion does not reside in regions important for enzyme functions and thus does not significantly influence the activity of the enzyme. In one embodiment, the deletion may be a short deletion of 1 to 2 amino acids. In one embodiment, when the amino acid sequence of a GLOD is compared to the amino acid sequence of another GLOD and an amino acid is deleted in one of the sequences, this deletion may be introduced into the other GLOD. Since both the GLODs exhibit activity, such a deletion is likely to have no significant influence on the activity of the enzyme.

Mutation(s) may be introduced into a GLOD so as not to destroy a secondary structure or structural motif, such as an $\alpha$ helix structure or $\beta$ sheet structure. The region of the secondary structure may be identified with, for example, a secondary structure predicting algorithm. Examples of the predicting algorithm include, but are not limited to, NetSurfP - 2.0. The same applies to other structural motifs such as nest or niche.

(Thermal stability improvement type mutation)

In one embodiment, the present disclosure provides a GLOD mutant having a thermal stability improvement type mutation introduced therein. The thermal stability of this mutant is improved compared to a GLOD before introduction of the mutation. In the present specification, with regard to the GLOD mutant of the present disclosure, improved thermal stability means that when a GLOD is subjected to heat treatment at a predetermined temperature for a predetermined time, the residual activity of the GLOD mutant after the heat treatment is improved compared to the residual activity after the heat treatment of the GLOD before introduction of the mutation.

In one embodiment, the GLOD mutant of the present disclosure comprises an amino acid substitution compared to SEQ ID NO: 1 or 58, at one or more positions, for example, 1, 2, 3, 4, 5, 6, 7, 8, 9, 10, 11, 12, or 13 positions, selected from the group consisting of

the position corresponding to the 87th position of SEQ ID NO: 1,
the position corresponding to the 103rd position of SEQ ID NO: 1,
the position corresponding to the 133rd position of SEQ ID NO: 1,
the position corresponding to the 186th position of SEQ ID NO: 1,
the position corresponding to the 297th position of SEQ ID NO: 1,
the position corresponding to the 376th position of SEQ ID NO: 1,
the position corresponding to the 393rd position of SEQ ID NO: 1,
the position corresponding to the 428th position of SEQ ID NO: 1,
the position corresponding to the 516th position of SEQ ID NO: 1,
the position corresponding to the 566th position of SEQ ID NO: 1,
the position corresponding to the 568th position of SEQ ID NO: 1,
the position corresponding to the 585th position of SEQ ID NO: 1, and
the position corresponding to the 615th position of SEQ ID NO: 1, and

the thermal stability of the mutant after the amino acid substitution is improved compared to the mutant before the substitution. With regard to the above positions, the phrase "amino acid is substituted (comprises an amino acid substitution)" means that the wild type amino acid is substituted with another amino acid and therefore, the wild type amino acid is excluded from amino acids after the substitution.

In one embodiment, with regard to the GLOD mutant of the present disclosure, the substituted amino acid (amino acid after the substitution) introduced into the position corresponding to the 87th position of SEQ ID NO: 1 may be tyrosine.

In one embodiment, with regard to the GLOD mutant of the present disclosure, the substituted amino acid introduced into the position corresponding to the 103rd position of SEQ ID NO: 1 may be selected from the group consisting of phenylalanine, leucine, valine, isoleucine and methionine.

In one embodiment, with regard to the GLOD mutant of the present disclosure, the substituted amino acid introduced into the position corresponding to the 133rd position of SEQ ID NO: 1 may be selected from the group consisting of leucine and tyrosine.

In one embodiment, with regard to the GLOD mutant of the present disclosure, the amino acid substitution introduced into the position corresponding to the 186th position of SEQ ID NO: 1 may be selected from the group consisting of glutamic acid, aspartic acid, tyrosine, glutamine, asparagine, alanine, leucine, cysteine, methionine, phenylalanine, serine, histidine and threonine.

In one embodiment, with regard to the GLOD mutant of the present disclosure, the substituted amino acid introduced into the position corresponding to the 297th position of SEQ ID NO: 1 may be selected from the group consisting of leucine, valine, isoleucine and methionine.

In one embodiment, with regard to the GLOD mutant of the present disclosure, the substituted amino acid introduced into the position corresponding to the 376th position of SEQ ID NO: 1 may be selected from the group consisting of phenylalanine, leucine, isoleucine and methionine.

In one embodiment, with regard to the GLOD mutant of the present disclosure, the substituted amino acid introduced into the position corresponding to the 393rd position of SEQ ID NO: 1 may be selected from the group consisting of leucine, valine, isoleucine and methionine.

In one embodiment, with regard to the GLOD mutant of the present disclosure, the substituted amino acid introduced into the position corresponding to the 428th position of SEQ ID NO: 1 may be selected from the group consisting of tyrosine and methionine.

In one embodiment, with regard to the GLOD mutant of the present disclosure, the substituted amino acid introduced into the position corresponding to the 516th position of SEQ ID NO: 1 may be phenylalanine.

In one embodiment, with regard to the GLOD mutant of the present disclosure, the substituted amino acid introduced into the position corresponding to the 566th position of SEQ ID NO: 1 may be selected from the group consisting of phenylalanine, leucine, valine and methionine.

In one embodiment, with regard to the GLOD mutant of the present disclosure, the substituted amino acid introduced into the position corresponding to the 568th position of SEQ ID NO: 1 may be selected from the group consisting of isoleucine and methionine.

In one embodiment, with regard to the GLOD mutant of the present disclosure, the substituted amino acid introduced into the position corresponding to the 585th position of SEQ ID NO: 1 may be selected from the group consisting of leucine and methionine.

In one embodiment, with regard to the GLOD mutant of the present disclosure, the substituted amino acid introduced into the position corresponding to the 615th position of SEQ ID NO: 1 may be selected from the group consisting of leucine, valine, and phenylalanine. The corresponding positions shall be described later.

In one embodiment, the thermal stability improvement type mutation above may be introduced into a wild type GLOD or conventional GLOD. A conventional GLOD described herein refers to a conventional GLOD that requires protease treatment for expression of the active protein. It is believed that not only does a GLOD having the thermal stability improvement type mutation introduced therein exert activity after being treated with protease, as in the wild type GLOD or conventional type GLOD, but also thermal stability thereof is improved compared to the GLOD before introduction of the mutation. For convenience, such mutant may also be referred to as a GLOD-T in the present specification. Further, a plurality of such thermal stability improvement type mutations may be introduced. In the present specification, a GLOD having one thermal stability improvement type mutation above introduced therein may also be referred to as GLOD-T1. Likewise, a mutant having 2, 3, 4, 5, 6, 7, 8, 9, 10, 11, 12, or 13 such mutations introduced therein may also be referred to as GLOD-T2, GLOD-T3, GLOD-T4, GLOD-T5, GLOD-T6, GLOD-T7, GLOD-T8, GLOD-T9, GLOD-T10, GLOD-T11, GLOD-T12, or GLOD-T13, respectively.

The present inventors have found that the thermal stability of a GLOD mutant having an amino acid substitution introduced at the position corresponding to the 87th, 103rd, 133rd, 186th, 297th, 376th, 393rd, 428th, 516th, 566th, 568th, 585th or 615th position of SEQ ID NO: 1 is improved compared to a GLOD before modification. Based on these findings, those skilled in the art shall appreciate that the thermal stability of a GLOD from another origin will also be improved by introducing an identical amino acid substitution into the position corresponding to the 87th position or the like of SEQ ID NO: 1 and this GLOD can be used in various reactions.

In one embodiment, the thermal stability improvement type mutation above may be introduced into the amino acid sequence deletion type GLOD mutant (GLODΔ) of the present disclosure. The amino acid sequence deletion type GLOD mutant of the present disclosure requires no protease treatment for expression of an active protein, unless otherwise specified. Surprisingly, the thermal stability of the amino acid sequence deletion type GLOD mutant of the present disclosure is improved, as described above, compared to the corresponding GLOD before the amino acid sequence deletion. The thermal stability improvement type mutation above may further be introduced into such an amino acid sequence deletion type GLOD mutant (GLODΔ) of the present disclosure. For convenience, such a mutant may also be referred to as GLODΔ-T in the present specification. In the present specification, a GLODΔ having one thermal stability improvement type mutation introduced therein may also be referred to as GLODΔ-T1. Likewise, a Δ mutant having 2, 3, 4, 5, 6, 7, 8, 9, 10, 11, 12, or 13 such mutations introduced therein may also be referred to as GLODΔ-T2, GLODΔ-T3, GLODΔ-T4, GLODΔ-T5, GLODΔ-T6, GLODΔ-T7, GLODΔ-T8, GLODΔ-T9, GLODΔ-T10, GLODΔ-T11, GLODΔ-T12, or GLODΔ-T13, respectively.

(Preliminary amino acid substitution)

In a preliminary embodiment, the GLOD mutant of the present disclosure, for example, a thermal stability improvement type mutant such as a GLOD-T or GLOD-T1 to GLOD-T13, an amino acid sequence deletion type GLOD mutant such as a GLODΔ-T or GLODΔ-T1 to GLODΔ-T13, or a GLOD mutant having, for example, the amino acid sequence of SEQ ID NO: 12, 13, 58, 59 or 60 or an amino acid sequence identity of 70% or more, 80% or more, or 90% or more with any of these may optionally have an amino acid substitution described in WO 2021/193598. For example, with regard to the GLOD mutant of the present disclosure, Ala at the 106th position with reference to SEQ ID NO: 1 in the present specification may be substituted with Ser. This amino acid substitution is referred to as A106S in the present specification. Likewise, the GLOD mutant of the present disclosure may also have one or more, for example, 1 to 25 amino acid substitutions selected from the group consisting of A106S, C210S, Q235E, D236E, D237E, P244H, T311S, W313F, Q333E, I334V, I334L, M336L, Q338E, R339K, T416S, A438P, K441E, Y455F, Q456R, Q457E, Q457K, L505I, P598A, C601S, and P609A with reference to SEQ ID NO: 1 in the present specification. The same applies to the positions corresponding to these positions.

For convenience, amino acid substitutions described in WO 2021/193598 may also be referred to as J for differentiating from the thermal stability improvement type amino acid deletion Δ of the present disclosure or the thermal stability improvement type mutation T of the present disclosure. That is, J is regarded as a set of the amino acid substitutions described in WO 2021/193598 and the order of the individual components is not limited.

J = {A106S, C210S, Q235E, D236E, D237E, P244H, T311S, W313F, Q333E, I334V, I334L, M336L, Q338E, R339K, T416S, A438P, K441E, Y455F, Q456R, Q457R, Q457K, L505L P598A, C601S, P609A}

A mutant having one of the mutations included in J is defined as J1 and mutants having n mutations are also defined as Jn ... and likewise, mutants up to J25 are defined in the same manner. In one embodiment, the present disclosure provides a combination mutant described below. Those skilled in the art are capable of preparing such finite combination mutants one by one and confirming their activity or thermal stability. In the description below, Δ to be deleted may be a sequence of 31 to 54 amino acids in length. In the description below, the C terminal point of origin of Δ to be deleted may be the position corresponding to the 510th, 508th or 507th position of SEQ ID NO: 1.

[Mathematical formula 2]

$$\text{GLOD-T} \cap \text{J}$$

[Mathematical formula 3]

$$\text{GLOD-T} \cap \{\text{J1 to J25}\}$$

[Mathematical formula 4]

$$\{\text{GLOD-T1 to GLOD-T13}\} \cap \text{J}$$

[Mathematical formula 5]

$$\{\text{GLOD-T1 to GLOD-T13}\} \cap \{\text{J1 to J25}\}$$

[Mathematical formula 6]

$$\text{GLOD}\Delta\text{-T} \cap \text{J}$$

[Mathematical formula 7]

$$\text{GLOD}\Delta\text{-T} \cap \{\text{J1 to J25}\}$$

[Mathematical formula 8]

$$\{\text{GLOD}\Delta\text{-T1 to GLOD}\Delta\text{-T13}\} \cap \text{J}$$

[Mathematical formula 9]

$$\{\text{GLOD}\Delta\text{-T1 to GLOD}\Delta\text{-T13}\} \cap \{\text{J1 to J25}\}$$

(GLOD mutant)

A GLODΔ mutant is prepared by introducing the amino acid sequence deletion of the present disclosure to a GLOD gene, and a GLOD, for example, a GLOD having high thermal stability, may be produced without carrying out protease treatment. A GLOD-T mutant is prepared by introducing the amino acid substitution T of the present disclosure to a GLOD gene, and a GLOD having high thermal stability may be produced. A GLODΔ-T mutant is prepared by introducing the amino acid sequence deletion and amino acid substitution of the present disclosure to a GLOD gene, and a GLOD, for example, a GLOD having high thermal stability, may be produced without carrying out protease treatment.

In some limited embodiments, reverse mutations (back mutations) from the amino acid after substitution back to the amino acid in the sequence of the naturally occurring GLOD (i.e., the naturally occurring amino acid) are excluded. In other embodiments, the amino acid after substitution at the position corresponding to the 87th position or the like of SEQ ID NO: 1 can be identical to the amino acid at the position in the sequence of the naturally occurring GLOD (i.e., the naturally occurring amino acid).

(Corresponding position)

In the present specification, when a particular position in a reference amino acid sequence corresponds to a particular position in another amino acid sequence similar thereto, such position is referred to as the "corresponding position". Further, the amino acid at the corresponding position is referred to as the "corresponding amino acid." In the present specification, for convenience, corresponding positions are described with reference to the amino acid sequence of the GLOD derived from *Streptomyces* sp. X-119-6 depicted in SEQ ID NO: 1. In such a case, a "corresponding position" in an amino acid sequence is a position in the amino acid sequence of a GLOD derived from another organism species that corresponds to the particular position in the amino acid sequence of the GLOD derived from *Streptomyces* sp. X-119-6 of SEQ ID NO: 1.

A method of identifying a "corresponding position" of an amino acid sequence can be performed by, for example, comparing amino acid sequences using a known algorithm such as the Lipman-Pearson method to assign maximum identity to conserved amino acid residues present in the amino acid sequence of each GLOD. By aligning the amino acid sequences of the GLODs by such method, the positions of the homologous amino acid residues in each of the GLOD sequences can be determined, regardless of insertion or deletion of amino acid residues in the amino acid sequences. Corresponding positions (homologous positions) are considered to exist at the same positions in the three-dimensional structures, and amino acid residues at such homologous positions are expected to exert similar effects in terms of specific function of the GLOD of interest.

(Corresponding position of mutation)

In the present specification, the term "position corresponding to the 87th position of the amino acid sequence of SEQ ID NO: 1" refers to the position corresponding to the 87th position of SEQ ID NO: 1, when the amino acid sequence of the target GLOD is compared with the amino acid sequence of SEQ ID NO: 1. The same applies to other positions, for example, the 103rd, 133rd, 186th, 297th, 376th, 393rd, 428th, 516th, 566th, 568th, 585th, and 615th positions, of SEQ ID NO: 1. The same applies to the 106th, 210th, 235th, 236th, 237th, 244th, 311th, 313th, 333rd, 334th, 334th, 336th, 338th, 339th, 416th, 438th, 441st, 455th, 456th, 457th, 457th, 505th, 598th, 601st, and 609th positions of SEQ ID NO: 1. For example, the position corresponding to the 87th position of the amino acid sequence of SEQ ID NO: 1 is the 87th position in SEQ ID NO: 58.

(Corresponding region)

The "corresponding region" in an amino acid sequence is also defined as described above in the "corresponding position." For example, the region corresponding to the 459th to 507th positions of SEQ ID NO: 1 is from the 459th to 507th positions in SEQ ID NO: 58. The region corresponding to the 459th to 466th positions of SEQ ID NO: 1 is from the 459th to 466th positions in SEQ ID NO: 58. The region corresponding to the 457th to 510th positions of SEQ ID NO: 1 is from the 457th to 510th positions in SEQ ID NO: 58. The region corresponding to the 670th to 687th positions of SEQ ID NO: 1 is from the 673rd to 690th positions in SEQ ID NO: 58.

(Amino acid sequence homology, identity, or similarity)

The amino acid sequence homology, identity, or similarity can be calculated by a program such as Maximum Matching or Search Homology of GENETYX (manufactured by GENETYX), a program such as Maximum Matching or Multiple Alignment of DNASIS Pro (manufactured by Hitachi Solutions, Ltd.), or a program such as Multiple Alignment of CLUSTALW. In order to calculate amino acid sequence identity, two or more GLODs may be aligned, and the positions of identical amino acids in such two or more GLODs may be determined. The identical regions in amino acid sequences can be determined based on such information. The percent identity of two or more amino acid sequences is determined by subjecting two or more amino acid sequences to alignment using the algorithm such as Blosum62 by designating the total number of amino acids in the aligned region as the denominator and the number of identical amino acids relative to the total number as the numerator. As such, if no identity is found in parts of the two or more amino acid sequences, for example, an amino acid sequence comprises at its C terminus an additional sequence in which no identity is observed, in general, such regions cannot be aligned and, therefore, such regions are not used for calculation of the percent identity.

Further, positions of similar amino acids in two or more GLODs can be examined. For example, a plurality of amino acid sequences can be subjected to alignment with the use of CLUSTALW. In such a case, Blosum62 can be used as the algorithm and a plurality of amino acid sequences can be subjected to alignment. Amino acids determined to be similar as a result of alignment may be referred to as "similar amino acids." In the mutant of the present disclosure, amino acid substitution can be carried out between such similar amino acids. By way of such alignment, amino acid sequences composed of identical amino acids or similar amino acids among a plurality of amino acid sequences can be examined.

Based on such information, homologous regions (conserved regions) in the amino acid sequences can be determined.

In one embodiment, the GLOD mutant of the present disclosure, when aligned with a GLOD having the amino acid sequence of SEQ ID NO: 1 or SEQ ID NO: 58, has a full length amino acid sequence identity of 50% or more, 55% or more, 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, for example, 99% or more and has improved thermal stability compared to a GLOD before modification.

In one embodiment, the GLOD variant of the present disclosure has an amino acid sequence comprising a modification or mutation, or a deletion, substitution, addition and/or insertion of one or several amino acids at a position other than the positions corresponding to the 87th, 103rd, 133rd, 186th, 297th, 376th, 393rd, 428th, 516th, 566th, 568th, 585th, and 615th positions of SEQ ID NO: 1 and has improved thermal stability compared to a GLOD before modification. In the present specification, one or several amino acids refer to 1 to 15, 1 to 10, 1 to 7, 1 to 5, 1 to 4, for example, 1 to 3, for example, 1 or 2 amino acids.

In one embodiment, the GLODΔ of the present disclosure meets at least one of the following:

(i) when the amino acid sequence of the GLODΔ is aligned with the amino acid sequence of SEQ ID NO: 1, the GLODΔ comprises a deletion of the amino acid sequence of a predetermined region of SEQ ID NO: 1, optionally comprises a deletion of the amino acid sequence of the region corresponding to the 670th to 687th positions of SEQ ID NO: 1, and comprises glutamate oxidase activity, wherein the predetermined region is a region consisting of 31 to 54 continuous amino acids whose C terminal point of origin is the position corresponding to the 510th, 508th, or 507th position of SEQ ID NO: 1,

(ii) the GLODΔ of said (i), wherein the GLODΔ consists of an amino acid sequence comprising a substitution, deletion or addition of one or several amino acids at a position other than the predetermined region of SEQ ID NO: 1, or consists of an amino acid sequence comprising a substitution, deletion or addition of one or several amino acids at a position other than the predetermined region of SEQ ID NO: 1 and the region corresponding to the 670th to 687th positions,

(iii) with regard to the GLODΔ of said (i) or (ii), the full length amino acid sequence thereof has a sequence identity of 70% or more, 75% or more, 80% or more, 85% or more, 90% or more, 91% or more, 92% or more, 93% or more, 94% or more, 95% or more, 96% or more, 97% or more, 98% or more, for example, 99% or more with the amino acid sequence of SEQ ID NO: 12, SEQ ID NO: 13 or SEQ ID NO: 59,

(iv) with regard to the GLODΔ of said (i) or (ii), the full length amino acid sequence thereof has a sequence identity of 70% or more, 75% or more, 80% or more, 85% or more, 90% or more, 91% or more, 92% or more, 93% or more, 94% or more, 95% or more, 96% or more, 97% or more, 98% or more, for example, 99% or more with the amino acid sequence of SEQ ID NO: 12, SEQ ID NO: 13 or SEQ ID NO: 59, the amino acid at the position corresponding to the 291st position of SEQ ID NO: 1 in the GLODΔ is arginine, and the amino acid sequence of the positions corresponding to the 51st to 56th positions of SEQ ID NO: 1 is Gly-Xaa-Gly-Xaa-Xaa-Gly (wherein Xaa represents any amino acid), or

(v) with regard to the GLODΔ of said (i) or (ii), the full length amino acid sequence thereof has a sequence identity of 70% or more, 75% or more, 80% or more, 85% or more, 90% or more, 91% or more, 92% or more, 93% or more, 94% or more, 95% or more, 96% or more, 97% or more, 98% or more, for example, 99% or more with the amino acid sequence of SEQ ID NO: 12, SEQ ID NO: 13 or SEQ ID NO: 59, the amino acid at the position corresponding to the 291st position of SEQ ID NO: 1 in the GLODΔ is arginine, the amino acid sequence of the positions corresponding to the 51st to 56th positions of SEQ ID NO: 1 is Gly-Xaa-Gly-Xaa-Xaa-Gly (wherein Xaa represents any amino acid), and thermal stability is improved compared to a GLOD before modification.

In one embodiment, the GLOD-T of the present disclosure meets at least one of the following:

(i) when the amino acid sequence of the GLOD-T is aligned with the amino acid sequence of SEQ ID NO: 1, the GLOD-T comprises a substitution of the amino acid at the position corresponding to a position selected from the group consisting of the 87th, 103rd, 133rd, 186th, 297th, 376th, 393rd, 428th, 516th, 566th, 568th, 585th, and 615th positions of SEQ ID NO: 1,

(ii) the GLOD-T of said (i), wherein the GLOD-T consists of an amino acid sequence comprising a substitution, deletion or addition of one or several amino acids at a position other than the positions corresponding to the 87th, 103rd, 133rd, 186th, 297th, 376th, 393rd, 428th, 516th, 566th, 568th, 585th, and 615th positions of SEQ ID NO: 1,

(iii) with regard to the GLOD-T of said (i) or (ii), the full length amino acid sequence thereof has a sequence identity of 70% or more, 75% or more, 80% or more, 85% or more, 90% or more, 91% or more, 92% or more, 93% or more, 94% or more, 95% or more, 96% or more, 97% or more, 98% or more, for example, 99% or more with the amino acid sequence of SEQ ID NO: 1 or SEQ ID NO: 58,

(iv) with regard to the GLOD-T of said (i) or (ii), the full length amino acid sequence thereof has a sequence identity of 70% or more, 75% or more, 80% or more, 85% or more, 90% or more, 91% or more, 92% or more, 93% or more, 94% or more, 95% or more, 96% or more, 97% or more, 98% or more, for example, 99% or more with the amino acid sequence of SEQ ID NO: 1 or SEQ ID NO: 58, the amino acid at the position corresponding to the 291st position of SEQ ID NO: 1 in the GLOD-T is arginine, and the amino acid sequence of the positions corresponding to the 51st to 56th positions of SEQ ID NO: 1 is Gly-Xaa-Gly-Xaa-Xaa-Gly (wherein Xaa represents any amino acid), or

(v) with regard to the GLOD-T of said (i) or (ii), the full length amino acid sequence thereof has a sequence identity of 70% or more, 75% or more, 80% or more, 85% or more, 90% or more, 91% or more, 92% or more, 93% or more, 94% or more, 95% or more, 96% or more, 97% or more, 98% or more, for example, 99% or more with the amino acid sequence of SEQ ID NO: 1 or SEQ ID NO: 58, the amino acid at the position corresponding to the 291st position of SEQ ID NO: 1 in the GLOD-T is arginine, the amino acid sequence of the positions corresponding to the 51st to 56th positions of SEQ ID NO: 1 is Gly-Xaa-Gly-Xaa-Xaa-Gly (wherein Xaa represents any amino acid), and thermal stability is improved compared to a GLOD before modification.

In a particular embodiment, with regard to the GLOD-T mutant of the present disclosure, the substituted amino acid introduced into the position corresponding to the 87th position of SEQ ID NO: 1 may be tyrosine. In a particular embodiment, with regard to the GLOD-T mutant of the present disclosure, the substituted amino acid introduced into the position corresponding to the 103rd position of SEQ ID NO: 1 may be selected from the group consisting of phenylalanine, leucine, valine, isoleucine and methionine. In a particular embodiment, with regard to the GLOD-T mutant of the present disclosure, the substituted amino acid introduced into the position corresponding to the 133rd position of SEQ ID NO: 1 may be selected from the group consisting of leucine and tyrosine. In a particular embodiment, with regard to the GLOD-T mutant of the present disclosure, the substituted amino acid introduced into the position corresponding to the 186th position of SEQ ID NO: 1 may be selected from the group consisting of glutamic acid, aspartic acid, tyrosine, glutamine, asparagine, alanine, leucine, cysteine, methionine, phenylalanine, serine, histidine and threonine. In a particular embodiment, with regard to the GLOD-T mutant of the present disclosure, the substituted amino acid introduced into the position corresponding to the 297th position of SEQ ID NO: 1 may be selected from the group consisting of leucine, valine, isoleucine and methionine. In a particular embodiment, with regard to the GLOD-T mutant of the present disclosure, the substituted amino acid introduced into the position corresponding to the 376th position of SEQ ID NO: 1 may be selected from the group consisting of phenylalanine, leucine, isoleucine and methionine. In a particular embodiment, with regard to the GLOD-T mutant of the present disclosure, the substituted amino acid introduced into the position corresponding to the 393rd position of SEQ ID NO: 1 may be selected from the group consisting of leucine, valine, isoleucine and methionine. In a particular embodiment, with regard to the GLOD-T mutant of the present disclosure, the substituted amino acid introduced into the position corresponding to the 428th position of SEQ ID NO: 1 may be selected from the group consisting of tyrosine and methionine. In a particular embodiment, with regard to the GLOD-T mutant of the present disclosure, the substituted amino acid introduced into the position corresponding to the 516th position of SEQ ID NO: 1 may be phenylalanine. In a particular embodiment, with regard to the GLOD-T mutant of the present disclosure, the substituted amino acid introduced into the position corresponding to the 566th position of SEQ ID NO: 1 may be selected from the group consisting of phenylalanine, leucine, valine and methionine. In a particular embodiment, with regard to the GLOD-T mutant of the present disclosure, the substituted amino acid introduced into the position corresponding to the 568th position of SEQ ID NO: 1 may be selected from the group consisting of isoleucine and methionine. In a particular embodiment, with regard to the GLOD-T mutant of the present disclosure, the substituted amino acid introduced into the position corresponding to the 585th position of SEQ ID NO: 1 may be selected from the group consisting of leucine and methionine. In a particular embodiment, with regard to the GLOD-T mutant of the present disclosure, the substituted amino acid introduced into the position corresponding to the 615th position of SEQ ID NO: 1 may be selected from the group consisting of leucine, valine, and phenylalanine.

In one embodiment, the GLODΔ-T of the present disclosure meets at least one of the following:

(i) when the amino acid sequence of the GLODΔ-T is aligned with the amino acid sequence of SEQ ID NO: 1, the GLODΔ-T comprises a deletion of the amino acid sequence of a predetermined region of SEQ ID NO: 1, optionally comprises a deletion of the amino acid sequence of the region corresponding to the 670th to 687th positions of SEQ ID NO: 1, and comprises glutamate oxidase activity, wherein the predetermined region is a region consisting of 31 to 54 continuous amino acids whose C terminal point of origin is the position corresponding to the 510th, 508th, or 507th position of SEQ ID NO: 1, and

when the amino acid sequence of the GLODΔ-T is aligned with the amino acid sequence of SEQ ID NO: 1, the GLODΔ-T comprises a substitution of an amino acid, for example, a modification to an amino acid selected from the group consisting of tyrosine, phenylalanine, tryptophan, leucine, isoleucine, valine, methionine, glutamic acid, aspartic acid, glutamine, asparagine, alanine, and threonine, at a position corresponding to a position selected from the group consisting of the 87th, 103rd, 133rd, 186th, 297th, 376th, 393rd, 428th, 516th, 566th, 568th, 585th, and 615th positions of SEQ ID NO: 1,

(ii) the GLODΔ-T of said (i), wherein the GLODΔ-T consists of an amino acid sequence comprising a substitution, deletion or addition of one or several amino acids at a position other than the positions corresponding to the 87th, 103rd, 133rd, 186th, 297th, 376th, 393rd, 428th, 516th, 566th, 568th, 585th, and 615th positions of SEQ ID NO: 1 and the predetermined region of SEQ ID NO: 1, or consists of an amino acid sequence comprising a substitution, deletion or addition of one or several amino acids at a position other than the positions corresponding to the 87th, 103rd, 133rd, 186th, 297th, 376th, 393rd, 428th, 516th, 566th, 568th, 585th, and 615th positions of SEQ ID NO: 1, the predetermined region of SEQ ID NO: 1 and the region corresponding to the 670th to 687th positions,

(iii) with regard to the GLODΔ-T of said (i) or (ii), the full length amino acid sequence thereof has a sequence identity of 70% or more, 75% or more, 80% or more, 85% or more, 90% or more, 91% or more, 92% or more, 93% or more, 94% or more, 95% or more, 96% or more, 97% or more, 98% or more, for example, 99% or more with the amino acid sequence of SEQ ID NO: 12, SEQ ID NO: 13 SEQ ID NO: 59, SEQ ID NO: 60 or SEQ ID NO: 69,

(iv) with regard to the GLODΔ-T of said (i) or (ii), the full length amino acid sequence thereof has a sequence identity of 70% or more, 75% or more, 80% or more, 85% or more, 90% or more, 91% or more, 92% or more, 93% or more, 94% or more, 95% or more, 96% or more, 97% or more, 98% or more, for example, 99% or more with the amino acid sequence of SEQ ID NO: 12, SEQ ID NO: 13, SEQ ID NO: 59, SEQ ID NO: 60 or SEQ ID NO: 69, the amino acid at the position corresponding to the 291st position of SEQ ID NO: 1 in the GLODΔ is arginine, and the amino acid sequence of the positions corresponding to the 51st to 56th positions of SEQ ID NO: 1 is Gly-Xaa-Gly-Xaa-Xaa-Gly (wherein Xaa represents any amino acid), or

(v) with regard to the GLODΔ-T of said (i) or (ii), the full length amino acid sequence thereof has a sequence identity of 70% or more, 75% or more, 80% or more, 85% or more, 90% or more, 91% or more, 92% or more, 93% or more, 94% or more, 95% or more, 96% or more, 97% or more, 98% or more, for example, 99% or more with the amino acid sequence of SEQ ID NO: 12, SEQ ID NO: 13, SEQ ID NO: 59, SEQ ID NO: 60 or SEQ ID NO: 69, the amino acid at the position corresponding to the 291st position of SEQ ID NO: 1 in the GLODΔ is arginine, the amino acid sequence of the positions corresponding to the 51 st to 56th positions of SEQ ID NO: 1 is Gly-Xaa-Gly-Xaa-Xaa-Gly (wherein Xaa represents any amino acid), and thermal stability is improved compared to a GLOD before modification.

In one embodiment, the present disclosure provides a GLOD mutant having an amino acid sequence identity of 70% or more, 75% or more, 80% or more, 85% or more, for example, 90% or more, for example, 95% or more with the amino acid sequence of SEQ ID NO: 60 or SEQ ID NO: 69, comprising a deletion of the amino acid sequence of a predetermined region of SEQ ID NO: 1, wherein the predetermined region is a region consisting of 31 to 54 continuous amino acids whose C terminal point of origin is the position corresponding to the 510th, 508th, or 507th position of SEQ ID NO: 1,

having an amino acid substitution compared to the amino acid sequence of SEQ ID NO: 58, at one or more positions selected from the group consisting of

the position corresponding to the 87th position of SEQ ID NO: 1,
the position corresponding to the 103rd position of SEQ ID NO: 1,
the position corresponding to the 133rd position of SEQ ID NO: 1,
the position corresponding to the 186th position of SEQ ID NO: 1,
the position corresponding to the 297th position of SEQ ID NO: 1,
the position corresponding to the 376th position of SEQ ID NO: 1,
the position corresponding to the 393rd position of SEQ ID NO: 1,
the position corresponding to the 428th position of SEQ ID NO: 1,
the position corresponding to the 516th position of SEQ ID NO: 1,
the position corresponding to the 566th position of SEQ ID NO: 1,
the position corresponding to the 568th position of SEQ ID NO: 1,
the position corresponding to the 585th position of SEQ ID NO: 1, and
the position corresponding to the 615th position of SEQ ID NO: 1, and

having glutamate oxidase activity. However, in particular embodiments, wild type amino acids are excluded from the substituted amino acids. In a particular embodiment, in the mutant, the region corresponding to the 670th to 687th positions may further be deleted.

In a particular embodiment, with regard to the GLODΔ-T mutant of the present disclosure, the substituted amino acid introduced into the position corresponding to the 87th position of SEQ ID NO: 1 may be tyrosine. In a particular embodiment, with regard to the GLODΔ-T mutant of the present disclosure, the substituted amino acid introduced into the position corresponding to the 103rd position of SEQ ID NO: 1 may be selected from the group consisting of phenylalanine, leucine, valine, isoleucine and methionine. In a particular embodiment, with regard to the GLODΔ-T mutant of the present disclosure, the substituted amino acid introduced into the position corresponding to the 133rd position of SEQ ID NO: 1 may be selected from the group consisting of leucine and tyrosine. In a particular embodiment, with regard to the GLODΔ-T mutant of the present disclosure, the substituted amino acid introduced into the position corresponding to the 186th position of SEQ ID NO: 1 may be selected from the group consisting of glutamic acid, aspartic acid, tyrosine, glutamine, asparagine, alanine, leucine, cysteine, methionine, phenylalanine, serine, histidine and threonine. In a particular embodiment, with regard to the GLODΔ-T mutant of the present disclosure, the substituted amino acid introduced into the position corresponding to the 297th position of SEQ ID NO: 1 may be selected from the group consisting of leucine, valine, isoleucine and methionine. In a particular embodiment, with regard to the GLODΔ-T mutant of the present disclosure, the substituted amino acid introduced into the position corresponding to the 376th position of SEQ ID NO: 1 may be selected from the group consisting of phenylalanine, leucine, isoleucine and methionine. In a particular embodiment, with regard to the GLODΔ-T mutant of the present disclosure, the substituted amino acid introduced into the position corresponding to the 393rd position of SEQ ID NO: 1 may be selected from the group consisting of leucine, valine, isoleucine and methionine. In a particular embodiment, with regard to the GLODΔ-T mutant of the present disclosure, the substituted amino acid introduced into the position corresponding to the 428th position of SEQ ID NO: 1 may be selected from the group consisting of tyrosine and methionine. In a particular embodiment, with regard to the GLODΔ-T mutant of the present disclosure, the substituted amino acid introduced into the position corresponding to the 516th position of SEQ ID NO: 1 may be phenylalanine. In a particular embodiment, with regard to the GLODΔ-T mutant of the present disclosure, the substituted amino acid introduced into the position corresponding to the 566th position of SEQ ID NO: 1 may be selected from the group consisting of phenylalanine, leucine, valine and methionine. In a particular embodiment, with regard to the GLODΔ-T mutant of the present disclosure, the substituted amino acid introduced into the position corresponding to the 568th position of SEQ ID NO: 1 may be selected from the group consisting of isoleucine and methionine. In a particular embodiment, with regard to the GLODΔ-T mutant of the present disclosure, the substituted amino acid introduced into the position corresponding to the 585th position of SEQ ID NO: 1 may be selected from the group consisting of leucine and methionine. In a particular embodiment, with regard to the GLODΔ-T mutant of the present disclosure, the substituted amino acid introduced into the position corresponding to the 615th position of SEQ ID NO: 1 may be selected from the group consisting of leucine, valine, and phenylalanine.

(Production of GLOD)

In one embodiment, the present invention provides a method for producing GLOD comprising a step of culturing a strain capable of producing GLOD under conditions where the GLOD can be expressed and a step of isolating GLOD from a culture product or culture solution. In such method, a host cell transformed with a vector comprising a gene encoding the GLOD of the present disclosure integrated therein can be used. The phrase conditions where the GLOD can be expressed refers to conditions where a GLOD gene is transcribed and translated, and a polypeptide encoded by such gene is produced.

Further, examples of media for culturing the strains mentioned above include media prepared by adding 1 or more inorganic salts selected from among, for example, sodium chloride, monopotassium phosphate, dipotassium phosphate, magnesium sulfate, magnesium chloride, ferric chloride, ferric sulfate, and manganese sulfate to 1 or more nitrogen sources, such as a yeast extract, tryptone, peptone, a meat extract, a corn steep liquor, or a leaching solution of soybean or wheat bran, and appropriately adding saccharine materials (sugar sources), vitamins, and the like thereto, where necessary.

Further, a substrate with which the GLOD can react or a compound similar thereto, such as a glycated protein, including a glycated amino acid, a glycated peptide, a degradation product of a glycated protein, glycated hemoglobin, or glycated albumin, may be added to the media, so as to increase the amount of the target enzyme to be produced.

It is appropriate to adjust the initial pH of the media to 7 to 9. Culture is preferably performed at 20°C to 42°C, and more preferably at about 25°C to 37°C for 4 to 24 hours, and further preferably at about 25°C to 37°C for 8 to 16 hours, by, for example, aeration spinner submerged culture, shake culture, or stationary culture.

Following the completion of culture, GLOD may be collected from the culture products with conventional enzyme collecting means. For example, a strain may be subjected to ultrasonication treatment or grinding treatment by a conventional method, the enzyme may be extracted using a lytic enzyme such as lysozyme, or bacteriolysis may be performed via shaking or allowing to stand still in the presence of toluene to excrete the enzyme from the microorganism body. The solution is filtered or centrifuged to remove solid content, and nucleic acids is removed with the aid of streptomycin sulfate, protamine sulfate, or manganese sulfate, according to need. Thereafter, ammonium sulfate, alcohol, acetone, or the like is added thereto, so as to fractionate the solution, and precipitates are then collected to obtain the crude enzymes.

A purified enzyme preparation can be obtained from the crude enzyme by a method appropriately selected from: gel filtration methods using Sephadex, Superdex, or Ultrogel; adsorption-elution methods using ion exchange carriers, hydrophobic carriers, or hydroxyapatite; electrophoretic methods using polyacrylamide gels, etc.; precipitation methods such as sucrose density-gradient centrifugation; affinity chromatographic methods; and fractionation methods using a molecular sieve membrane, a hollow-fiber membrane, etc. Alternatively, the methods mentioned above can be performed appropriately in combination and the purified GLOD preparation can be thus obtained.

In one embodiment, the GLOD mutant of the present disclosure is capable of, for example,

> (i) using FAD as a coenzyme,
> (iii) recognizing glutamate as a substrate, and
> (iii) acting so as to oxidize glutamate to generate 2-oxoglutaric acid, ammonia and hydrogen peroxide.

In one embodiment, the residual activity of the GLOD mutant of the present disclosure after being subjected to heat treatment at 30 to 40°C, for example, 35°C, for 30 minutes or 35 minutes may be 50% or more, 60% or more, 65% or more, 70% or more, 75% or more, 80% or more, 85% or more, for example, 90% or more when its activity before the heat treatment is defined as 100%. In one embodiment, the residual activity of the GLOD mutant of the present disclosure after being subjected to heat treatment at, for example, 60°C, for 30 minutes or 35 minutes may be 50% or more, 60% or more, 65% or more, 70% or more, 75% or more, 80% or more, 85% or more, for example, 90% or more when its activity before the heat treatment is defined as 100%. In one embodiment, the residual activity of the GLOD mutant of the present disclosure after being subjected to heat treatment at, for example, 65°C, for 30 minutes or 35 minutes may be 50% or more, 60% or more, 65% or more, 70% or more, for example, 75% or more when its activity before the heat treatment is defined as 100%. In one embodiment, the residual activity of the GLOD mutant of the present disclosure after being subjected to heat treatment at, for example, 70°C, for 30 minutes or 35 minutes may be 10% or more, 15% or more, 20% or more, 25% or more, 30% or more, for example, 35% or more when its activity before the heat treatment is defined as 100%.

GLODs that do not exhibit any activity on glutamate are excluded from the GLOD mutant of the present disclosure.

(Composition, reagent, electrode, sensor, and kit)

In one embodiment, the present invention provides a kit, sensor, electrode, reagent for measurement, or reagent composition for measurement of glutamate, comprising a GLOD. The composition, reagent, electrode, sensor or kit may comprise reagents for measurement of a reduced compound, reagents for measurement of hydrogen peroxide, a buffer, a surfactant, a salt, a preservative, or the like. The composition, reagent, electrode, sensor or kit may be supplemented with, for example, a solubilizer, a stabilizer, a reaction-improving agent, a glycated hemoglobin denaturation agent, a reducing agent, bovine serum albumin, or a saccharide (e.g., glycerin, lactose, or sucrose) and the like. The composition, reagent, electrode, sensor or kit may be supplemented with other known stabilizers, or systems that eliminate contaminants, and the like, according to need. Techniques that are employed for various conventional reagents, electrodes, sensors, or kits may be modified appropriately, and be employed for the composition, reagent, electrode, sensor or kit of the present disclosure.

Examples of surfactants include nonionic surfactants and ionic surfactants, such as cationic surfactants, anionic surfactants, and amphoteric surfactants.

Examples of nonionic surfactants include polyoxyethylene alkyl ether, sorbitan fatty acid ester, alkyl polyglucoside, fatty acid diethanol amide, and alkyl monoglyceryl ether.

Examples of cationic surfactants include alkyltrimethylammonium salt, dialkyldimethylammonium salt, alkylbenzyldimethylammonium salt, pyridinium salt, such as alkylpyridinium salt, phosphonium salt, such as alkylphosphonium salt, imidazolium salt, such as alkylimidazolium salt, and isoquinolinium salt, such as alkylisoquinolinium salt.

A reagent for measurement of hydrogen peroxide may comprise peroxidase and/or a chromogenic substrate. Examples of chromogenic substrates include, in addition to 4-aminoantipyrine, ADOS (N-ethyl-N-(2-hydroxy-3-sulfopropyl)-m-anisidine), ALOS (N-ethyl-N-(2-hydroxy-3-sulfopropyl)aniline), TOOS (N-ethyl-N-(2-hydroxy-3-sulfopropyl)-m-toluidine sodium), DA-67 (10-(carboxymethylaminocarbonyl)-3,7-bis(dimethylamino)-phenothiazine), and DA-64 (N-(carboxymethylaminocarbonyl)-4,4'-bis(dimethylamino)-diphenylamine) and the like.

(Method for measurement of glutamate)

In one embodiment, the present disclosure provides a method for measurement of glutamate. The method for measurement of glutamate may be a qualitative or quantitative method. The quantitative method comprises a step of bringing a glutamate-containing sample into contact with the GLOD of the present disclosure and a step of measuring the amount of substances produced or consumed by the reaction. The term "contact" used in accordance with the quantifying method encompasses any aspect of physical contact between the GLOD and a sample, such that the GLOD can catalyze the oxidation reaction of the glutamate. This includes, for example, not only cases in which a free enzyme is mixed with glutamate in a solution, but also cases in which a liquid sample comprising glutamate can be added or dropped (added dropwise) to the enzyme immobilized on a solid support.

A sample used for measurement can be any type of sample that can contain glutamate. A sample can appropriately be a processed sample.

When the amount of the enzyme used and the duration of the reaction are maintained at a constant level and the amount of glutamate to be added is altered, the range of glutamate concentration in which the absorbance of the detected chromogenic substrate proportionally decreases as the amount of added glutamate decreases can be investigated in order to determine the lowest glutamate concentration that can be detected (detection limit concentration) using that GLOD. It is possible to configure the amount of enzyme and duration of reaction, so as to adjust the detection limit of glutamate to a level lower than the glutamate level in the sample or in the blood.

According to the quantitative method of measurement, a calibration curve can be prepared in advance by performing regression analysis such as the method of least squares based on the measured absorbance of the control sample comprising glutamate at a known concentration. The measured value of the sample containing glutamate at an unknown concentration may be plotted on the prepared calibration curve, to quantify the glutamate level in the sample.

The time GLOD is allowed to act on a sample containing glutamate may be 5 seconds or longer, 10 seconds or longer, 20 seconds or longer, 30 seconds or longer, or 1 minute or longer to less than 60 minutes, less than 30 minutes, less than 10 minutes, or less than 5 minutes, for example, 0.5 minutes or more to less than 60 minutes, 1 minute or more to less than 30 minutes, 1 minute or more to less than 20 minutes, 1 minute or more to less than 10 minutes, or 1 minute or more to less than 5 minutes. While the reaction temperature may vary depending on the optimal temperature of the enzyme being used, the reaction temperature may for example be from 20°C to 45°C, and a temperature that is generally employed for an enzymatic reaction can appropriately be selected.

While the amount of the GLOD to be used varies depending on the amount of the substrate contained in the sample solution, the enzyme can be added to the solution to a final concentration of, for example, 0.1 to 50 U/ml or for example 0.2 to 10 U/ml. The pH level at the time of reaction can be adjusted using a buffer by taking the pH levels at which the GLOD can act, for example the optimal pH level, into consideration. The reaction pH level is, for example, 3 to 11, 5 to 9, or 6 to 8.

Measurement of hydrogen peroxide can be carried out simultaneously during the step of hydrogen peroxide generation, and measurement can be allowed to proceed simultaneously with the reaction with a GLOD. A substance consumed by the reaction may be subjected to measurement instead of the reaction product. An example of the substance consumed by the reaction to be measured is dissolved oxygen, and the amount of dissolved oxygen in the reaction solution can be measured with the use of a dissolved oxygen meter or the like.

(Method for measurement of GLOD activity)

Hereinbelow, an example of a method for measurement of GLOD activity involving the use of glutamate as a substrate is provided. However, the method for measurement is not limited thereto. The glutamate may be a commercially available glutamate. In the present specification, regarding the enzyme titer, unless specified otherwise, the amount of the enzyme that generates 1 $\mu$mol of hydrogen peroxide per minute, when carrying out measurement at 30°C and pH 7.4 using glutamate as the substrate, is defined as 1 U.

A: Reagents for measurements of activity

(Reagent 1) 250 mM potassium phosphate buffer, pH 7.4
(Reagent 2) 30 mM 4-aminoantipyrine (4-AA) solution
(Reagent 3) 15 mM N-ethyl-N-(2-hydroxy-3-sulfopropyl)-3-methylaniline sodium salt (TOOS) solution
(Reagent 4) 300 U/ml horseradish peroxidase (POD) solution
(Reagent 5) 300 mM sodium hydrogen glutamate solution

B: Method for measurement of activity

300 $\mu$l of Reagent 1, 12.5 $\mu$l of Reagent 2, 25 $\mu$l of Reagent 3, 12.5 $\mu$l of Reagent 4, (375 - V) $\mu$l of deionized water, and V $\mu$l of the GLOD solution are mixed and kept at a temperature of 30°C for 5 minutes. Subsequently, 25 $\mu$l of Reagent 5 is added, the resultant is thoroughly mixed, and the light absorbance at a wavelength of 555 nm ($A_{555}$) is then measured using spectrophotometer U-3900 (manufactured by Hitachi High-Tech Science Corporation), while maintaining the cell holder thereof at a temperature of 30°C, to compute the change in $A_{555}$ per minute ($\Delta A_s$). Incidentally, as a control experiment, 25 $\mu$l of deionized water is added instead of 25 $\mu$l of Reagent 5, and the light absorbance at a wavelength of 555 nm ($A_{555}$) is then measured to compute the change in $A_{555}$ per minute ($\Delta A_0$).

Oxidase activity (U/ml) may be calculated according to the calculation formula given below. In the formula, "39.2" represents the millimolar extinction coefficient ($mM^{-1}cm^{-1}$) of quinoneimine dye formed by the condensation between 4-AA and TOOS, against light at a wavelength of 555 nm.

[Formula]

$$U/ml = (\Delta A_S - \Delta A_0) \times 600 \times df / (39.2 \times 0.5 \times V)$$

$$= 30.6 \times (\Delta A_S - \Delta A_0) \times df / V.$$

In the case of using different chromogenic reagents, wavelengths appropriate for the chromogenic reagents and millimolar extinction coefficients at the wavelengths may be used.

In one embodiment, the present disclosure provides a polynucleotide encoding a glutamate oxidase mutant. In one embodiment, the present disclosure provides a vector comprising such a polynucleotide. In one embodiment, the present disclosure provides a host cell transformed with such a vector, that is, a host cell comprising such vector. In one embodiment, the present disclosure provides a method for producing glutamate oxidase mutant comprising the steps of: culturing such host cell to produce a glutamate oxidase mutant, and obtaining the produced glutamate oxidase mutant. In one embodiment, the present disclosure provides a method for bringing a glutamate oxidase mutant or a composition, reagent, electrode, sensor or kit comprising this into contact with a sample containing glutamic acid to oxidize the glutamic acid contained in the sample. In one embodiment, this method is capable of detecting glutamic acid. In one embodiment, this method is capable of measuring glutamic acid.

(Method for preparing further GLOD(s))

The present inventor prepared a GLODΔ having a particular amino acid sequence deletion introduced into GLOD. Further, a GLODΔ-T having a particular amino acid substitution introduced into GLODΔ was prepared. The deletion and mutation of the present disclosure can appropriately be combined. Based on the findings of the present disclosure, further mutants may be prepared. As such, in one embodiment, the present invention provides a method for modifying a GLOD or GLODΔ to prepare a thermal stability improvement type GLOD mutant, comprising the following steps:

(i) obtaining a GLOD gene or GLODΔ gene,
(ii) integrating the GLOD gene or GLODΔ gene into a vector, transforming a host cell, expressing the GLOD or GLODΔ, and isolating the expressed product,
(iii) measuring the activity of the expressed product,
(iv) subjecting the expressed product to heat treatment and then measuring the residual activity thereof,
(v) modifying said GLOD gene or GLODΔ gene such that, when the amino acid sequence of the GLOD or GLODΔ is aligned with the amino acid sequence of SEQ ID NO: 1, the amino acid at the position corresponding to a position selected from the group consisting of the 87th, 103rd, 133rd, 186th, 297th, 376th, 393rd, 428th, 516th, 566th, 568th, 585th, and 615th positions of SEQ ID NO: 1 is substituted with, for example, an amino acid selected from the group consisting of tyrosine, phenylalanine, tryptophan, leucine, isoleucine, valine and methionine,
(vi) integrating the modified gene into a vector, transforming a host cell, expressing the variant, and isolating the expressed product;
(vii) subjecting the expressed product of the variant to heat treatment, and then measuring the residual activity thereof, and comparing the measured value with the value measured in step (iv),
(viii) when the residual activity after the heat treatment of the modified variant is increased by 5%, 6%, 7%, 8%, 9%, or 10% or more compared to the residual activity after the heat treatment of the GLOD or GLODΔ before modification, designating the modified variant as a thermal stability improved type GLOD mutant,
(ix) optionally repeating steps (v) to (vii) on the variant of step (viii), and
(x) formulating the thermal stability improved type GLOD mutant of step (viii) or (ix) into a kit or composition.

In a particular embodiment, with regard to the GLOD mutant of the present disclosure, for example, GLODΔ or GLODΔ-T, a peptide linker may be inserted into a region in which an amino acid sequence was deleted. The peptide linker may be, for example, a linker composed of 1 to 20 amino acid residues. The peptide linker may be composed of an amino acid sequence different from a partial amino acid sequence of a GLOD. The peptide linker may be composed of, for example, 2, 3, 4, 5, 6, 7, 8, 9, 10, 11, 12, 13, 14, 15, 16, 17, 18, 19 or 20 amino acid residues. The peptide linker may be composed of, for example, 2 to 19, 3 to 18, 4 to 17, 5 to 16, 6 to 15, for example, 7 to 14 amino acid residues. The amino acid residues constituting the peptide linker may be natural amino acids and glycine. Examples thereof include Ala, Asn, Cys, Gln, Ile, Leu, Met, Phe, Pro, Ser, Thr, Trp, Tyr, Val, Asp, Glu, Arg, His, and Lys. Examples thereof include Gly, Ala, Ser, and Thr. In one embodiment, the peptide linker may be GGGGS or a repeated sequence thereof. The number of repeats may be 2, 3, or 4. In one embodiment, the GLOD mutant of the present disclosure, for example, GLODΔ or GLODΔ-T, has no peptide linker.

In one embodiment, the GLOD mutant of the present disclosure, for example, GLODΔ or GLODΔ-T, comprises an amino acid sequence identity of 90% or more with SEQ ID NO: 1. If a deletion Δ is present, the same is not included in the calculation of the sequence identity. In another embodiment, sequences having an amino acid sequence identity of 90% or more with SEQ ID NO: 1 are excluded from the GLOD mutant of the present disclosure, for example, GLODΔ or GLODΔ-T.

In one embodiment, the GLOD mutant of the present disclosure, for example, GLODΔ or GLODΔ-T, comprises an amino acid sequence identity of 90% or more with SEQ ID NO: 58. If a deletion Δ is present, the same is not included in the calculation of the sequence identity. In another embodiment, sequences having an amino acid sequence identity of 90% or more with SEQ ID NO: 58 are excluded from the GLOD mutant of the present disclosure, for example, GLODΔ or GLODΔ-T. In one embodiment, known GLODs or known GLOD mutants are excluded from the GLOD mutant of the present disclosure, for example, GLODΔ or GLODΔ-T.

In a mutant comprising a deletion of the 459th to 507th positions of SEQ ID NO: 1, amino acid numbers are shifted. The position corresponding to the 516th position of SEQ ID NO: 1 is the 467th position counted from the N terminus of the deletion mutant. The position corresponding to the 566th position of SEQ ID NO: 1 is the 517th position counted from the N terminus of the deletion mutant. The position corresponding to the 568th position of SEQ ID NO: 1 is the 519th position counted from the N terminus of the deletion mutant. The position corresponding to the 585th position of SEQ ID NO: 1 is the 536th position counted from the N terminus of the deletion mutant. The position corresponding to the 615th position of SEQ ID NO: 1 is the 566th position counted from the N terminus of the deletion mutant.

Examples

The GLOD of the present disclosure will be further illustrated by way of the following Examples. However, these examples are provided for mere illustration and the present disclosure is not limited by these examples in any way.

[Example 1] Improvement in heat resistance of glutamate oxidase (StGLOD) from *Streptomyces* sp. X-119-6 by deletion of amino acid sequence

1. Construction of plasmid for glutamate oxidase (GLOD) expression

A plasmid (pKK223-3-StGLOD) for expression of a glutamate oxidase from *Streptomyces* sp. X-119-6 (StGLOD) having the amino acid sequence of SEQ ID NO: 1 was prepared using NEBuilder HiFi DNA assembly (manufactured by New England Biolabs Inc.).

StGLOD gene having the nucleotide sequence of SEQ ID NO: 2 was divided into 3 fragments of SEQ ID NO: 3 (StGLOD-f1), SEQ ID NO: 4 (StGLOD-f2) and SEQ ID NO: 5 (StGLOD-f3) and their synthesis was commissioned to Integrated DNA Technologies, Inc. Fifteen 3' terminal bases of StGLOD-f1 and Fifteen 5' terminal bases of StGLOD-f2 are overlapping sequences for gene assembly. Fifteen 3' terminal bases of StGLOD-f2 and Fifteen 5' terminal bases of StGLOD-f3 are overlapping sequences for gene assembly.

A plasmid fragment was prepared by PCR using pKK223 plasmid as the template and primers of SEQ ID NO: 6 (ggtcatttcattcatgaattctgtttcctgtgtgaaattg) and SEQ ID NO: 7 (gcgttaacttcttaagcttggctgttttggcggatgag). The primer of SEQ ID NO: 6 or SEQ ID NO: 7 had a sequence of 15 bases overlapping with StGLOD-f1 or StGLOD-f3, respectively, which was added to the 5' end of the sequence that anneals to pKK223-3. The solution after PCR was supplemented with 1.0 μl of DpnI (manufactured by New England BioLabs Inc.) and treated at 37°C for 1 hour, and then, the amplified fragment was purified using GFX PCR DNA and Gel Band Purification Kit (manufactured by Cytiva).

The plasmid (pKK223-3-StGLOD) for StGLOD expression was obtained through reaction at 50°C for 60 minutes according to the composition of the table below. *E. coli* JM109 strain was transformed with the obtained plasmid. The obtained transformant was cultured, and the nucleotide sequence of an extracted plasmid was confirmed by DNA sequence analysis to be the intended sequence.

[Table 5]

| Reagent | Volume |
|---|---|
| NEBuilder HiFi DNA assembly | 10.0 μl |
| 32 ng/μl pKK223-3 fragment | 3.1 μl |
| 40 ng/μl StGLOD-f1 | 2.3 μl |
| 40 ng/μl StGLOD-f2 | 2.3 μl |
| 40 ng/μl StGLOD-f3 | 2.3 μl |

(continued)

| Reagent | Volume |
|---|---|
| Total volume | 20.0 μl |

2. Preparation of StGLOD deletion mutant

A plasmid carrying a gene encoding a StGLOD deletion mutant was obtained by site directed mutagenesis with pKK223-3-StGLOD as the template. The PCR reaction solution was prepared by mixing 10 μl of KOD one PCR Master Mix (manufactured by Toyobo Co., Ltd.), 3 μl of 2 μM Fw primer, 3 μl of 2 μM Rv primer, 0.5 μl of 40 μg/ml template DNA (pKK223-3-StGLOD), and 3.5 μl of ion exchange water. A deletion mutant prepared using primers of SEQ ID NO: 8 (gataagaccgaagcgaccaatgcgtacgga) and SEQ ID NO: 9 (cagcttacgataatagtcgtacagacccgg) was designated as StGLODΔ49Ai, and a deletion mutant prepared using primers of SEQ ID NO: 10 (gcagagccacctgcgaccaatgcgtacgga) and SEQ ID NO: 11 (cgcatcttgataatagtcgtacagacccgg) was designated as StGLODΔ49C. The amino acid sequence of StGLODΔ49Ai or StGLODΔ49C is shown in SEQ ID NO: 12 or SEQ ID NO: 13, respectively. PCR reaction conditions involved a cycle of "98°C for 10 sec -> 55°C for 5 sec -> 68°C for 35 sec" and this was repeated 7 times.

The solution after PCR was supplemented with 1 μl of DpnI and treated at 37°C for 1 hour to degrade the template pKK223-3-StGLOD. 2 μl of the obtained solution treated with DpnI was mixed with 5 μl of Ligation High Ver. 2 (manufactured by Toyobo Co., Ltd.), 1 μl of 5 U/μl T4 polynucleotide kinase, and 7 μl of ion exchange water and reacted at 16°C for 1 hour, and then, *E. coli* JM109 strain was transformed with the reaction solution. The obtained transformant was cultured, and the nucleotide sequence of an extracted plasmid was confirmed by DNA sequence analysis to be the intended sequence.

The solution after PCR was supplemented with 1 μl of DpnI and treated at 37°C for 1 hour to degrade the template pKK223-3-StGLODΔ49C. *E. coli* JM109 strain was transformed with the obtained solution treated with DpnI. The obtained transformant was cultured, and the nucleotide sequence of the extracted plasmid was confirmed by DNA sequence analysis to be the intended sequence.

3. Recombinant production of GLOD

A GLOD producing strain was inoculated to 2.5 ml of LB-amp medium (ampicillin concentration: 50 μg/ml) added to a test tube, and cultured overnight at 160 rpm at 37°C. 2.5 ml of a seed culture solution was inoculated to 250 ml of LB-amp medium (ampicillin concentration: 50 μg/ml) containing 0.1 mM IPTG added to a Sakaguchi flask, and cultured at 130 rpm at 25°C for 16 hours.

The culture solution was centrifuged at 8,000 rpm for 10 minutes and the obtained pellets were resuspended in 4 ml of 10 mM potassium phosphate buffer (PPB), pH 7.5. The bacterial body suspension was ultrasonicated and then centrifuged at 15,000 rpm for 15 minutes, and the obtained supernatant was recovered as the GLOD crude enzyme solution.

4. Measurement of GLOD activity

4-Aminoantipyrine (4-AA) (manufactured by FUJIFILM Wako Pure Chemical Corporation), TOOS (manufactured by Dojindo Laboratories), and horseradish peroxidase (POD) (manufactured by Toyobo Co., Ltd.) were used as a reagent for measurement of GLOD activity. The composition of the reagent for activity measurement is shown in Table 2. A GLOD solution was diluted with 10 mM PPB (pH 7.4) containing 0.15% bovine serum albumin (BSA, manufactured by Sigma-Aldrich).

[Table 6]

| Reagent | Volume | Final concentration |
|---|---|---|
| 250 mM PPB pH 7.4 | 300 μl | 100 mM |
| 30 mM 4-AA | 12.5 μl | 0.5 mM |
| 15 mM TOOS | 25 μl | 0.5 mM |
| 300 U/ml POD | 12.5 μl | 5 U/ml |
| Deionized water | (375 - V) μl | |
| GLOD solution | V μl | |

(continued)

| Reagent | Volume | Final concentration |
|---------|--------|---------------------|
| Total volume | 725 μl | |

725 μl of the reagent of the above table was kept at a temperature of 30°C for 5 minutes and then supplemented and mixed with 25 μl of 300 mM sodium hydrogen glutamate (GluNa, manufactured by FUJIFILM Wako Pure Chemical Corporation) solution, and the light absorbance at a wavelength of 555 nm ($A_{555}$) was measured using spectrophotometer U-3900 (manufactured by Hitachi High-Tech Science Corporation), while maintaining the cell holder thereof at a temperature of 30°C, to compute the change in $A_{555}$ per minute ($\Delta A_s$). 25 μl of ion exchange water was added instead of the substrate solution (GluNa solution), and the same measurement as above was performed to compute the change in $A_{555}$ per minute ($\Delta A_0$).

Oxidase activity (U/ml) was calculated according to the calculation formula given below. In the formula, "39.2" represents the millimolar extinction coefficient ($mM^{-1}cm^{-1}$) of quinoneimine dye formed by the condensation between 4-AA and TOOS, against light at a wavelength of 555 nm.

[Formula]

$$U/ml = (\Delta A_S - \Delta A_0) \times 600 \times df / (39.2 \times 0.5 \times V)$$

$$= 30.6 \times (\Delta A_S - \Delta A_0) \times df / V.$$

5. Evaluation of thermal stability of GLOD

A GLOD crude enzyme solution was diluted with 10 mM PPB, pH 6.0 such that the final concentration of the GLOD was 0.05 U/ml. Next, 240 μl of 0.05 U/ml GLOD solution and 160 μl of 250 mM PPB, pH 6.0 were mixed and heated for 30 minutes or 35 minutes in a water bath kept at a predetermined temperature. The GLOD solution thus heated was immediately cooled on ice and activity was measured using 375 μl of the GLOD solution. The residual activity of the heated sample was calculated when the activity of a sample cooled on ice without being heated was defined as 1. The residual activity was calculated three times for each GLOD and thermal stability was evaluated from an average value thereof.

Table 7 shows the residual activity of StGLOD deletion mutants heated at 45°C for 30 minutes.

[Table 7]

| Mutant name | Residual activity |
|-------------|-------------------|
| StGLOD | 0.57 |
| StGLODΔ49Ai | 0.68 |
| StGLODΔ49C | 0.94 |

The residual activities of StGLOD deletion mutants shown in the table above were increased by 0.11 to 0.37 compared to StGLOD and the thermal stabilities of all StGLOD deletion mutants were improved. In other words, the residual activities were improved by about 20% and about 65% compared to the wild type.

[Example 2] Improvement in heat resistance of StGLODΔ49C by amino acid substitution 6. Preparation of modified StGLODΔ49C

The expression and residual activity of StGLODΔ49C were confirmed. As such, modified mutants based on StGLODΔ49C were further prepared.

A plasmid carrying a gene encoding a modified StGLOD was obtained by site directed mutagenesis using a plasmid for StGLODΔ49C expression (pKK223-3-StGLODΔ49C) as the template. The PCR reaction solution was prepared by mixing 10 μl of KOD one PCR Master Mix (manufactured by Toyobo Co., Ltd.), 3 μl of 2 μM Fw primer, 3 μl of 2 μM Rv primer, 0.5 μl of 40 μg/ml template DNA (pKK223-3-StGLOD), and 3.5 μl of ion exchange water. Table 8 shows the names of prepared mutants and combinations of Fw and Rv primers. PCR reaction conditions involved a cycle of "98°C for 10 sec -> 55°C for 5 sec -> 68°C for 35 sec" and this was repeated 15 times. A plasmid for expression of multiple mutation type StGLODΔ49C was constructed by repeating introduction of single mutations. For example, a plasmid for expression of a double mutation type StGLODΔ49C/F87Y/Y103I was constructed by PCR using pKK223-3-StGLODΔ49C/F87Y as the template and primers of SEQ ID NO: 20 and SEQ ID NO: 16.

[Table 8]

| Mutant name | Fw primer | Rv primer |
|---|---|---|
| StGLODΔ49C/F87Y | cgtatcaagacgtaccatgcaaaa aaagga (SEQ ID NO: 15) | cgtcttgatacgccctcccacacg gttggc (SEQ ID NO: 14) |
| StGLODΔ49C/Y103F | gatccagcgcagttcgcggaagct ggggcg (SEQ ID NO: 17) | ctgcgctggatcagcgaatggaga tggctc (SEQ ID NO: 16) |
| StGLODΔ49C/Y103L | gatccagcgcagttagcggaagct ggggcg (SEQ ID NO: 18) | ctgcgctggatcagcgaatggaga tggctc (SEQ ID NO: 16) |
| StGLODΔ49C/Y103V | gatccagcgcaggtcgcggaagct ggggcg (SEQ ID NO: 19) | ctgcgctggatcagcgaatggaga tggctc (SEQ ID NO: 16) |
| StGLODΔ49C/Y103I | gatccagcgcagatcgcggaagct ggggcg (SEQ ID NO: 20) | ctgcgctggatcagcgaatggaga tggctc (SEQ ID NO: 16) |
| StGLODΔ49C/Y103M | gatccagcgcagatggcggaagct ggggcg (SEQ ID NO: 21) | ctgcgctggatcagcgaatggaga tggctc (SEQ ID NO: 16) |
| StGLODΔ49C/F133Y | cgccgcctgtttttataatgtagat attgac (SEQ ID NO: 23) | aaacaggcggcgtttttaagcccag cttgtc (SEQ ID NO: 22) |
| StGLODΔ49C/F133L | cgccgcctgttttttaaatgtagat attgac (SEQ ID NO: 24) | aaacaggcggcgtttttaagcccag cttgtc (SEQ ID NO: 22) |
| StGLODΔ49C/F297L | caccttgccttttttacattcgttt ttgggc (SEQ ID NO: 26) | aaaggcaaggtgcaggcgcgaggt catgtt (SEQ ID NO: 25) |
| StGLODΔ49C/F297V | caccttgcctttgttcattcgttt ttgggc (SEQ ID NO: 27) | aaaggcaaggtgcaggcgcgaggt catgtt (SEQ ID NO: 25) |
| StGLODΔ49C/F297I | caccttgcctttattcattcgttt ttgggc (SEQ ID NO: 28) | aaaggcaaggtgcaggcgcgaggt catgtt (SEQ ID NO: 25) |
| StGLODΔ49C/F297M | caccttgcctttatgcattcgttt ttgggc (SEQ ID NO: 29) | aaaggcaaggtgcaggcgcgaggt catgtt (SEQ ID NO: 25) |
| StGLODΔ49C/Y376F | gagggagagcctttcgccgcaact caaacg (SEQ ID NO: 31) | aggctctccctctggaaccgtctg aatggc (SEQ ID NO: 30) |
| StGLODΔ49C/Y376L | gagggagagcctttagccgcaact caaacg (SEQ ID NO: 32) | aggctctccctctggaaccgtctg aatggc (SEQ ID NO: 30) |
| StGLODΔ49C/Y376I | gagggagagcctatcgccgcaact caaacg (SEQ ID NO: 33) | aggctctccctctggaaccgtctg aatggc (SEQ ID NO: 30) |
| StGLODΔ49C/Y376M | gagggagagcctatggccgcaact caaacg (SEQ ID NO: 34) | aggctctccctctggaaccgtctg aatggc (SEQ ID NO: 30) |
| StGLODΔ49C/F393L | gttactatccccttaagctcttta cgcttt (SEQ ID NO: 36) | ggggatagtaacaatcgccaggtc tcctgt (SEQ ID NO: 35) |
| StGLODΔ49C/F393V | gttactatccccgttagctcttta cgcttt (SEQ ID NO: 37) | ggggatagtaacaatcgccaggtc tcctgt (SEQ ID NO: 35) |

(continued)

| Mutant name | Fw primer | Rv primer |
|---|---|---|
| StGLODΔ49C/F3931 | gttactatccccattagctcttta cgcttt (SEQ ID NO: 38) | ggggatagtaacaatcgccaggtc tcctgt (SEQ ID NO: 35) |
| StGLODΔ49C/F393M | gttactatccccatgagctcttta cgcttt (SEQ ID NO: 39) | ggggatagtaacaatcgccaggtc tcctgt (SEQ ID NO: 35) |
| StGLODΔ49C/F428Y | gtcttacttgaatatagccgtcgt tggtgg (SEQ ID NO: 41) | ttcaagtaagactttggtggcctg atcata (SEQ ID NO: 40) |
| StGLODΔ49C/F428M | gtcttacttgaaatgagccgtcgt tggtgg (SEQ ID NO: 42) | ttcaagtaagactttggtggcctg atcata (SEQ ID NO: 40) |
| StGLODΔ49C/Y467F | gcgaccaatgcgttcggaggggggt agtacg (SEQ ID NO: 44) | cgcattggtcgcaggtggctctgc cgcatc (SEQ ID NO: 43) |
| StGLODΔ49C/Y517F | gatgctgagcgctttggctatgcg cttgaa (SEQ ID NO: 46) | gcgctcagcatcgtcaaaagaatc ccaacg (SEQ ID NO: 45) |
| StGLODΔ49C/Y517L | gatgctgagcgcttaggctatgcg cttgaa (SEQ ID NO: 47) | gcgctcagcatcgtcaaaagaatc ccaacg (SEQ ID NO: 45) |
| StGLODΔ49C/Y517V | gatgctgagcgcgttggctatgcg cttgaa (SEQ ID NO: 48) | gcgctcagcatcgtcaaaagaatc ccaacg (SEQ ID NO: 45) |
| StGLODΔ49C/Y517M | gatgctgagcgcatgggctatgcg cttgaa (SEQ ID NO: 49) | gcgctcagcatcgtcaaaagaatc ccaacg (SEQ ID NO: 45) |
| StGLODΔ49C/Y5191 | gagcgctatggcattgcgcttgaa aactta (SEQ ID NO: 51) | gccatagcgctcagcatcgtcaaa agaatc (SEQ ID NO: 50) |
| StGLODΔ49C/Y519M | gagcgctatggcatggcgcttgaa aactta (SEQ ID NO: 52) | gccatagcgctcagcatcgtcaaa agaatc (SEQ ID NO: 50) |
| StGLODΔ49C/Y536L | attgaggtgttcttaacaggagcg ggacag (SEQ ID NO: 54) | gaacacctcaatacgacggccgtg gacaga (SEQ ID NO: 53) |
| StGLODΔ49C/Y536M | attgaggtgttcatgacaggagcg ggacag (SEQ ID NO: 55) | gaacacctcaatacgacggccgtg gacaga (SEQ ID NO: 53) |
| StGLODΔ49C/F566L | caaatgaccgcgttacacttagat gtggtg (SEQ ID NO: 57) | cgcggtcatttgatggggtgtata gacagc (SEQ ID NO: 56) |

PCR, recombinant production of GLOD, activity measurement and thermal stability evaluation of the prepared mutants were all carried out in the same manner as in the above StGLODΔ49C. The following table shows the residual activity of modified StGLODΔ49C heated at 50°C for 35 minutes.

[Table 9]

| Mutant name | Residual activity |
|---|---|
| StGLODΔ49C | 0.22 |
| StGLODΔ49C/F87Y | 0.61 |
| StGLODΔ49C/Y103F | 0.26 |

(continued)

| Mutant name | Residual activity |
|---|---|
| StGLOD∆49C/Y103L | 0.36 |
| StGLOD∆49C/Y103V | 0.33 |
| StGLOD∆49C/Y103I | 0.32 |
| StGLOD∆49C/Y103M | 0.32 |
| StGLOD∆49C/F133Y | 0.37 |
| StGLOD∆49C/F133L | 0.24 |
| StGLOD∆49C/F297L | 0.59 |
| StGLOD∆49C/F297V | 0.53 |
| StGLOD∆49C/F297I | 0.75 |
| StGLOD∆49C/F297M | 0.74 |
| StGLOD∆49C/Y376F | 0.27 |
| StGLOD∆49C/Y376L | 0.28 |
| StGLOD∆49C/Y376I | 0.25 |
| StGLOD∆49C/Y376M | 0.27 |
| StGLOD∆49C/F393L | 0.55 |
| StGLOD∆49C/F393V | 0.26 |
| StGLOD∆49C/F393I | 0.25 |
| StGLOD∆49C/F393M | 0.24 |
| StGLOD∆49C/F428Y | 0.79 |
| StGLOD∆49C/F428M | 0.29 |
| StGLOD∆49C/Y467F | 0.25 |
| StGLOD∆49C/Y517F | 0.92 |
| StGLOD∆49C/Y517L | 0.74 |
| StGLOD∆49C/Y517V | 0.94 |
| StGLOD∆49C/Y517M | 0.87 |
| StGLOD∆49C/Y519I | 0.31 |
| StGLOD∆49C/Y519M | 0.26 |
| StGLOD∆49C/Y536L | 0.51 |
| StGLOD∆49C/Y536M | 0.25 |
| StGLOD∆49C/F566L | 0.43 |

The residual activities of the modified StGLOD∆49C shown in the table above were increased by 0.02 to 0.72 compared to StGLOD∆49C and the thermal stabilities of all the modified StGLODA49Cs were improved. In other words, the residual activities of the prepared modified enzymes were improved by about 9% or more to about 325% or more compared to StGLOD∆49C before modification.

An octuple mutant was prepared by combining 8 types of amino acid substitutions (F87Y, Y103I, F133Y, F297M, F393L, F428Y, Y517F, Y536L), which contributed to the improved thermal stability of StGLOD∆49C, and heated under more harsh heat treatment conditions (60°C, 65°C or 70°C for 35 min) than those of Table 9. The following table shows the residual activity of the StGLOD deletion mutant thereafter.

[Table 10]

| Heat treatment conditions | Residual activity |
|---|---|
| 60°C, 35 min | 0.90 |
| 65°C, 35 min | 0.78 |
| 70°C, 35 min | 0.36 |

The octuple mutant of StGLODΔ49C shown in the above table was stable even when heated at 60°C for 35 minutes and 1/3 or more of the activity remained even when the mutant was heated at 70°C for 35 minutes. It became evident that the thermal stability of StGLODΔ49C was drastically improved by combining the amino acid substitutions shown in Table 9.

[Example 3] Obtaining thermal stability improved type StGLOD homolog(s)

7. Construction of plasmid for expression of StGLOD homolog(s) having thermal stability improving mutation

A putative glutamate oxidase from *Streptomyces* sp. MOE7 (M7GLOD) having the amino acid sequence of SEQ ID NO: 58 was designed so as to have an amino acid sequence having the same deletion as that of StGLODΔ49C (M7GLODΔ49C, SEQ ID NO: 59) and further designed so as to have an amino acid sequence having 8 types of amino acid substitutions (F87Y, Y103I, F133Y, F297M, F393L, F428Y, Y517F, Y536L) introduced therein (M7GLODΔ49C-T8, SEQ ID NO: 60).

A plasmid (pET22b-M7GLODA49C-T8) for M7GLODΔ49C-T8 expression was prepared using In-Fusion HD Cloning Kit (manufactured by Clontech Laboratories, Inc.). M7GLODΔ49C-T8 gene having the nucleotide sequence of SEQ ID NO: 61 was divided into 3 fragments of SEQ ID NO: 62 (M7GLODΔ49C-T8-f1), SEQ ID NO: 63 (M7GLODA49C-T8-f2) and SEQ ID NO: 64 (M7GLODA49C-T8-f3) and their synthesis was commissioned to Integrated DNA Technologies, Inc. Fifteen 5' terminal bases of M7GLODA49C-T8-f1 are an overlapping sequence for assembly with pET-22b(+). Fifteen 3' terminal bases of M7GLODΔ49C-T8-f1 and fifteen 5' terminal bases of M7GLODΔ49C-T8-f2 are overlapping sequences for gene assembly. Fifteen 3' terminal bases of M7GLODΔ49C-T8-f2 and fifteen 5' terminal bases of M7GLODΔ49C-T8-f3 are overlapping sequences for gene assembly. Fifteen 3' terminal bases of M7GLODΔ49C-T8-f3 are an overlapping sequence for assembly with pET-22b(+).

A plasmid fragment was prepared by PCR using pET-22b(+) plasmid as the template and primers of SEQ ID NO: 65 (catatgtatatctccttcttaaag) and SEQ ID NO: 66 (taacaaagcccgaaaggaag). The solution after PCR was supplemented with 1.0 μl of DpnI (manufactured by New England BioLabs Inc.) and treated at 37°C for 1 hour, and then, the amplified fragment was purified using GFX PCR DNA and Gel Band Purification Kit (manufactured by Cytiva).

The plasmid (pET22b-M7GLODΔ49C-T8) for M7GLODΔ49C-T8 expression was obtained through reaction at 50°C for 15 minutes according to the composition of the table below. *E. coli* JM109 strain was transformed with the obtained plasmid. The obtained transformant was cultured, and the nucleotide sequence of an extracted plasmid was confirmed by DNA sequence analysis to be the intended sequence. Then, *E. coli* BL21 (DE3) strain was transformed with pET22b-M7GLODΔ49C-T8 to prepare a M7GLODΔ49C-T8 producing strain.

[Table 11]

| Reagent | Volume |
|---|---|
| 5×In-Fusion HD Enzyme Premix | 2 μl |
| 50 ng/μl pET-22b(+) fragment | 2 μl |
| 10 ng/μl M7GLODΔ49C-8T-f1 | 2 μl |
| 10 ng/μl M7GLODΔ49C-8T-f2 | 2 μl |
| 10 ng/μl M7GLODΔ49C-8T-f3 | 2 μl |
| Total volume | 10 μl |

A plasmid (pET22b-M7GLODΔ49C-T7) carrying a gene encoding M7GLODΔ49C-T8 not comprising the amino acid substitution Y536L was obtained in accordance with the method described in "6. Preparation of modified StGLODΔ49C" using pET22b-M7GLODΔ49C-T8 as the template and primers of SEQ ID NO: 67 (atcgaggtctttatactg-gagctggacaa) and SEQ ID NO: 68 (aaagacctcgatgcggcggccatggaccga). M7GLODΔ49C-T8 not comprising the amino acid substitution Y536L was designated as M7GLODΔ49C-T7 (SEQ ID NO: 69).

A plasmid for expression of each modified M7GLODΔ49C-T7 was constructed with the same method as in "6. Preparation of modified StGLODΔ49C" using pET22b-M7GLODΔ49C-T7 as the template and primers of Table 12. Table 12 shows the names of prepared mutants and combinations of Fw and Rv primers.

[Table 12]

| Mutant name | Fw primer | Rv primer |
|---|---|---|
| M7GLODΔ49C-T7/R186G | attcgtaccaatggcactcaagt acgtcgc (SEQ ID NO: 71) | attggtacgaatccaagtgtggtt acg (SEQ ID NO: 70) |
| M7GLODΔ49C-T7/R186A | attcgtaccaatgcgactcaagt acgtcgc (SEQ ID NO: 72) | attggtacgaatccaagtgtggtt acg (SEQ ID NO: 70) |
| M7GLODΔ49C-T7/R186C | attcgtaccaattgcactcaagt acgtcgc (SEQ ID NO: 73) | attggtacgaatccaagtgtggtt acg (SEQ ID NO: 70) |
| M7GLODΔ49C-T7/R186L | attcgtaccaatctgactcaagt acgtcgc (SEQ ID NO: 74) | attggtacgaatccaagtgtggtt acg (SEQ ID NO: 70) |
| M7GLODΔ49C-T7/R186M | attcgtaccaatatgactcaagt acgtcgc (SEQ ID NO: 75) | attggtacgaatccaagtgtggtt acg (SEQ ID NO: 70) |
| M7GLODΔ49C-T7/R186F | attcgtaccaatttttactcaagt acgtcgc (SEQ ID NO: 76) | attggtacgaatccaagtgtggtt acg (SEQ ID NO: 70) |
| M7GLODΔ49C-T7/R186Y | attcgtaccaattatactcaagt acgtcgc (SEQ ID NO: 77) | attggtacgaatccaagtgtggtt acg (SEQ ID NO: 70) |
| M7GLODΔ49C-T7/R186S | attcgtaccaatagcactcaagt acgtcgc (SEQ ID NO: 78) | attggtacgaatccaagtgtggtt acg (SEQ ID NO: 70) |
| M7GLODΔ49C-T7/R186T | attcgtaccaataccactcaagt acgtcgc (SEQ ID NO: 79) | attggtacgaatccaagtgtggtt acg (SEQ ID NO: 70) |
| M7GLODΔ49C-T7/R186N | attcgtaccaataacactcaagt acgtcgc (SEQ ID NO: 80) | attggtacgaatccaagtgtggtt acg (SEQ ID NO: 70) |
| M7GLODΔ49C-T7/R186Q | attcgtaccaatcagactcaagt acgtcgc (SEQ ID NO: 81) | attggtacgaatccaagtgtggtt acg (SEQ ID NO: 70) |
| M7GLODΔ49C-T7/R186H | attcgtaccaatcatactcaagt acgtcgc (SEQ ID NO: 82) | attggtacgaatccaagtgtggtt acg (SEQ ID NO: 70) |
| M7GLODΔ49C-T7/R186D | attcgtaccaatgatactcaagt acgtcgc (SEQ ID NO: 83) | attggtacgaatccaagtgtggtt acg (SEQ ID NO: 70) |
| M7GLODΔ49C-T7/R186E | attcgtaccaatgaaactcaagt acgtcgc (SEQ ID NO: 84) | attggtacgaatccaagtgtggtt acg (SEQ ID NO: 70) |

Next, *E. coli* BL21 (DE3) strain was transformed with pET22b-M7GLODΔ49C-T7 or the plasmid for expression of each modified M7GLODΔ49C-T7 to prepare M7GLODΔ49C-T7 or each modified M7GLODΔ49C-T7 producing strain.

8. Recombinant production of M7GLODΔ49C-T7 and thermal stability evaluation

A M7GLODΔ49C-T7 or modified M7GLODΔ49C-T7 producing strain was inoculated to 2.5 ml of LB-amp medium (ampicillin concentration: 50 μg/ml) added to a test tube, and cultured overnight at 180 rpm at 37°C. 2.5 ml of a seed culture solution was inoculated to 250 ml of LB-amp medium (ampicillin concentration: 100 μg/ml) added to a Sakaguchi flask, and cultured at 130 rpm at 37°C. At the stage where OD600 of the culture solution reached 0.6 to 1.0, isopropyl-β-D-thiogalactopyranoside (IPTG) was added at a final concentration of 1 mM or 0.1 mM, followed by culture at 130 rpm at 15°C for 16 hours.

The culture solution was centrifuged at 8,000 rpm for 10 minutes and the obtained pellets were resuspended in 4 to 25 ml of 10 mM potassium phosphate buffer (PPB), pH 6.0. The bacterial body suspension was ultrasonicated and then centrifuged at 15,000 rpm for 15 minutes, and the obtained supernatant was recovered to prepare a M7GLODΔ49C-T7 or modified M7GLODΔ49C-T7 crude enzyme solution.

M7GLODΔ49C-T7 or the modified M7GLODΔ49C-T7 was heated at 70°C for 30 minutes and the residual activity thereof was calculated in accordance with the above method (Table 13). M7GLODΔ49C-T7 was also heated at 60°C or 65°C for 30 minutes and the residual activity thereof was calculated.

The residual activity of M7GLODΔ49C-T7 heated at 60°C for 30 minutes was 0.93, and the residual activity of M7GLODΔ49C-T7 heated at 65°C for 30 minutes was 0.69, indicating high thermal stability. It was demonstrated that the thermal stability of GLOD was improved by combining the amino acid substitutions shown in Table 9, without being limited to StGLOD.

[Table 13]

| Mutant name | Residual activity after treatment at 70°C for 30 min |
|---|---|
| M7GLODΔ49C-T7 | 0.17 |
| M7GLODΔ49C-T7/R186G | 0.33 |
| M7GLODΔ49C-T7/R186A | 0.43 |
| M7GLODΔ49C-T7/R186C | 0.26 |
| M7GLODΔ49C-T7/R186L | 0.29 |
| M7GLODΔ49C-T7/R186M | 0.29 |
| M7GLODΔ49C-T7/R186F | 0.22 |
| M7GLODΔ49C-T7/R186Y | 0.56 |
| M7GLODΔ49C-T7/R186S | 0.37 |
| M7GLODΔ49C-T7/R186T | 0.25 |
| M7GLODΔ49C-T7/R186N | 0.54 |
| M7GLODΔ49C-T7/R186Q | 0.54 |
| M7GLODΔ49C-T7/R186H | 0.34 |
| M7GLODΔ49C-T7/R186D | 0.66 |
| M7GLODΔ49C-T7/R186E | 0.75 |

Furthermore, the residual activities of each modified M7GLODΔ49C-T7 were improved by 0.05 to 0.58 compared to M7GLODΔ49C-T7 and the thermal stabilities of all modified M7GLODΔ49C-T7 were improved. In other words, the residual activities of the prepared modified enzymes were improved by about 29% to about 341% compared to M7GLODΔ49C-T7 before modification.

[Example 4] Verification of thermal stability improving effect of deletion carried out on M7GLOD-T8

9. Construction of plasmid for M7GLOD expression

A plasmid (pET22b-M7GLOD) for expression of M7GLOD (SEQ ID NO: 58) was prepared by the same method as in "7. Construction of plasmid for expression of StGLOD homolog having thermal stability improving mutation." The synthesis of a DNA fragment (SEQ ID NO: 86) comprising M7GLOD gene (SEQ ID NO: 85) was commissioned to Integrated DNA Technologies, Inc. Fifteen 5' terminal bases and Fifteen 3' terminal bases of SEQ ID NO: 86 are overlapping sequences for assembly with pET-22b(+). A pET-22b(+) plasmid fragment amplified using SEQ ID NO: 65 and SEQ ID NO: 66 was ligated to the nucleotide sequence of SEQ ID NO: 86 through in-fusion reaction to obtain pET22b-M7GLOD. *E. coli* JM109 strain was transformed with the obtained plasmid. The obtained transformant was cultured, and the nucleotide sequence of an extracted plasmid was confirmed by DNA sequence analysis to be the intended sequence.

10. Construction of plasmid for M7GLOD-T8 expression

A plasmid (pET22b-M7GLOD-T8) for expression of M7GLOD-T8 (SEQ ID NO: 87) was prepared by introducing 8 types of amino acid substitutions (F87Y, Y103I, F133Y, F297M, F393L, F428Y, Y566F, Y585L) into M7GLOD by the same method as above. The amino acid substitutions of Y566F and Y585L were introduced as Y517F and Y536L in M7GLODΔ49C-T8 (SEQ ID NO: 60).

A fragment was amplified by PCR using pET22b-M7GLOD as the template and primers of SEQ ID NO: 88 (ccgtcgttggtgggaattc) and SEQ ID NO: 89 (gcgctcagcatcgtcaaaag). A fragment was amplified by PCR using pET22b-M7GLODΔ49C-T8 as the template and primers of SEQ ID NO: 90 (cttttgacgatgctgagcgc) and SEQ ID NO: 91 (gaattcccaccaacgacgg). Both the fragments were treated with DpnI, then purified and ligated through in-fusion reaction to obtain pET22b-M7GLOD-T8. *E. coli* JM109 strain was transformed with the obtained plasmid. The obtained transformant was cultured, and the nucleotide sequence of an extracted plasmid was confirmed by DNA sequence analysis to be the intended sequence.

11. Preparation of each deletion type M7GLOD-T8 and thermal stability evaluation

Each deletion type M7GLOD-T8 was prepared by performing PCR in the same manner as in "6. Preparation of modified StGLODΔ49C" using pET22b-M7GLOD-T8 as the template, thereby deleting all or part of a region encoding the 460th to 508th positions of SEQ ID NO: 87. PCR was performed by 35 cycles of "98°C for 10 sec, 68°C for 40 sec". The primers used are described in Table 14.

[Table 14]

| Mutant name | Fw primer | Rv primer |
|---|---|---|
| M7GLOD-DΔ32-T8 | gtacgcccggcaactcaagttcat ggagga (SEQ ID NO: 92) | aagattgcgtacactttcgggtac cgtaag (SEQ ID NO: 93) |
| M7GLOD-DΔ35-T8 | gtacgcccggcaactcaagttcat ggagga (SEQ ID NO: 92) | tacactttcgggtaccgtaagggc tgcttc (SEQ ID NO: 94) |
| M7GLOD-DΔ39-T8 | gtacgcccggcaactcaagttcat ggagga (SEQ ID NO: 92) | taccgtaagggctgcttcggcatc gtcctc (SEQ ID NO: 95) |
| M7GLOD-DΔ43-T8 | gtacgcccggcaactcaagttcat ggagga (SEQ ID NO: 92) | tgcttcggcatcgtcctcgcccca acgctg (SEQ ID NO: 96) |
| M7GLOD-DΔ47-T8 | gtacgcccggcaactcaagttcat ggagga (SEQ ID NO: 92) | gtcctcgccccaacgctggtagta ttcata (SEQ ID NO: 97) |
| M7GLOD-DΔ49-T8 | gtacgcccggcaactcaagttcat ggagga (SEQ ID NO: 92) | gccccaacgctggtagtattcata caggcc (SEQ ID NO: 98) |

The PCR product was treated with DpnI, and then, 2 μL thereof was mixed with 5 μE of Ligation High Ver. 2 (manufactured by Toyobo Co., Ltd.), 1 μL of T4 polynucleotide kinase (manufactured by Toyobo Co., Ltd.), and 7 μL of ion exchange water and ligation reaction was performed at 16°C for 60 minutes. *E. coli* JM109 strain was transformed with the obtained reaction product. The obtained transformant was cultured, and the nucleotide sequences of extracted plasmids were confirmed by DNA sequence analysis to be the intended sequence(s).

Next, *E. coli* BL21 (DE3) strain was transformed with the plasmid for M7GLOD-T8 or each deletion type M7GLOD-T8 expression to prepare a M7GLOD-T8 or each deletion type M7GLOD-T8 producing strain. Recombinant production was performed in accordance with "8. Recombinant production of M7GLODΔ49C-T7." The M7GLOD-T8 or each deletion type M7GLOD-T8 was heated at 65°C for 30 minutes and the residual activity thereof was calculated in accordance with the above method (Table 15).

[Table 15]

| Mutant name | Residual activity after treatment at 65°C for 30 min |
|---|---|
| M7GLOD-T8 | 0.13 |
| M7GLOD-DΔ32-T8 | 0.27 |
| M7GLOD-DΔ35-T8 | 0.27 |
| M7GLOD-DΔ39-T8 | 0.18 |
| M7GLOD-DΔ43-T8 | 0.56 |
| M7GLOD-DΔ47-T8 | 0.67 |
| M7GLOD-DΔ49-T8 | 0.31 |

The residual activities of each deletion type M7GLOD-T8 were increased by 0.05 to 0.54 compared to the M7GLOD-T8 and the thermal stabilities of all deletion type M7GLOD-T8 were improved. In other words, the residual activities of the prepared modified enzymes were improved by about 38% to about 415% compared to M7GLODΔ49C-T7 before modification.

[Example 5] Improvement of thermal stability of StGLOD by amino acid substitution 12. Construction of plasmid for expression of StGLOD

A plasmid (pET22b-StGLOD) for expression of StGLOD (SEQ ID NO: 1) was prepared by the same method as in "7. Construction of plasmid for expression of StGLOD homolog having thermal stability improving mutation." A fragment comprising a StGLOD gene was amplified by PCR using pKK223-3-StGLOD as the template and primers of SEQ ID NO: 99 (gaaggagatatacatatgaatgaaatgacctacgagcaattg) and SEQ ID NO: 100 (tcctttcgggctttgttaagaagttaacgcctcctc). A fragment was amplified by PCR using pET-22b(+) plasmid as the template and primers of SEQ ID NO: 101 (caaagcccgaaaggaagctgagttggctgc) and SEQ ID NO: 102 (tcctttcgggctttgttaagaagttaacgcctcctc). PCR was carried out in the same manner as in "2. Preparation of StGLOD deletion mutant".

Both the fragments were treated with DpnI, then purified and ligated through in-fusion reaction to obtain pET22b-StGLOD. *E. coli* JM109 strain was transformed with the obtained plasmid. The obtained transformant was cultured, and the nucleotide sequence of an extracted plasmid was confirmed by DNA sequence analysis to be the intended sequence.

13. Preparation of StGLOD mutant

A plasmid for expression of each modified StGLOD was constructed with the same method as in "6. Preparation of modified StGLODΔ49C" by site directed mutagenesis with a plasmid (pET22b-StGLOD) for StGLOD expression as the template. Table 16 shows the names of prepared mutants and combinations of Fw and Rv primers.

[Table 16]

| Mutant name | Fw primer | Rv primer |
|---|---|---|
| StGLOD/F87Y | (SEQ ID NO: 15) | (SEQ ID NO: 14) |
| StGLOD/Y1031 | (SEQ ID NO: 20) | (SEQ ID NO: 16) |
| StGLOD/F133Y | (SEQ ID NO: 23) | (SEQ ID NO: 22) |
| StGLOD/F297L | (SEQ ID NO: 26) | (SEQ ID NO: 25) |
| StGLOD/F2971 | (SEQ ID NO: 28) | (SEQ ID NO: 25) |
| StGLOD/F297M | (SEQ ID NO: 29) | (SEQ ID NO: 25) |
| StGLOD/F393L | (SEQ ID NO: 36) | (SEQ ID NO: 35) |
| StGLOD/F428Y | (SEQ ID NO: 41) | (SEQ ID NO: 40) |
| StGLOD/Y566L | (SEQ ID NO: 47) | (SEQ ID NO: 45) |
| StGLOD/Y566V | (SEQ ID NO: 48) | (SEQ ID NO: 45) |
| StGLOD/Y566M | (SEQ ID NO: 49) | (SEQ ID NO: 45) |
| StGLOD/Y5681 | (SEQ ID NO: 51) | (SEQ ID NO: 50) |

(continued)

| Mutant name | Fw primer | Rv primer |
|---|---|---|
| StGLOD/Y585L | (SEQ ID NO: 54) | (SEQ ID NO: 53) |
| StGLOD/F615L | (SEQ ID NO: 57) | (SEQ ID NO: 56) |
| StGLOD/R186G | atccgtacgaatggcgaacaagta cgc (SEQ ID NO: 104) | attcgtacggatccaggtgtgatt acgttt (SEQ ID NO: 103) |
| STGLOD/R186A | atccgtacgaatgcggaacaagta cgccgc (SEQ ID NO: 105) | attcgtacggatccaggtgtgatt acgttt (SEQ ID NO: 103) |
| STGLOD/R186C | atccgtacgaattgcgaacaagta cgccgc (SEQ ID NO: 106) | attcgtacggatccaggtgtgatt acgttt (SEQ ID NO: 103) |
| STGLOD/R186L | atccgtacgaatctggaacaagta cgccgc (SEQ ID NO: 107) | attcgtacggatccaggtgtgatt acgttt (SEQ ID NO: 103) |
| STGLOD/R186M | atccgtacgaatatggaacaagta cgccgc (SEQ ID NO: 108) | attcgtacggatccaggtgtgatt acgttt (SEQ ID NO: 103) |
| STGLOD/R186F | atccgtacgaattttgaacaagta cgccgc (SEQ ID NO: 109) | attcgtacggatccaggtgtgatt acgttt (SEQ ID NO: 103) |
| STGLOD/R186Y | atccgtacgaattatgaacaagta cgccgc (SEQ ID NO: 110) | attcgtacggatccaggtgtgatt acgttt (SEQ ID NO: 103) |
| STGLOD/R186S | atccgtacgaatagcgaacaagta cgccgc (SEQ ID NO: 111) | attcgtacggatccaggtgtgatt acgttt (SEQ ID NO: 103) |
| STGLOD/R186T | atccgtacgaataccgaacaagta cgccgc (SEQ ID NO: 112) | attcgtacggatccaggtgtgatt acgttt (SEQ ID NO: 103) |
| STGLOD/R186N | atccgtacgaataacgaacaagta cgccgc (SEQ ID NO: 113) | attcgtacggatccaggtgtgatt acgttt (SEQ ID NO: 103) |
| STGLOD/R186Q | atccgtacgaatcaggaacaagta cgccgc (SEQ ID NO: 114) | attcgtacggatccaggtgtgatt acgttt (SEQ ID NO: 103) |
| STGLOD/R186H | atccgtacgaatcatgaacaagta cgccgc (SEQ ID NO: 115) | attcgtacggatccaggtgtgatt acgttt (SEQ ID NO: 103) |
| STGLOD/R186D | atccgtacgaatgatgaacaagta cgccgc (SEQ ID NO: 116) | attcgtacggatccaggtgtgatt acgttt (SEQ ID NO: 103) |
| STGLOD/R186E | atccgtacgaatgaagaacaagta cgccgc (SEQ ID NO: 117) | attcgtacggatccaggtgtgatt acgttt (SEQ ID NO: 103) |

Next, *E. coli* BL21 (DE3) strain was transformed with the plasmid for pET22b-StGLOD or each modified StGLOD expression to prepare a StGLOD or each modified StGLOD producing strain. Recombinant production was performed in accordance with "8. Recombinant production of M7GLODΔ49C-T7." StGLOD or each modified StGLOD was heated at 50°C for 30 minutes and the residual activity thereof was calculated in accordance with the above method (Table 17).

[Table 17]

| Mutant name | Residual activity after treatment at 50°C for 30 min |
|---|---|
| StGLOD | 0.18 |
| StGLOD/F87Y | 0.24 |
| StGLOD/Y103I | 0.19 |
| StGLOD/F133Y | 0.26 |
| StGLOD/F297L | 0.29 |
| StGLOD/F297I | 0.50 |
| StGLOD/F297M | 0.33 |
| StGLOD/F393L | 0.37 |
| StGLOD/F428Y | 0.52 |
| StGLOD/F566L | 0.45 |
| StGLOD/F566V | 0.94 |
| StGLOD/F566M | 0.72 |
| StGLOD/Y568I | 0.81 |
| StGLOD/Y585L | 0.72 |
| StGLOD/F615L | 0.68 |
| StGLOD/R186G | 0.41 |
| StGLOD/R186A | 0.21 |
| StGLOD/R186C | 0.38 |
| StGLOD/R186L | 0.36 |
| StGLOD/R186M | 0.38 |
| StGLOD/R186F | 0.36 |
| StGLOD/R186Y | 0.43 |
| StGLOD/R186S | 0.44 |
| StGLOD/R186T | 0.21 |
| StGLOD/R186N | 0.41 |
| StGLOD/R186Q | 0.29 |
| StGLOD/R186H | 0.47 |
| StGLOD/R186D | 0.62 |
| StGLOD/R186E | 0.70 |

The residual activities of each modified StGLOD were increased by 0.01 to 0.76 compared to StGLOD and the thermal stabilities of all modified StGLODs were improved. In other words, the residual activities of the prepared modified enzymes were improved by about 6% to about 422% compared to StGLOD before modification. It was demonstrated that the amino acid substitutions improving thermal stability, found by studying StGLODΔ49C or M7GLODΔ49C-T7, also contributed to the improved thermal stability of StGLOD without being limited by their parent enzymes. Thus, it is believed that the thermal stability of a GLOD of other origin or a GLOD having a high sequence identity thereto is also improved by introducing these amino acid substitutions.

Industrial Applicability

The glutamate oxidase mutant of the present disclosure requires no protease treatment and can be produced at a large scale. The glutamate oxidase mutant of the present disclosure may also be used in the oxidation reaction of glutamic acid.

Various literatures including patent applications and manufacturers' manuals are cited in the present specification. The disclosure of these literatures should not be interpreted as being related to the patentability of the present disclosure and however, is incorporated herein by reference in their entirety. More specifically, all references are incorporated herein by reference, as in the case of specifically and individually stating that each literature is incorporated by reference.

[Sequence Listing]

SEQ ID NO: 1 Amino acid sequence of the glutamate oxidase from *Streptomyces* sp. X-119-6 (StGLOD)

MNEMTYEQLARELLLVGPAPTNEDLKLRYLDVLIDNGLNPPGPPKRILIVGAGIAGLVAG

DLLTRAGHDVTILEANANRVGGRIKTFHAKKGEPSPFADPAQYAEAGAMRLPSFHPLTLA

LIDKLGLKRRLFFNVDIDPQTGNQDAPVPPVFYKSFKDGKTWTNGAPSPEFKEPDKRNHT

WIRTNREQVRRAQYATDPSSINEGFHLTGCETRLTVSDMVNQALEPVRDYYSVKQDDGTR

VNKPFKEWLAGWADVVRDFDGYSMGRFLREYAEFSDEAVEAIGTIENMTSRLHLAFFHSF

LGRSDIDPRATYWEIEGGSRMLPETLAKDLRDQIVMGQRMVRLEYYDPGRDGHHGELTGP

GGPAVAIQTVPEGEPYAATQTWTGDLAIVTIPFSSLRFVKVTPPFSYKKRRAVIETHYDQ

ATKVLLEFSRRWWEFTEADWKRELDAIAPGLYDYYQQWGEDDAEAALALPQSVRNLPTGL

LGAHPSVDESRIGEEQVEYYRNSELRGGVRPATNAYGGGSTTDNPNRFMYYPSHPVPGTQ

GGVVLAAYSWSDDAARWDSFDDAERYGYALENLQSVHGRRIEVFYTGAGQTQSWLRDPYA

CGEAAVYTPHQMTAFHLDVVRPEGPVYFAGEHVSLKHAWIEGAVETAVRAAIAVNEAPVG

DTGVTAAAGRRGAAAATEPMREEALTS

SEQ ID NO: 2 Nucleotide sequence of the glutamate oxidase from *Streptomyces* sp. X-119-6 (StGLOD)

atgaatgaaatgacctacgagcaattggcccgcgaattgttattggttggtccggccccgacgaacgaggatcttaaattacgctatttgg

atgtgctgattgataacgggctgaacccgccgggtccgcctaagcgtatcttgatcgtcggggctggtattgcaggccttgttgccggag

atttgttgacccgcgctgggcatgatgttactattcttgaagctaatgccaaccgtgtgggagggcgtatcaagacgttccatgcaaaaaa

aggagagccatctccattcgctgatccagcgcagtacgcggaagctggggcgatgcgtcttccttcctttcacccacttaccttggcactt

atcgacaagctgggcttaaaacgccgcctgttttttaatgtagatattgacccacaaactggaaatcaagatgctccagtcccaccggtctt

ctataagtcttttaaggacggcaagacgtggacaaatggagccccgagccctgagtttaaagagcccgataaacgtaatcacacctgga

tccgtacgaatcgcgaacaagtacgccgcgcccaatacgccacagacccgagctccatcaatgaaggcttccatctgacgggatgtga

gacccgccttactgtctccgacatggttaaccaagcgcttgaaccggtacgcgactattattcagtcaagcaggacgatggaactcgtgt

gaataagcctttcaaagaatggcttgcagggtgggccgacgtcgtgcgtgatttcgacggttattctatgggccgcttcttacgtgaatatg

ccgagttttcggatgaggcagtggaagcaattggcacaattgaaaacatgacctcgcgcctgcaccttgcctttttttcattcgttttttgggcc

gttccgatatcgatccgcgcgccacttactgggagatcgagggggggtcacgcatgcttcctgaaacactggcaaaggacctgcgcga

tcagatcgttatgggtcagcgcatggtacgcttagagtactatgacccgggccgcgatggccatcatggtgagcttactgggcctgggg

gtccggccgtggccattcagacggttccagagggagagccttacgccgcaactcaaacgtggacaggagacctggcgattgttactat

ccccttttagctctttacgctttgttaaagtaacgccaccccttctcttataaaaagcgccgcgcggtaattgagacccattatgatcaggccac

caaagtcttacttgaatttagccgtcgttggtgggaattcacagaggcggactggaagcgtgagcttgatgctatcgccccgggtctgtac

gactattatcaacagtggggtgaagacgacgccgaagcagcgcttgcactgccgcagtcagtccgcaacttgcctaccggcttgcttgg

ggcccacccaagcgtcgacgaatcgcgtatcggagaggagcaagttgagtattatcgtaacagcgaactgcgtggcggagtgcgccc

tgcgaccaatgcgtacggagggggtagtacgaccgataatcctaatcgtttcatgtactatccctcccaccccgttccaggcacgcagg

ggggggtcgtacttgcagcatacagttggagcgatgatgcagctcgttgggattctttttgacgatgctgagcgctatggctatgcgcttga

aaacttacagtctgtccacggccgtcgtattgaggtgttctatacaggagcgggacagacccaatcatggctgcgcgatccgtacgcatg

cggcgaggcagctgtctatacaccccatcaaatgaccgcgtttcacttagatgtggtgcgtcccgagggcccgtctattttgcgggtga

gcacgtttcattaaaacatgcctggatcgagggggcggtggaaacagccgttcgtgcggcgatcgctgtaaatgaagcccccgtaggt

gacactggggtcacagccgctgctgggcgccgtggggcggccgccgctactgagcctatgcgcgaggaggcgttaacttcttaa

SEQ ID NO: 3 StGLOD-f1

atgaatgaaatgacctacgagcaattggcccgcgaattgttattggttggtccggcccccgacgaacgaggatcttaaattacgctatttgg

atgtgctgattgataacgggctgaacccgccgggtccgcctaagcgtatcttgatcgtcggggctggtattgcaggccttgttgccggag

atttgttgacccgcgctgggcatgatgttactattcttgaagctaatgccaaccgtgtgggagggcgtatcaagacgttccatgcaaaaaa

aggagagccatctccattcgctgatccagcgcagtacgcggaagctggggcgatgcgtcttccttcctttcacccacttaccttggcactt

atcgacaagctgggcttaaaacgccgcctgtttttttaatgtagatattgacccacaaactggaaatcaagatgctccagtcccaccggtctt

ctataagtcttttaaggacggcaagacgtggacaaatggagccccgagccctgagtttaaagagcccgataaacgtaatcacacctgga

tccgtacgaatcgcgaacaagtacgccgcgcccaatacgccacagacccgagctccatcaatgaaggcttccatctgacgggatgtga

gacccgccttactgtctccgacatggttaaccaagcgcttgaaccggtacgcgactattattcagtcaagcaggac

SEQ ID NO: 4 StGLOD-f2

tcagtcaagcaggacgatggaactcgtgtgaataagcctttcaaagaatggcttgcagggtgggccgacgtcgtgcgtgatttcgacgg

ttattctatgggccgcttcttacgtgaatatgccgagttttcggatgaggcagtggaagcaattggcacaattgaaaacatgacctcgcgcc

tgcaccttgcctttttttcattcgttttggggccgttccgatatcgatccgcgcgccacttactgggagatcgagggggggtcacgcatgcttc

ctgaaacactggcaaaggacctgcgcgatcagatcgttatgggtcagcgcatggtacgcttagagtactatgacccgggccgcgatgg

ccatcatggtgagcttactgggcctggggggtccggccgtggccattcagacggttccagagggagagccttacgccgcaactcaaacg

tggacaggagacctggcgattgttactatcccctttagctctttacgctttgttaaagtaacgccacccttctcttataaaaagcgccgcgcg

gtaattgagacccattatgatcaggccaccaaagtcttacttgaatttagccgtcgttggtgggaattcacagaggcggactggaagcgtg

agcttgatgctatcgccccgggtctgtacgactattatcaacagtggggtgaagac

SEQ ID NO: 5 StGLOD-f3

cagtgggggtgaagacgacgccgaagcagcgcttgcactgccgcagtcagtccgcaacttgcctaccggcttgcttgggggcccaccca

agcgtcgacgaatcgcgtatcggagaggagcaagttgagtattatcgtaacagcgaactgcgtggcggagtgcgccctgcgaccaat

gcgtacggagggggtagtacgaccgataatcctaatcgtttcatgtactatccctcccaccccgttccaggcacgcaggggggggtcgt

acttgcagcatacagttggagcgatgatgcagctcgttgggattcttttgacgatgctgagcgctatggctatgcgcttgaaaacttacagt

ctgtccacggccgtcgtattgaggtgttctatacaggagcgggacagacccaatcatggctgcgcgatccgtacgcatgcggcgaggc

agctgtctatacaccccatcaaatgaccgcgtttcacttagatgtggtgcgtcccgagggcccgtctattttgcgggtgagcacgtttcat

taaaacatgcctggatcgagggggcggtggaaacagccgttcgtgcggcgatcgctgtaaatgaagcccccgtaggtgacactggg

tcacagccgctgctgggcgccgtggggcggccgccgctactgagcctatgcgcgaggaggcgttaacttcttaa

SEQ ID NOs: 6 to 11 Primer sequences
SEQ ID NO: 12 Amino acid sequence of StGLODΔ49Ai

MNEMTYEQLARELLLVGPAPTNEDLKLRYLDVLIDNGLNPPGPPKRILIVGAGIAGLV

AGDLLTRAGHDVTILEANANRVGGRIKTFHAKKGEPSPFADPAQYAEAGAMRLPSFH

PLTLALIDKLGLKRRLFFNVDIDPQTGNQDAPVPPVFYKSFKDGKTWTNGAPSPEFKE

PDKRNHTWIRTNREQVRRAQYATDPSSINEGFHLTGCETRLTVSDMVNQALEPVRDY

YSVKQDDGTRVNKPFKEWLAGWADVVRDFDGYSMGRFLREYAEFSDEAVEAIGTIE

NMTSRLHLAFFHSFLGRSDIDPRATYWEIEGGSRMLPETLAKDLRDQIVMGQRMVRL

EYYDPGRDGHHGELTGPGGPAVAIQTVPEGEPYAATQTWTGDLAIVTIPFSSLRFVKV

TPPFSYKKRRAVIETHYDQATKVLLEFSRRWWEFTEADWKRELDAIAPGLYDYYRKL

DKTEATNAYGGGSTTDNPNRFMYYPSHPVPGTQGGVVLAAYSWSDDAARWDSFDD

AERYGYALENLQSVHGRRIEVFYTGAGQTQSWLRDPYACGEAAVYTPHQMTAFHLD

VVRPEGPVYFAGEHVSLKHAWIEGAVETAVRAAIAVNEAPVGDTGVTAAAGRRGAA

AATEPMREEALTS

SEQ ID NO: 13 Amino acid sequence of StGLODΔ49C

MNEMTYEQLARELLLVGPAPTNEDLKLRYLDVLIDNGLNPPGPPKRILIVGAGIAGLV

AGDLLTRAGHDVTILEANANRVGGRIKTFHAKKGEPSPFADPAQYAEAGAMRLPSFH

PLTLALIDKLGLKRRLFFNVDIDPQTGNQDAPVPPVFYKSFKDGKTWTNGAPSPEFKE

PDKRNHTWIRTNREQVRRAQYATDPSSINEGFHLTGCETRLTVSDMVNQALEPVRDY

YSVKQDDGTRVNKPFKEWLAGWADVVRDFDGYSMGRFLREYAEFSDEAVEAIGTIE

NMTSRLHLAFFHSFLGRSDIDPRATYWEIEGGSRMLPETLAKDLRDQIVMGQRMVRL

EYYDPGRDGHHGELTGPGGPAVAIQTVPEGEPYAATQTWTGDLAIVTIPFSSLRFVKV

TPPFSYKKRRAVIETHYDQATKVLLEFSRRWWEFTEADWKRELDAIAPGLYDYYQD

AAEPPATNAYGGGSTTDNPNRFMYYPSHPVPGTQGGVVLAAYSWSDDAARWDSFD

DAERYGYALENLQSVHGRRIEVFYTGAGQTQSWLRDPYACGEAAVYTPHQMTAFHL

DVVRPEGPVYFAGEHVSLKHAWIEGAVETAVRAAIAVNEAPVGDTGVTAAAGRRGA

AAATEPMREEALTS

SEQ ID NOs: 14 to 57 Primer sequences
SEQ ID NO: 58 Amino acid sequence of the putative glutamate oxidase from *Streptomyces* sp. MOE7 (M7GLOD)

MDDKTYQQLARELLLVGPEPANEDLKLRYLDVLIDNGLEPPVDRKRILIVGAGIAGLV
AGHLLTRAGHDVTILEANANRVGGRIKTFHAKKGEPAPFTDPAQYAEAGAMRLPSFH
PLTLALIDKLGLKRRLFFNVDIDPKTGNQGAALPPVVYKSFKDGKTWTYGKPSPEFRE
PDKRNHTWIRTNRTQVRRAQYVKDPSAINEGFHLTGCESRLTVSDMVNQALEPVRDY
YSVLQSDGRRVNKPFKEWLDGWAGVIRDFDGFSMGRFLREYAGFSDEAVEAIGTIEN
MTSRLHLAFFHSFLGRSDIDPSATYWEIEGGSRQLPEALAKDLRDQIVMGQRMVRLE
YYDPGRDGHHGGLAGPSGPAVAIETVPENEPSAEPQTWTADLAIVTVPFSSLRFVAVT
PPFSYKKRRAVIETHYDQATKVLLEFSRRWWEFTEEDWKRELDAIAPGLYEYYQRW
GEDDAEAALTVPESVRNLPTGLLGAHPSVDEQLIDDEQVEYLRNSTLRGGVRPATQV
HGGGSTTDNPNRFMYYPSHAVPGSKGGVVLAAYSWSDDAARWDSFDDAERYGYAL
ENLQSVHGRRIEVFYTGAGQTQSWLRDPYACGEAAVYTPHQMTSFHLDVVRPEGPV
YFAGEHVSLKHAWIEGAVETAVRAAIAVNEAPVPYDTAAARAEAPRERAGTASATR
TREKAVTS

SEQ ID NO: 59 Amino acid sequence of deletion type M7GLOD (M7GLODΔ49C)

MDDKTYQQLARELLLVGPEPANEDLKLRYLDVLIDNGLEPPVDRKRILIVGAGIAGLV
AGHLLTRAGHDVTILEANANRVGGRIKTFHAKKGEPAPFTDPAQYAEAGAMRLPSFH
PLTLALIDKLGLKRRLFFNVDIDPKTGNQGAALPPVVYKSFKDGKTWTYGKPSPEFRE
PDKRNHTWIRTNRTQVRRAQYVKDPSAINEGFHLTGCESRLTVSDMVNQALEPVRDY
YSVLQSDGRRVNKPFKEWLDGWAGVIRDFDGFSMGRFLREYAGFSDEAVEAIGTIEN
MTSRLHLAFFHSFLGRSDIDPSATYWEIEGGSRQLPEALAKDLRDQIVMGQRMVRLE
YYDPGRDGHHGGLAGPSGPAVAIETVPENEPSAEPQTWTADLAIVTVPFSSLRFVAVT
PPFSYKKRRAVIETHYDQATKVLLEFSRRWWEFTEEDWKRELDAIAPGLYEYYQDAA
EPPATQVHGGGSTTDNPNRFMYYPSHAVPGSKGGVVLAAYSWSDDAARWDSFDDA
ERYGYALENLQSVHGRRIEVFYTGAGQTQSWLRDPYACGEAAVYTPHQMTSFHLDV
VRPEGPVYFAGEHVSLKHAWIEGAVETAVRAAIAVNEAPVPYDTAAARAEAPRERA
GTASATRTREKAVTS

SEQ ID NO: 60 Amino acid sequence of M7GLODΔ49C having 8 types of mutations for thermal stability introduced therein (M7GLODΔ49C-T8)

MDDKTYQQLARELLLVGPEPANEDLKLRYLDVLIDNGLEPPVDRKRILIVGAGIAGLV
AGHLLTRAGHDVTILEANANRVGGRIKTYHAKKGEPAPFTDPAQIAEAGAMRLPSFH
PLTLALIDKLGLKRRLFYNVDIDPKTGNQGAALPPVVYKSFKDGKTWTYGKPSPEFRE
PDKRNHTWIRTNRTQVRRAQYVKDPSAINEGFHLTGCESRLTVSDMVNQALEPVRDY
YSVLQSDGRRVNKPFKEWLDGWAGVIRDFDGFSMGRFLREYAGFSDEAVEAIGTIEN
MTSRLHLAFMHSFLGRSDIDPSATYWEIEGGSRQLPEALAKDLRDQIVMGQRMVRLE
YYDPGRDGHHGGLAGPSGPAVAIETVPENEPSAEPQTWTADLAIVTVPLSSLRFVAVT
PPFSYKKRRAVIETHYDQATKVLLEYSRRWWEFTEEDWKRELDAIAPGLYEYYQDA
AEPPATQVHGGGSTTDNPNRFMYYPSHAVPGSKGGVVLAAYSWSDDAARWDSFDD
AERFGYALENLQSVHGRRIEVFLTGAGQTQSWLRDPYACGEAAVYTPHQMTSFHLD
VVRPEGPVYFAGEHVSLKHAWIEGAVETAVRAAIAVNEAPVPYDTAAARAEAPRER
AGTASATRTREKAVTS

SEQ ID NO: 61 Nucleotide sequence of M7GLODΔ49C-T8

atggacgacaagacctatcagcagttagcacgcgaactgctgcttgtagggcccgagccagcgaatgaagatcttaaattgcgctattta

gacgtacttattgataacgggctggagcccccagtcgatcgtaaacgtatcttaattgtgggggcagggatcgccggcctggtcgcggg

acatttgttgacacgcgcgggacacgatgtcaccatcctggaggcgaatgcaaatcgcgttggaggtcgcattaagacatatcatgcaa

agaaaggcgaaccggccccccttcaccgacccggctcaaattgcagaggcgggagctatgcgtttgccttcattccatcccttaacgttag

cgctgattgataaattagggttgaagcgtcgcttgttctataatgttgacattgaccctaagacgggcaatcaaggtgcagcattgccgcct

gtcgtttacaagagctttaaggacggtaaaacttggacgtatggtaaaccttctccggaattccgtgaaccagacaaacgtaaccacactt

ggattcgtaccaatcgcactcaagtacgtcgcgcccagtatgtgaaggacccgagtgcgattaacgaagggttccatttaaccgggtgc

gagtcgcgtttgacagtttctgatatggtgaaccaagctttagaaccggttcgtgactactattctgtattgcagagcgacgggcgccgcgt

aaataagccatttaaggagtggttagacggctgggccggtgtaatccgcgatttcgacggattctcaatgggacgttttctgcgcgagtac

gctgggttttcggacgaggcggtagaagcaatcggtaccatcgagaatatgacaagtcgtttgcatttggcatttatgcacagcttcttagg

tcgcagtgacattgaccccagcgcgacatattgggagattgaaggcggtagccgtcagctgcctgaggctctggcaaaagacctgcgt

gatcagattgtaatgggccaacgcatggtgcgtttagagtactacgacccaggacgtgatggacaccatggaggtctggcaggaccct

ctggtcctgcggttgcaatcgaaactgtacccgagaacgagccaagtgcagagccgcagacgtggactgcggacctggcgattgtca

ctgtaccactttcgtctcttcgctttgttgcggtgactcccccattcagttataaaaaacgtcgtgcagttatcgaaactcactatgatcaggc

aacaaaagtgctgttagagtattctcgtcgctggtgggagtttacagaagaggactggaagcgtgagttggacgcgattgcccccggcc

tgtatgaatactaccaggatgctgcagaaccgccggcaactcaagttcatggaggaggttcaacaactgacaatccaaaccgtttatgta

ttatccgagtcacgcggtgcctggttctaagggtggtgtggttttggccgcgtatagttggagtgacgatgcagcgcgttgggattcctttg

atgacgccgaacgttttggctacgccctggaaaaccttcaatcggtccatggccgccgcatcgaggtctttttaactggagctggacaaa

ctcagtcatggttgcgcgacccctacgcctgcggtgaggccgcagtttacacaccacatcagatgacatcttttcacttagacgtagtgcg

cccggagggggccagtatactttgcgggagagcatgtctctcttaagcatgcgtggatcgaggggggccgttgaaaccgcggtccgtgct

gcgatcgcggttaacgaagcccccgtaccatacgacacagctgctgctcgtgcggaggcccctcgcgaacgtgccggtacagcatca

gccactcgcacgcgcgagaaggccgtgacttcctaa

SEQ ID NO: 62 M7GLODΔ49C-T8-f1

ggagatatacatatggacgacaagacctatcagcagttagcacgcgaactgctgcttgtagggcccgagccagcgaatgaagatcttaa

attgcgctatttagacgtacttattgataacgggctggagcccccagtcgatcgtaaacgtatcttaattgtggggggcagggatcgccggc

ctggtcgcgggacatttgttgacacgcgcgggacacgatgtcaccatcctggaggcgaatgcaaatcgcgttggaggtcgcattaaga

catatcatgcaaagaaaggcgaaccggcccccttcaccgacccggctcaaattgcagaggcgggagctatgcgtttgccttcattccat

cccttaacgttagcgctgattgataaattagggttgaagcgtcgcttgttctataatgttgacattgaccctaagacgggcaatcaaggtgc

agcattgccgcctgtcgtttacaagagctttaaggacggtaaaacttggacgtatggtaaaccttctccggaattccgtgaaccagacaaa

cgtaaccacacttggattcgtaccaatcgcactcaagtacgtcgcgcccagtatgtgaaggacccgagtgcgattaacgaagggttccat

ttaaccgggtgcgagtcgcgtttga

SEQ ID NO: 63 M7GLODΔ49C-T8-f2

gcgagtcgcgtttgacagtttctgatatggtgaaccaagctttagaaccggttcgtgactactattctgtattgcagagcgacgggcgccg

cgtaaataagccatttaaggagtggttagacggctgggccggtgtaatccgcgatttcgacggattctcaatgggacgttttctgcgcgag

tacgctgggttttcggacgaggcggtagaagcaatcggtaccatcgagaatatgacaagtcgtttgcatttggcatttatgcacagcttctt

aggtcgcagtgacattgaccccagcgcgacatattgggagattgaaggcggtagccgtcagctgcctgaggctctggcaaaagacctg

cgtgatcagattgtaatgggccaacgcatggtgcgtttagagtactacgacccaggacgtgatggacaccatggaggtctggcaggac

cctctggtcctgcggttgcaatcgaaactgtacccgagaacgagccaagtgcagagccgcagacgtggactgcggacctggcgattgt

cactgtaccactttcgtctcttcgctttgttgcggtgactcccccattcagttataaaaaacgtcgtgcagttatcgaaactcactatgatcag

gcaacaaaagtgctgttagagtattctcgtcgctggtg

SEQ ID NO: 64 M7GLODΔ49C-T8-f3

ttctcgtcgctggtgggagtttacagaagaggactggaagcgtgagttggacgcgattgcccccggcctgtatgaatactaccaggatg

ctgcagaaccgccggcaactcaagttcatggaggaggttcaacaactgacaatccaaaccgtttatgtattatccgagtcacgcggtgc

ctggttctaagggtggtgtggttttggccgcgtatagttggagtgacgatgcagcgcgttgggattcctttgatgacgccgaacgttttggc

tacgccctggaaaaccttcaatcggtccatggccgccgcatcgaggtcttttttaactggagctggacaaactcagtcatggttgcgcgac

ccctacgcctgcggtgaggccgcagtttacacaccacatcagatgacatcttttcacttagacgtagtgcgcccggaggggccagtata

ctttgcgggagagcatgtctctcttaagcatgcgtggatcgaggggggccgttgaaaccgcggtccgtgctgcgatcgcggttaacgaag

cccccgtaccatacgacacagctgctgctcgtgcggaggcccctcgcgaacgtgccggtacagcatcagccactcgcacgcgcgag

aaggccgtgacttcctaacaaagcccgaaa

SEQ ID NOs: 65 to 68 Primer sequences
SEQ ID NO: 69 M7GLODΔ49C having 7 types of mutations for thermal stability introduced therein (M7GLODΔ49C-T7)

MDDKTYQQLARELLLVGPEPANEDLKLRYLDVLIDNGLEPPVDRKRILIVGAGIAGLV

AGHLLTRAGHDVTILEANANRVGGRIKTYHAKKGEPAPFTDPAQIAEAGAMRLPSFH

PLTLALIDKLGLKRRLFYNVDIDPKTGNQGAALPPVVYKSFKDGKTWTYGKPSPEFRE

PDKRNHTWIRTNRTQVRRAQYVKDPSAINEGFHLTGCESRLTVSDMVNQALEPVRDY

YSVLQSDGRRVNKPFKEWLDGWAGVIRDFDGFSMGRFLREYAGFSDEAVEAIGTIEN

MTSRLHLAFMHSFLGRSDIDPSATYWEIEGGSRQLPEALAKDLRDQIVMGQRMVRLE

YYDPGRDGHHGGLAGPSGPAVAIETVPENEPSAEPQTWTADLAIVTVPLSSLRFVAVT

PPFSYKKRRAVIETHYDQATKVLLEYSRRWWEFTEEDWKRELDAIAPGLYEYYQDA

AEPPATQVHGGGSTTDNPNRFMYYPSHAVPGSKGGVVLAAYSWSDDAARWDSFDD

AERFGYALENLQSVHGRRIEVFYTGAGQTQSWLRDPYACGEAAVYTPHQMTSFHLD

VVRPEGPVYFAGEHVSLKHAWIEGAVETAVRAAIAVNEAPVPYDTAAARAEAPRER

AGTASATRTREKAVTS

SEQ ID NOs: 70 to 84 Primer sequences
SEQ ID NO: 85 Nucleotide sequence of M7GLOD

ATGGACGACAAGACCTATCAGCAGTTAGCACGCGAACTGCTGCTTGTAGGGCCCG

AGCCAGCGAATGAAGATCTTAAATTGCGCTATTTAGACGTACTTATTGATAACGG

GCTGGAGCCCCAGTCGATCGTAAACGTATCTTAATTGTGGGGGCAGGGATCGCC

GGCCTGGTCGCGGGACATTTGTTGACACGCGCGGGACACGATGTCACCATCCTGG

AGGCGAATGCAAATCGCGTTGGAGGTCGCATTAAGACATTTCATGCAAAGAAAG

GCGAACCGGCCCCCTTCACCGACCCGGCTCAATATGCAGAGGCGGGAGCTATGCG

TTTGCCTTCATTCCATCCCTTAACGTTAGCGCTGATTGATAAATTAGGGTTGAAGC

GTCGCTTGTTCTTTAATGTTGACATTGACCCTAAGACGGGCAATCAAGGTGCAGC

ATTGCCGCCTGTCGTTTACAAGAGCTTTAAGGACGGTAAAACTTGGACGTATGGT

AAACCTTCTCCGGAATTCCGTGAACCAGACAAACGTAACCACACTTGGATTCGTA

CCAATCGCACTCAAGTACGTCGCGCCCAGTATGTGAAGGACCCGAGTGCGATTAA

CGAAGGGTTCCATTTAACCGGGTGCGAGTCGCGTTTGACAGTTTCTGATATGGTG

AACCAAGCTTTAGAACCGGTTCGTGACTACTATTCTGTATTGCAGAGCGACGGGC

GCCGCGTAAATAAGCCATTTAAGGAGTGGTTAGACGGCTGGGCCGGTGTAATCCG

CGATTTCGACGGATTCTCAATGGGACGTTTTCTGCGCGAGTACGCTGGGTTTTCGG

ACGAGGCGGTAGAAGCAATCGGTACCATCGAGAATGACAAGTCGTTTGCATTT

GGCATTTTTTCACAGCTTCTTAGGTCGCAGTGACATTGACCCCAGCGCGACATATT

GGGAGATTGAAGGCGGTAGCCGTCAGCTGCCTGAGGCTCTGGCAAAAGACCTGC

GTGATCAGATTGTAATGGGCCAACGCATGGTGCGTTTAGAGTACTACGACCCAGG
ACGTGATGGACACCATGGAGGTCTGGCAGGACCCTCTGGTCCTGCGGTTGCAATC
GAAACTGTACCCGAGAACGAGCCAAGTGCAGAGCCGCAGACGTGGACTGCGGAC
CTGGCGATTGTCACTGTACCATTTTCGTCTCTTCGCTTTGTTGCGGTGACTCCCCCA
TTCAGTTATAAAAAACGTCGTGCAGTTATCGAAACTCACTATGATCAGGCAACAA
AAGTGCTGTTAGAGTTTTCTCGTCGCTGGTGGGAGTTTACAGAAGAGGACTGGAA
GCGTGAGTTGGACGCGATTGCCCCCGGCCTGTATGAATACTACCAGCGTTGGGGC
GAGGACGATGCCGAAGCAGCCCTTACGGTACCCGAAAGTGTACGCAATCTTCCAA
CGGGATTGTTAGGGGCCCATCCTTCCGTTGACGAGCAACTGATTGATGACGAGCA
GGTAGAGTATCTGCGTAATTCAACATTGCGTGGAGGCGTACGCCCGGCAACTCAA
GTTCATGGAGGAGGTTCAACAACTGACAATCCAAACCGTTTTATGTATTATCCGA
GTCACGCGGTGCCTGGTTCTAAGGGTGGTGTGGTTTTGGCCGCGTATAGTTGGAGT
GACGATGCAGCGCGTTGGGATTCCTTTGATGACGCCGAACGTTATGGCTACGCCC
TGGAAAACCTTCAATCGGTCCATGGCCGCCGCATCGAGGTCTTTTATACTGGAGCT
GGACAAACTCAGTCATGGTTGCGCGACCCCTACGCCTGCGGTGAGGCCGCAGTTT
ACACCACATCAGATGACATCTTTTCACTTAGACGTAGTGCGCCCGGAGGGGCC
AGTATACTTTGCGGGAGAGCATGTCTCTCTTAAGCATGCGTGGATCGAGGGGGCC
GTTGAAACCGCGGTCCGTGCTGCGATCGCGGTTAACGAAGCCCCCGTACCATACG
ACACAGCTGCTGCTCGTGCGGAGGCCCCTCGCGAACGTGCCGGTACAGCATCAGC
CACTCGCACGCGCGAGAAGGCCGTGACTTCCTAA

SEQ ID NO: 86 DNA fragment comprising the nucleotide sequence of M7GLOD

GGAGATATACATATGGACGACAAGACCTATCAGCAGTTAGCACGCGAACTGCTGC
TTGTAGGGCCCGAGCCAGCGAATGAAGATCTTAAATTGCGCTATTTAGACGTACT
TATTGATAACGGGCTGGAGCCCCAGTCGATCGTAAACGTATCTTAATTGTGGGG
GCAGGGATCGCCGGCCTGGTCGCGGGACATTTGTTGACACGCGCGGGACACGATG
TCACCATCCTGGAGGCGAATGCAAATCGCGTTGGAGGTCGCATTAAGACATTTCA
TGCAAAGAAAGGCGAACCGGCCCCCTTCACCGACCCGGCTCAATATGCAGAGGC
GGGAGCTATGCGTTTGCCTTCATTCCATCCCTTAACGTTAGCGCTGATTGATAAAT

TAGGGTTGAAGCGTCGCTTGTTCTTTAATGTTGACATTGACCCTAAGACGGGCAAT

CAAGGTGCAGCATTGCCGCCTGTCGTTACAAGAGCTTTAAGGACGGTAAAACTT

GGACGTATGGTAAACCTTCTCCGGAATTCCGTGAACCAGACAAACGTAACCACAC

TTGGATTCGTACCAATCGCACTCAAGTACGTCGCGCCCAGTATGTGAAGGACCCG

AGTGCGATTAACGAAGGGTTCCATTTAACCGGGTGCGAGTCGCGTTTGACAGTTT

CTGATATGGTGAACCAAGCTTTAGAACCGGTTCGTGACTACTATTCTGTATTGCAG

AGCGACGGGCGCCGCGTAAATAAGCCATTTAAGGAGTGGTTAGACGGCTGGGCC

GGTGTAATCCGCGATTTCGACGGATTCTCAATGGGACGTTTTCTGCGCGAGTACGC

TGGGTTTTCGGACGAGGCGGTAGAAGCAATCGGTACCATCGAGAATATGACAAGT

CGTTTGCATTTGGCATTTTTTCACAGCTTCTTAGGTCGCAGTGACATTGACCCCAG

CGCGACATATTGGGAGATTGAAGGCGGTAGCCGTCAGCTGCCTGAGGCTCTGGCA

AAAGACCTGCGTGATCAGATTGTAATGGGCCAACGCATGGTGCGTTTAGAGTACT

ACGACCCAGGACGTGATGGACACCATGGAGGTCTGGCAGGACCCTCTGGTCCTGC

GGTTGCAATCGAAACTGTACCCGAGAACGAGCCAAGTGCAGAGCCGCAGACGTG

GACTGCGGACCTGGCGATTGTCACTGTACCATTTTCGTCTCTTCGCTTTGTTGCGG

TGACTCCCCCATTCAGTTATAAAAAACGTCGTGCAGTTATCGAAACTCACTATGAT

CAGGCAACAAAAGTGCTGTTAGAGTTTTCTCGTCGCTGGTGGGAGTTTACAGAAG

AGGACTGGAAGCGTGAGTTGGACGCGATTGCCCCCGGCCTGTATGAATACTACCA

GCGTTGGGGCGAGGACGATGCCGAAGCAGCCCTTACGGTACCCGAAAGTGTACG

CAATCTTCCAACGGGATTGTTAGGGGCCCATCCTTCCGTTGACGAGCAACTGATTG

ATGACGAGCAGGTAGAGTATCTGCGTAATTCAACATTGCGTGGAGGCGTACGCCC

GGCAACTCAAGTTCATGGAGGAGGTTCAACAACTGACAATCCAAACCGTTTTATG

TATTATCCGAGTCACGCGGTGCCTGGTTCTAAGGGTGGTGTGGTTTTGGCCGCGTA

TAGTTGGAGTGACGATGCAGCGCGTTGGGATTCCTTTGATGACGCCGAACGTTAT

GGCTACGCCCTGGAAAACCTTCAATCGGTCCATGGCCGCCGCATCGAGGTCTTTT

ATACTGGAGCTGGACAAACTCAGTCATGGTTGCGCGACCCCTACGCCTGCGGTGA

GGCCGCAGTTTACACACCACATCAGATGACATCTTTTCACTTAGACGTAGTGCGCC

CGGAGGGGCCAGTATACTTTGCGGGAGAGCATGTCTCTCTTAAGCATGCGTGGAT

CGAGGGGGCCGTTGAAACCGCGGTCCGTGCTGCGATCGCGGTTAACGAAGCCCC

53

GTACCATACGACACAGCTGCTGCTCGTGCGGAGGCCCCTCGCGAACGTGCCGGTA
CAGCATCAGCCACTCGCACGCGCGAGAAGGCCGTGACTTCCTAACAAAGCCCGA
AA

SEQ ID NO: 87 Amino acid sequence of M7GLOD-T8

MDDKTYQQLARELLLVGPEPANEDLKLRYLDVLIDNGLEPPVDRKRILIVGAGIAGLV
AGHLLTRAGHDVTILEANANRVGGRIKTYHAKKGEPAPFTDPAQIAEAGAMRLPSFH
PLTLALIDKLGLKRRLFNVDIDPKTGNQGAALPPVVYKSFKDGKTWTYGKPSPEFREP
DKRNHTWIRTNRTQVRRAQYVKDPSAINEGFHLTGCESRLTVSDMVNQALEPVRDY
YSVLQSDGRRVNKPFKEWLDGWAGVIRDFDGFSMGRFLREYAGFSDEAVEAIGTIEN
MTSRLHLAFMHSFLGRSDIDPSATYWEIEGGSRQLPEALAKDLRDQIVMGQRMVRLE
YYDPGRDGHHGGLAGPSGPAVAIETVPENEPSAEPQTWTADLAIVTVPLSSLRFVAVT
PPFSYKKRRAVIETHYDQATKVLLEYSRRWWEFTEEDWKRELDAIAPGLYEYYQRW
GEDDAEAALTVPESVRNLPTGLLGAHPSVDEQLIDDEQVEYLRNSTLRGGVRPATQV
HGGGSTTDNPNRFMYYPSHAVPGSKGGVVLAAYSWSDDAARWDSFDDAERFGYAL
ENLQSVHGRRIEVFLTGAGQTQSWLRDPYACGEAAVYTPHQMTSFHLDVVRPEGPV
YFAGEHVSLKHAWIEGAVETAVRAAIAVNEAPVPYDTAAARAEAPRERAGTASATR
TREKAVTS

SEQ ID NOs: 88 to 117 Primer sequences

All publications, patents and patent applications used in the present specification are incorporated herein in their entirety by reference.

**Claims**

1. A glutamate oxidase mutant having a deleted region, wherein

   all or part of an inter-γ-β region located between the γ chain region and the β chain region of a wild type glutamate oxidase is deleted,
   1 to 10 carboxy terminal amino acids of the γ chain region of the wild type glutamate oxidase are deleted or not deleted,
   1 to 4 amino terminal amino acids of the β chain region of the wild type glutamate oxidase are deleted or not deleted,
   wherein, the deleted region is one continuous deleted region, and the glutamate oxidase mutant comprises glutamate oxidase activity.

2. The glutamate oxidase mutant according to claim 1, wherein 31 to 54 continuous amino acids are deleted.

3. The glutamate oxidase mutant according to claim 1, wherein the C terminus of the deleted region is located at the position corresponding to the 510th position, the position corresponding to the 508th position, or the position corresponding to the 507th position of SEQ ID NO: 1.

4. The mutant according to claim 1, wherein the amino acid sequence of the region corresponding to the 459th to 507th positions of SEQ ID NO: 1 is deleted and thermal stability is improved compared to a glutamate oxidase in which all or part of the region corresponding to the 459th to 507th positions of SEQ ID NO: 1 is not deleted.

5. The mutant according to claim 4, wherein the amino acid sequence of the region corresponding to the 459th to 507th positions of SEQ ID NO: 1 is deleted and thermal stability is improved compared to a glutamate oxidase in which the amino acid sequence of the region corresponding to the 459th to 466th positions of SEQ ID NO: 1 is not deleted.

6. The mutant according to claim 4 or 5, wherein

the improved thermal stability is evaluated from residual activity after heat treatment at 45°C for 30 minutes or 35 minutes or heat treatment at 65°C for 30 minutes, and
the residual activity of the mutant according to claim 4 after being subjected to heat treatment at 45°C for 30 minutes or 35 minutes or heat treatment at 65°C for 30 minutes is 110% or more when the residual activity of a glutamate oxidase comprising no deletion of the amino acid sequence after being subjected to the same treatment as above is defined as 100%, or
the residual activity of the mutant according to claim 5 after being subjected to heat treatment at 45°C for 30 minutes or 35 minutes or heat treatment at 65°C for 30 minutes is 110% or more when the residual activity of a glutamate oxidase comprising no deletion of the amino acid sequence after being subjected to the same treatment as above is defined as 100%.

7. The mutant according to any one of claims 1 to 6, wherein the mutant comprises an amino acid sequence identity of 90% or more with the amino acid sequence of SEQ ID NO: 58, SEQ ID NO: 59, SEQ ID NO: 12 or SEQ ID NO: 13, comprises a deletion of the amino acid sequence, and comprises glutamate oxidase activity.

8. The mutant according to any one of claims 1 to 7, wherein

the mutant further comprises an amino acid substitution compared to the amino acid sequence of SEQ ID NO: 1 and/or SEQ ID NO: 58, at one or more positions selected from the group consisting of
the position corresponding to the 87th position of SEQ ID NO: 1,
the position corresponding to the 103rd position of SEQ ID NO: 1,
the position corresponding to the 133rd position of SEQ ID NO: 1,
the position corresponding to the 186th position of SEQ ID NO: 1,
the position corresponding to the 297th position of SEQ ID NO: 1,
the position corresponding to the 376th position of SEQ ID NO: 1,
the position corresponding to the 393rd position of SEQ ID NO: 1,
the position corresponding to the 428th position of SEQ ID NO: 1,
the position corresponding to the 516th position of SEQ ID NO: 1,
the position corresponding to the 566th position of SEQ ID NO: 1,
the position corresponding to the 568th position of SEQ ID NO: 1,
the position corresponding to the 585th position of SEQ ID NO: 1, and
the position corresponding to the 615th position of SEQ ID NO: 1, and
the thermal stability of the mutant after the amino acid substitution is improved compared to a glutamate oxidase before the substitution.

9. The mutant according to claim 8, wherein

the amino acid substitution at the position corresponding to the 87th position of SEQ ID NO: 1 is substitution with tyrosine,
the amino acid substitution at the position corresponding to the 103rd position of SEQ ID NO: 1 is substitution with an amino acid selected from the group consisting of phenylalanine, leucine, valine, isoleucine and methionine,
the amino acid substitution at the position corresponding to the 133rd position of SEQ ID NO: 1 is substitution with an amino acid selected from the group consisting of leucine and tyrosine,
the amino acid substitution at the position corresponding to the 186th position of SEQ ID NO: 1 is substitution with an amino acid selected from the group consisting of glutamic acid, aspartic acid, tyrosine, glutamine, asparagine, alanine, leucine, cysteine, methionine, phenylalanine, serine, histidine and threonine,
the amino acid substitution at the position corresponding to the 297th position of SEQ ID NO: 1 is substitution with an amino acid selected from the group consisting of leucine, valine, isoleucine and methionine,

the amino acid substitution at the position corresponding to the 376th position of SEQ ID NO: 1 is substitution with an amino acid selected from the group consisting of phenylalanine, leucine, isoleucine and methionine, the amino acid substitution at the position corresponding to the 393rd position of SEQ ID NO: 1 is substitution with an amino acid selected from the group consisting of leucine, valine, isoleucine and methionine, the amino acid substitution at the position corresponding to the 428th position of SEQ ID NO: 1 is substitution with an amino acid selected from the group consisting of tyrosine and methionine, the amino acid substitution at the position corresponding to the 516th position of SEQ ID NO: 1 is substitution with phenylalanine, the amino acid substitution at the position corresponding to the 566th position of SEQ ID NO: 1 is substitution with an amino acid selected from the group consisting of phenylalanine, leucine, valine and methionine, the amino acid substitution at the position corresponding to the 568th position of SEQ ID NO: 1 is substitution with an amino acid selected from the group consisting of isoleucine and methionine, the amino acid substitution at the position corresponding to the 585th position of SEQ ID NO: 1 is substitution with an amino acid selected from the group consisting of leucine and methionine, or the amino acid substitution at the position corresponding to the 615th position of SEQ ID NO: 1 is substitution with an amino acid selected from the group consisting of leucine, valine, and phenylalanine.

10. The mutant according to claim 9, wherein

the mutant comprises an amino acid sequence identity of 90% or more with the amino acid sequence of SEQ ID NO: 69, comprises a deletion of the amino acid sequence, comprises an amino acid substitution compared to the amino acid sequence of SEQ ID NO: 58, at one or more positions selected from the group consisting of
the position corresponding to the 87th position of SEQ ID NO: 1,
the position corresponding to the 103rd position of SEQ ID NO: 1,
the position corresponding to the 133rd position of SEQ ID NO: 1,
the position corresponding to the 186th position of SEQ ID NO: 1,
the position corresponding to the 297th position of SEQ ID NO: 1,
the position corresponding to the 376th position of SEQ ID NO: 1,
the position corresponding to the 393rd position of SEQ ID NO: 1,
the position corresponding to the 428th position of SEQ ID NO: 1,
the position corresponding to the 516th position of SEQ ID NO: 1,
the position corresponding to the 566th position of SEQ ID NO: 1,
the position corresponding to the 568th position of SEQ ID NO: 1,
the position corresponding to the 585th position of SEQ ID NO: 1, and
the position corresponding to the 615th position of SEQ ID NO: 1,
the amino acid substitution being any of the amino acid substitutions described in claim 9, and
wherein, the mutant comprises glutamate oxidase activity.

11. The glutamate oxidase mutant according to any one of claims 1 to 10, wherein the amino acid sequence of the region corresponding to the 670th to 687th positions of SEQ ID NO: 1 is further deleted.

12. A composition, reagent, electrode, sensor or kit comprising the glutamate oxidase mutant according to any one of claims 1 to 10.

13. A polynucleotide encoding the glutamate oxidase mutant according to any one of claims 1 to 11.

14. A vector comprising the polynucleotide according to claim 13.

15. A host cell comprising the vector according to claim 14.

16. A method for producing a glutamate oxidase mutant, comprising the steps of:

culturing the host cell according to claim 15 to produce the glutamate oxidase mutant according to any one of claims 1 to 11, and
obtaining the produced glutamate oxidase mutant.

17. A method for bringing the glutamate oxidase mutant according to any one of claims 1 to 11 or the composition, reagent, electrode, sensor or kit according to claim 12 into contact with a sample containing glutamic acid to oxidize the glutamic acid contained in the sample.

18. The method according to claim 17, comprising detecting glutamic acid.

19. A glutamate oxidase mutant comprising an amino acid substitution, wherein

the thermal stability of the glutamate oxidase mutant after the amino acid substitution is improved compared to the glutamate oxidase before the substitution, and
the glutamate oxidase mutant meets a condition selected from the group consisting of the following:

(i) when the amino acid sequence of the glutamate oxidase mutant is aligned with the amino acid sequence of SEQ ID NO: 1, the glutamate oxidase mutant comprises a substitution of the amino acid at the position corresponding to a position selected from the group consisting of the 87th, 103rd, 133rd, 186th, 297th, 376th, 393rd, 428th, 516th, 566th, 568th, 585th, and 615th positions of SEQ ID NO: 1,
(ii) the glutamate oxidase mutant of said (i), wherein the glutamate oxidase mutant consists of an amino acid sequence comprising a substitution, deletion or addition of one or several amino acids at a position other than the positions corresponding to the 87th, 103rd, 133rd, 186th, 297th, 376th, 393rd, 428th, 516th, 566th, 568th, 585th, and 615th positions of SEQ ID NO: 1,
(iii) with regard to the glutamate oxidase mutant of said (i) or (ii), the full length amino acid sequence thereof comprises an amino acid sequence identity of 70% or more, 80% or more, or 90% or more with the amino acid sequence of SEQ ID NO: 1 or SEQ ID NO: 58, and
(iv) with regard to the glutamate oxidase mutant of said (i) or (ii), the full length amino acid sequence thereof comprises an amino acid sequence identity of 70% or more, 80% or more, or 90% or more with the amino acid sequence of SEQ ID NO: 1 or SEQ ID NO: 58, the amino acid substitution at the position corresponding to the 291st position of SEQ ID NO: 1 in the glutamate oxidase mutant is arginine, and the amino acid sequence of the positions corresponding to the 51st to 56th positions of SEQ ID NO: 1 is Gly-Xaa-Gly-Xaa-Xaa-Gly (wherein Xaa represents any amino acid).

20. The glutamate oxidase mutant according to claim 19, wherein

the amino acid substitution at the position corresponding to the 87th position of SEQ ID NO: 1 is substitution with tyrosine,
the amino acid substitution at the position corresponding to the 103rd position of SEQ ID NO: 1 is substitution with an amino acid selected from the group consisting of phenylalanine, leucine, valine, isoleucine and methionine,
the amino acid substitution at the position corresponding to the 133rd position of SEQ ID NO: 1 is substitution with an amino acid selected from the group consisting of leucine and tyrosine,
the amino acid substitution at the position corresponding to the 186th position of SEQ ID NO: 1 is substitution with an amino acid selected from the group consisting of glutamic acid, aspartic acid, tyrosine, asparagine, glutamine, alanine, leucine, cysteine, methionine, phenylalanine, serine, histidine and threonine,
the amino acid substitution at the position corresponding to the 297th position of SEQ ID NO: 1 is substitution with an amino acid selected from the group consisting of leucine, valine, isoleucine and methionine,
the amino acid substitution at the position corresponding to the 376th position of SEQ ID NO: 1 is substitution with an amino acid selected from the group consisting of phenylalanine, leucine, isoleucine and methionine,
the amino acid substitution at the position corresponding to the 393rd position of SEQ ID NO: 1 is substitution with an amino acid selected from the group consisting of leucine, valine, isoleucine and methionine,
the amino acid substitution at the position corresponding to the 428th position of SEQ ID NO: 1 is substitution with an amino acid selected from the group consisting of tyrosine and methionine,
the amino acid substitution at the position corresponding to the 516th position of SEQ ID NO: 1 is substitution with phenylalanine,
the amino acid substitution at the position corresponding to the 566th position of SEQ ID NO: 1 is substitution with an amino acid selected from the group consisting of phenylalanine, leucine, valine and methionine,
the amino acid substitution at the position corresponding to the 568th position of SEQ ID NO: 1 is substitution with an amino acid selected from the group consisting of isoleucine and methionine,
the amino acid substitution at the position corresponding to the 585th position of SEQ ID NO: 1 is substitution with an amino acid selected from the group consisting of leucine and methionine, or
the amino acid substitution at the position corresponding to the 615th position of SEQ ID NO: 1 is substitution with an amino acid selected from the group consisting of leucine, valine, and phenylalanine.

# EP 4 467 649 A1

<table>
<tr><td colspan="2">INTERNATIONAL SEARCH REPORT</td><td>International application No.<br>**PCT/JP2023/001336**</td></tr>
</table>

| A. | CLASSIFICATION OF SUBJECT MATTER |
|---|---|

*C12N 15/53*(2006.01)i; *C12N 1/15*(2006.01)i; *C12N 1/19*(2006.01)i; *C12N 1/21*(2006.01)i; *C12N 5/10*(2006.01)i; *C12N 9/04*(2006.01)i; *C12N 15/63*(2006.01)i; *C12P 21/02*(2006.01)i
FI: C12N15/53; C12N15/63 Z; C12N1/15; C12N1/19; C12N1/21; C12N5/10; C12P21/02 C; C12N9/04 ZNA

According to International Patent Classification (IPC) or to both national classification and IPC

| B. | FIELDS SEARCHED |
|---|---|

Minimum documentation searched (classification system followed by classification symbols)

C12N15/00-15/90; C12N9/00-9/99

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN); GenBank/EMBL/DDBJ/GeneSeq

| C. | DOCUMENTS CONSIDERED TO BE RELEVANT |
|---|---|

| Category* | Citation of document, with indication, where appropriate, of the relevant passages | Relevant to claim No. |
|---|---|---|
| X | WO 2021/193598 A1 (AJINOMOTO CO., INC.) 30 September 2021 (2021-09-30) | 1-7, 11-18 |
| | claims, examples, seq. ID no. 3 | |
| Y | | 1-20 |
| X | CN 109266664 A (UNIV. NANJING TECH.) 25 January 2019 (2019-01-25) | 1-7, 12-18 |
| | abstract, claims, paragraphs [0002], [0004], examples, seq. ID no. 2 | |
| Y | | 1-20 |
| X | Database GenBank [online]. Accession No. BCH42006.1. 25 December 2021 uploaded, [retrieved on 8 March 2023] SUGIMORI, D. Definition: L-glutamate oxidase [Streptomyces sp.]. <URL:https://www.ncbi.nlm.nih.gov/protein/BCH42006.1/><br>particularly, Definition, Origin | 19-20 |
| Y | | 1-20 |
| X | CN 106282205 A (SHANGHAI ACAD. AGRI. SCIENCES) 04 January 2017 (2017-01-04) | 19-20 |
| | abstract, seq. ID no. 65 | |
| Y | | 1-20 |

☑ Further documents are listed in the continuation of Box C. ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **08 March 2023** | **20 March 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
| --- |
| **PCT/JP2023/001336** |

**C. DOCUMENTS CONSIDERED TO BE RELEVANT**

| Category* | Citation of document, with indication, where appropriate, of the relevant passages | Relevant to claim No. |
| --- | --- | --- |
| Y | ARIMA, Jiro et al. Recombinant Expression, Biochemical Characterization and Stabilization through Proteolysis of an L-Glutamate Oxidase from Streptomyces sp. X-119-6. J. Biochem. 2003, 134(6), pages 805-812<br>    particularly, abstract | 1-20 |
| Y | 杉森大助ほか, 人工タンパク質設計によるStreptomyces sp. NT1株由来L-グルタミン酸オキシダーゼの耐熱化, 日本農芸化学会2019年度大会講演要旨集 (オンライン), 05 March 2019, 4E1a07, (SUGIMORI, Daisuke et al. Proceedings (Online) of the Annual Meeting of Japan Society for Bioscience, Biotechnology, and Agrochemistry 2019.), non-official translation (Thermal resistance of L-glutamate oxidase derived from Streptomyces sp. NT1 strain by artificial protein design.)<br>    particularly, 方法•結果と考察の項, non-official translation (methods • results and discussion) | 1-20 |
| Y | 林優花ほか, Streptomyces sp. NT1株由来L-グルタミン酸オキシダーゼの人工タンパク質設計による耐熱性の向上, 日本農芸化学会2018年度大会講演要旨集 (オンライン), 05 March 2018, 2A25p06, (HAYASHI, Yuka et al. Proceedings (Online) of the Annual Meeting of Japan Society for Bioscience, Biotechnology, and Agrochemistry 2018.), non-official translation (Streptomyces sp. Improvement of heat resistance by artificial protein design of L-glutamate oxidase derived from strain NT1.)<br>    particularly, 方法•結果の項, non-official translation (method • results) | 1-20 |
| P, X | HAYASHI, Yuka et al. Thermostability enhancement of L-glutamate oxidase from Streptomyces sp. NT1 by full consensus protein design. J. Biosci. Bioeng. 2022, 133(4), pages 309-315, Epub 19 January 2022<br>    particularly, abstract, fig. 1 | 19-20 |
| P, Y | | 1-20 |

Form PCT/ISA/210 (second sheet) (January 2015)

**INTERNATIONAL SEARCH REPORT**

| International application No. |
| --- |
| **PCT/JP2023/001336** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

   a. ☑ forming part of the international application as filed:

       ☑ in the form of an Annex C/ST.25 text file.

       ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

       ☐ in the form of an Annex C/ST.25 text file (Rule 13*ter*.1(a)).

       ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   "The form of Annex C/ST.25 text file" above shall read as "the form of ST.26.".

Form PCT/ISA/210 (continuation of first sheet) (January 2015)

INTERNATIONAL SEARCH REPORT
Information on patent family members

International application No.

**PCT/JP2023/001336**

| Patent document cited in search report | | | Publication date (day/month/year) | Patent family member(s) | Publication date (day/month/year) |
|---|---|---|---|---|---|
| WO | 2021/193598 | A1 | 30 September 2021 | (Family: none) | |
| CN | 109266664 | A | 25 January 2019 | (Family: none) | |
| CN | 106282205 | A | 04 January 2017 | (Family: none) | |

Form PCT/ISA/210 (patent family annex) (January 2015)

## REFERENCES CITED IN THE DESCRIPTION

**Patent documents cited in the description**

- WO 2021193598 A
- JP 2022005869 A
- JP 2007222055 A
- JP 2011239681 A

**Non-patent literature cited in the description**

- **KUSAKABE H et al.** *Agric. Biol. Chem.*, 1983, vol. 47, 1323-1328
- **ARIMA J et al.** *J. Biochem.*, 2003, vol. 134, 805-812
- *Nucleic Acids Res.*, 1984, vol. 12, 9441-9456
- *Nucleic Acids Res.*, 1985, vol. 13, 8749-8764
- *Nucleic Acids Res.*, 1985, vol. 13, 8765
- *Nucleic Acids Res*, 1986, vol. 14, 9679
- *Proc. Natl. Acid. Sci. U.S.A.*, 1985, vol. 82, 488-492
- *Methods Mol. Cell. Biol.*, 1995, vol. 5, 255-269
- *J Microbiol Methods*, 2003, vol. 55, 481-484
- *Mol. Gen. Genet.*, 1989, vol. 218, 99-104
- **OZEKI et al.** *Biosci. Biotechnol. Biochem.*, 1995, vol. 59, 1133
- *Chem. Rev.*, 2005, vol. 105 (11), 4056-72
- *Comb Chem High Throughput Screen.*, 2008, vol. 11 (2), 127-34
- *Curr Opin Struct Biol*, 2007, vol. 17, 474-80
- **AGRESTI et al.** Ultrahigh-throughput screening in drop-based microfluidics for directed evolution. *Proceedings of the National Academy of Sciences*, March 2010, vol. 107 (9), 4004-4009
- **STRYER et al.** *Biochemistry*, 2002, vol. 5, 44-49